# EUROPÄISCHE PATENTSCHRIFT

(11) **EP 0 462 065 B1**
(45) Veröffentlichungstag und Bekanntmachung des Hinweises auf die Patenterteilung: **02.03.2005**
(21) Anmeldenummer: 91810430.8
(22) Anmeldetag: 06.06.1991
(51) Int. Cl.: C12N 15/62, C12N 15/56, C12N 15/29, C07K 7/08, C12N 5/10, C12N 1/21, A01H 5/00

(54) **Neue Signalsequenzen**
New signal sequences
Nouvelles séquences de signal

(30) Priorität: 15.06.1990 CH 200790
(43) Veröffentlichungstag der Anmeldung: 18.12.1991
(73) Patentinhaber: Syngenta Participations AG, 4058 Basel (CH)
(72) Erfinder: Boller, Thomas, Prof.Dr., CH-4104 Oberwil (CH); Neuhaus, Jean-Marc, Dr., CH-4054 Basel (CH); Ryals, John, Dr., Durham, N.C. 27713 (US)

(56) Entgegenhaltungen:
- EP-A- 0 351 924
- EP-A- 0 353 191
- EP-A- 0 392 225
- EP-A- 0 418 695
- EP-A- 0 440 304
- EP-A- 0 442 592
- EP-A- 0 448 511
- EP-A- 0 460 753
- WO-A-90/07001
- THE PLANT CELL, Band 2, Nr. 4, April 1990, Seiten 301-313, Rockville, MD, US; T.A. WILKINS et al.: "Role of propeptide glycan in post-translational processing and transport of barley lectin to vacuoles in transgenic tobacco"
- IDEM
- MOLECULAR PLANT-MICROBE INTERACTIONS, Band 3, Nr. 4, 1990, Seiten 252-258; H.J.M. LINTHORST et al.: "Analysis of acidic and basic chitinases from tobacco and petunia and their constitutive expression in transgenic tobacco"
- PLANT MOLECULAR BIOLOGY, Band 14, Nr. 3, März 1990, Seiten 357-368, Dordrecht, NL; H. SHINSHI et al.: "Structure of a tobacco endochitinase gene: evidence that different chitinase genes can arise by transposition of sequence encoding a cysteine-rich domain"
- PROCEEDINGS OF THE NATIONAL ACADEMY OF SCIENCES OF THE USA, Band 86, April 1989, Seiten 2673-2677, Washington, US; M. VAN DEN BULCKE et al.: "Characterization of vacuolar and extracellular beta[1,3]-glucanases of tobacco: Evidence for a strictly compartmentalized plant defense system"
- THE PLANT CELL, Band 2, Nr. 12, Dezember 1990, Seiten 1145-1155, Rockville, MD, US; S.Y. BEDNAREK et al.: "A carboxyl-terminal propeptide is necessary for proper sorting of barley lectin to vacuoles of tobacco"
- Shinshi H. et al: Proc. Natl. Acad. Sci. USA, 85: 5541-5545 (1988)
- Narendra K.S. et al: Plant Physiol., 90:1096-1101 (1989)

## Beschreibung

Die vorliegende Erfindung betrifft neue Peptidfragmente ['Targeting'-Signal], die aus der C-terminalen Region [C-terminale Extension] pflanzlicher Chitinasen erhältlich sind und die in operabler Verknüpfung mit einem beliebigen Proteinmolekül heterologen Ursprungs für eine gezielte Einschleusung der mit diesen Peptidfragmenten assoziierten Proteine in die pflanzliche Vakuole sorgen.

Die vorliegenden Erfindung betrifft weiterhin DNA-Sequenzen, welche die zuvor näher charakterisierten Peptidfragmente kodieren und in operabler Verknüpfung mit einer beliebigen, exprimierbaren DNA zu einem Genprodukt führen, das gezielt in die pflanzliche Vakuole eingeschleust wird, sowie Mutanten und Varianten davon.

Ein weiterer Gegenstand der vorliegenden Erfindung betrifft rekombinante DNA Moleküle worin eine DNA-Sequenz, die aus der 3'-terminalen Region eines Gens erhältlich ist, das für ein natürlicherweise vakuolär vorliegendes Proteinmolekül kodiert und worin besagte DNA Sequenz ein C-terminales Peptid [C-terminale Extension] kodiert, das für die gezielte Einschleusung ['Targeting'] eines assoziierten Genproduktes heterotogen Ursprungs in die pflanzliche Vakuole verantwortlich ist, und wobei besagte DNA-Sequenz nicht die ca. 20 C-terminalen Aminosäuren des primären Translationsprodukts von Wild-Typ Osmotin kodiert, mit einem heterologen Gen operabel verknüpft ist, sowie die davon abgeleiteten Vektoren. Ebenso umfasst sind Wirtszellen und/oder Wirtsorganismen, einschliesslich transgener Pflanzen, die besagte rekombinante DNA Moleküle enthalten.

Die Verfahren zur Herstellung der erfindungsgemässen rekombinanten DNA Moleküle bilden einen weiteren Bestandteil der vorliegenden Erfindung. Ebenso umfasst sind transgene Pflanzen, die die erfindungsgemässen rekombinanten DNA - Moleküle enthalten.

Im Rahmen des Genetic Engineering ist man in jüngster Zeit in zunehmendem Masse daran interessiert über die reine Expression des eingeschleusten Fremdgens hinaus DNA-Sequenzen aufzuspüren, die sog. Signalsequenzen kodieren, welche es dem assoziierten Genprodukt erlauben, entsprechend seiner Funktion an seinen spezifischen Bestimmungsort zu gelangen, wo es dann seine optimale Wirkung entfalten oder aber in geeigneter Weise abgelagert werden kann. Bezogen auf die ganze Pflanze bedeuted dies, dass man bespielsweise versucht Promotoren zu identifizieren oder zu entwickeln, die eine gewebe- und/oder entwicklungsspezifische Expression des eingeschleusten Fremdgens ermöglichen.

Die ziel- bzw. bestimmungsorientierte Plazierung eingeschleuster Fremdgene oder deren Expressionsprodukte ist jedoch nicht nur auf dem Niveau der Pflanze von Relevanz, sondern kann auch bereits auf der Zellebene von grosser Bedeutung sein, insbesondere was die Effektivität der Transformation betrifft.

So ist beispielsweise bekannt, dass in pflanzlichen wie auch in anderen eukaryontischen Zellen die Mehrzahl der Proteine zwar an cytoplasmatischen Ribosomen synthetisiert wird, dass aber ein Grossteil dieser Proteine in ganz unterschiedlichen subzellulären Kompartimenten benötigt wird. Eine Ausnahme hiervon bilden lediglich einige Mitochondrien- und Chloroplasten-Proteine, die direkt am Ort ihrer Verwendung hergestellt werden. Die cytoplasmatisch hergestellten Proteine dagegen werden entweder entlang des die Zelle durchziehenden Endomembransystems in das lytische Kompartiment [Vakuole, Lysosomen] der Zelle und den Extrazellularraum transportiert oder aber sie werden direkt von ihrem jeweiligen Kompartiment [Vakuole, Chloroplasten, Peroxisomen] aufgenommen.

Für die Aufrechterhaltung dieser auf der subzellulären Ebene verwirklichten Kompartimentierung müssen innerhalb der Zelle spezifische Transport- und Sortiersysteme vorliegen, die eine funktionsgerechte Verteilung der cytoplasmatisch produzierten Proteine gewährleisten. Diese Proteine müssen daher eine oder aber mehrere zusätzliche Informationen enthalten, die es den besagten Transport- und Sortiersystemen der Zelle erlauben ihr jeweiliges Substrat zu erkennen und dieses an ihren spezifischen Bestimmungsort zu dirigieren. So konnte beispielsweise bei zahlreichen cytoplasmatischen Precursoren von Mitochondrien- und Chloroplasten-Proteinen am N-terminalen Ende ein sog. Transit-Peptid nachgewiesen werden, welches die Aufnahme dieser Proteine in ihr jeweiliges Kompartiment sicherstellt. Ganz entsprechend besitzten die nukleären Proteine eine Zellkern-spezifische Sequenz.

Von besonderer Bedeutung für den Intrazellulären Protein-Transport ist das Endomembransystem der Zelle. Dieses die Zelle durchziehende Membransystem, das sich aus dem Endoplasmatischen Retikulum und dem Golgi-Apparat zusammensetzt, dient im wesentlichen dem Transport von Proteinen, insbesondere von cytoplasmatisch hergestellten Proteinen, zum lytischen Kompartiment [Vakuole, Lysosomen] und zum extrazellulären Raum.

Proteine, die über das Endomembransystem transportiert werden, gelangen zunächst ins Endoplasmatische Retikulum. Das für diesen Schritt notwendige Transportsignal wird durch eine Signalsequenz am N-terminalen Ende des Moleküls repräsentiert, das sog. Signalpeptid. Dieses Signalpeptid wird, sobald es seine Funktion erfüllt hat, proteolytisch vom Precursor-Protein abgespalten. Entsprechend seiner spezifischen Funktion wurde dieser Sequenz-Typ des Signal-Peptids im Verlaufe der Evolution bei allen lebenden Zellen in hohem Masse konserviert, unabhängig davon, ob es sich um Bakterien, Hefen, Pilze, Tiere oder Pflanzen handelt.

Ein weiterer Sortierschritt findet schliesslich im Golgi-Apparat statt, wo die Trennung der für das lytische Kompartiment [Vakuole, Lysosomen] und der für die Sekretion in den extrazellulären Raum bestimmten Proteine stattfindet.
Bei Untersuchungen an Hefen und Tieren hat man gefunden, dass Proteine, die kein zusätzliches Signal enthalten, offenbar automatisch in den extrazellulären Raum sezerniert werden, während Proteine, die ein solches zusätzliches Sortiersignal enthalten, in das lytische Kompartiment ausgeschleust werden.

Dieses Sortiersignal kann dabei ganz unterschiedlicher Natur sein. Bei den bisher untersuchten Tieren, beispielsweise, handelt es sich in der Regel um eine spezifische Modifikation innerhalb der Glykankette von Glykoproteinen, und zwar um eine Mannose-6-phosphat-Gruppe. Diese Gruppe wird von einem spezifischen Mannose-6-phosphat-Rezeptor erkannt, was dazu führt, dass die entsprechenden Proteine in spezifischen Vesikeln aus dem Golgiapparat freigesetzt und zu den Lysosomen transportiert werden. Dabei ist es aber bisher nicht gelungen, diejenige Polypeptidsequenz zu ermitteln, welche den Anstoss für die Phosphorylierung einer Mannose-Gruppe an der Glykanseitenkette gibt.

In Hefen handelt es sich bei dem entsprechenden Sortiersignal für das lytische Kompartiment nicht um eine Glykankette, sondern um eine Aminosäure-Sequenz, die nach Abspaltung des Signalpeptids den N-Terminus des Proteins bildet. Dieses N-terminale 'Targeting'-Signal für die Vakuole wird im allgemeinen in der Vakuole selbst mit Hilfe der Proteinase A abgespalten. Oft wird das in die Vakuole transportierte Protein erst durch diese Abspaltung des 'Targeting'-Signals zu einem katalytisch aktiven Enzym.

Bei Pflanzen ist die Frage nach dem für die Einschleusung von Proteinen in die Vakuole verantwortlichen 'Targeting'-Signal deshalb besonders interessant, -insbesondere unter anwendungsortientierten Gesichtspunkten-, weil die Vakuole nicht nur das lytische Komparitment der Pflanzenzelle darstellt sondern auch das grösste Speicher-Kompartiment für Reservestoffe, Entgiftungsprodukte und Abwehrstoffe bildet [Boller und Wiemken (1986)].

Es wäre daher von grossem Vorteil, wenn es gelänge, Proteine, die im Zusammenhang mit einer Verbesserung des Nährstoffgehaltes der Pflanze stehen, gezielt in die Vakuole zu dirigieren und dort abzuspeichern, da es sich hierbei um das mit Abstand geräumigste Kompartiment der pflanzlichen Zelle für gelöste Substanzen handelt. Die wichtigsten Speicher-Proteine von Knollen, Zwiebeln, Wurzeln und Stengeln beispielsweise, befinden sich in den Vakuolen der diese Organe aufbauenden Zellen [Boller und Wiemke (1986)]. Ferner sind die Speicher-Proteine der meisten Samen in sog. 'Protein bodies' lokalisiert, spezialisierten Vakuolen, für die die gleichen 'Targeting-Signale gelten dürften wie für die Vakuolen der vegetativen Organe.

Ähnliche Überlegungen gelten auch für Substanzen, die bei der Bekämpfung von Schädlingen oder Krankheiten eingesetzt werden können, insbesondere dann, wenn sich diese Substanzen als toxisch für die Pflanze selbst erweisen. Es ist daher vorteilhaft auch diese Substanzen in der pflanzlichen Vakuole zu deponieren.
Schliesslich dient die Vakuole in bestimmten Fällen auch als Entgiftungsorgan, indem sie z.B. die von der Pflanze synthetisierten Entgiftungsprodukte speichert [Boller und Wiemke (1986)]. Es wird daher angestrebt, Entgiftungs-Enzyme ebenfalls gezielt in die Vakuole einzuschleusen.

Das genau umgekehrte Problem stellt sich dagegen beispielsweise beim Befall von Kulturpflanzen mit bestimmten Pilzpathogenen. Diese infizieren ihre Wirtspflanze, indem sie ihr Mycel über die interzellulären Räume der Pflanze vorantreiben. Da die für die Bekämpfung dieser Pathogene einsetzbaren Chitinasen und Glucanasen sehr oft vakuolär vorliegen, ist deren Bioverfügbarkeit im Interzellularraum naturgemäss nur gering. In diesem Falle wäre es daher wünschenswert, wenn man in der Lage wäre, diese Proteine gezielt in das extrazelluläre Kompartiment auszuschleusen, um so die Bioverfügbarkeit dieser Substanzen im Interzellularraum zu erhöhen und auf diese Weise eine wirksame Bekämpfung des Pilzpathogens zu ermöglichen.

Eine Sekretion in den extrazellulären Raum ist auch dann von Vorteil, wenn man beabsichtigt bestimmte Substanzen unter Verwendung von Zellkulturen herzustellen. In diesem Fall können die eingeschleusten Fremdproteine, da sie in den Extrazellularraum sezerniert werden, sehr einfach aus dem umgebenden Medium isoliert werden. Das bei intrazellulärer Produktion notwendige Aufbrechen der Zellen kann unterbleiben.

Bisher ist es jedoch nicht gelungen ein solches 'Targeting'-Signal für die Pflanzen-Vakuole zu identifizieren oder gar zu isolieren.

Tague und Chrispeels (1987) haben, ausgehend von der Annahme, dass in Pflanzen ein vergleichbares N-terminales 'Targeting'-Signal für die Einschleusung von Proteinen in die Vakuole verwirklicht sein könnte wie bei den Hefen, verschiedene Teile des Phytohemagglutinin-Moleküls aus Erbsen mit einem Reportergen verknüpft und in Hefen getestet. Dabei stellte sich heraus, dass im Hefewirt auch im Falle des Phytohemagglutinins der N-terminale Teil des Moleküls als 'Targeting'-Signal für die Vakuole fungiert.

Ein weiteres Vakuolenprotein, die basische β-1,3-Glucanase, wird mit einer C-terminalen Extension systhetisiert, die im Verlaufe der Reifung des Moleküls verloren geht. Das gleiche gilt auch für die Lectine aus Reis, Gerste und Weizen (Weizenkeimagglutinin). Die Funktion dieser Extension war bisher unbekannt.

Van den Bulcke et al. (Proc. Natl. Acad. Sci. USA, Vol 86, pp 2673-2677, 1989) verglichen die Aminosäuresequenzen tryptischer Fragmente einer vakuolären Tabak β-1,3-Glucanase mit der deduzierten Aminosäuresequenz der cDNA einer prozessierten β-1,3-Glucanase (Shinshi et al., 1988, Proc. Natl. Acad. Sci. USA, Vol 85, pp. 5541-5545) und stellten dabei fest, dass die vakuoläre β-1,3-Glucanase dasselbe C-terminale Fragment aufweist wie die ebenfalls untersuchten tryptischen Fragmente von sekretierten β-1,3-Glucanasen, und dass die deduzierte Aminosäuresequenz der prozessierten β-1,3-Glucanase noch eine C-terminale Extension von 22 Aminosäuren besitzt.

In EP 460753A2 ist beschrieben, dass das natürlicherweise vakuolär vorliegende Protein Osmotin durch Deletion des C-terminalen Endes in den extrazellulären Raum sekretiert wird. EP 460753A2 hat ein Prioritätsdatum, das vor dem Prioritätsdatum der vorliegenden Erfindung liegt, wurde aber erst nach Einreichung der vorliegende Erfindung publiziert.

Auch ein Vergleich der C-Termini verschiedener Proteine aus der Vakuole liess bisher keine offensichtlichen Homologien in diesem Bereich erkennen. Daher wurde bisher die Hypothese favorisiert, dass ähnlich den Hefen auch bei Pflanzen die entscheidende Signalwirkung vom N-Terminus dieser Vakuolenproteine ausgeht.

Eine der wesentlichen Aufgaben, die es im Rahmen dieser Erfindung zu lösen galt, bestand somit darin, Peptidfragmente ['Targeting'-Sequenzen], welche für die gezielte Einschleusung eines assoziierten Proteinmoleküls heterologen Ursprungs in die pflanzliche Vakuole verwendet werden können, bereitzustellen, um rekombinante DNA-Moleküle herzustellen, die, wenn sie in einer transgenen Pflanze exprimiert werden, zur gezielten Einschleusung des Expressionsprodukts in die Vakuole führen.

Diese Aufgabe konnte jetzt im Rahmen der vorliegenden Erfindung unter Anwendung z.T. bekannter Verfahrensmassnahmen überraschenderweise gelöst werden.

Im einzelnen betrifft die vorliegende Erfindung ein kurzes Peptidfragment, welches für die gezielte Einschleusung ['Targeting'] eines beliebigen, assoziierten Proteinmoleküls in die pflanzliche Vakuole verantwortlich ist sowie die besagtes Peptidfragment kodierende DNA. Bei besagtem assoziiertem Proteinmolekül handelt es sich um ein Protein heterologen Ursprungs, in Bezug auf die verwendete 'Targeting'-Sequenz.

Das erfindungsgemässe Peptidfragment ist aus der C-terminalen Region eines natürlicherweise vakuolär vorliegenden Chitinasemoleküls erhältlich. Die besagtes Peptidfragment kodierende DNA ist entsprechend aus der 3'-terminalen Region eines pflanzlichen Chitinasegens erhältlich ist.

Ganz besonders bevorzugt ist ein Peptidfragment, das als 'Targeting'-Signal für die pflanzliche Vakuole fungiert und das die folgende, in SEQ ID NO: 1 und 2 wiedergegebene Aminosäuresequenz aufweist: Arg Ser Phe Gly Asn Gly Leu Leu Val Asp Thr Met sowie die besagtes Peptidfragment kodierenden DNA-Sequenzen der in SEQ ID NO: 1 wiedergegebenen, allgemeinen Formel 
CGN/AGR TCN/AGW TTY GGN AAY GGN CTN/TTR TTR/CTN GTN GAY
ACN ATG TAA
 worin
- N: A oder G oder C oder T/U;
- R: G oder A;
- W: A oder T/U; und
- Y: T/U oder C bedeuten.

Bevorzugt sind dabei diejenigen DNA Sequenzen, die in der Mehrzahl die von der Pflanze bevorzugt verwendeten Kodons aufweisen.

Besonders bevorzugt ist eine DNA-Sequenz, die in einer im wesentlichen reinen Form vorliegt, und die beispielsweise aus dem 3'-terminalen Ende [C-terminale Extension] eines basischen Chitinasegens von *Nicotiana tabacum* L. c.v. Havana 425 Pflanzen erhältlich ist und im wesentlichen die folgende, in SEQ ID NO: 2 wiedergegebene DNA-Sequenz aufweist:

AGG TCT TTT GGA AAT GGA CTT TTA GTC GAT ACT ATG TAA

Weiterhin bevorzugt ist eine DNA-Sequenz, die in einer im wesentlichen reinen Form vorliegt, und die beispielsweise aus dem 3'-terminalen Ende [C-terminale Extension] eines basischen Glucanasegens von *Nicotiana tabacum* L. c.v. Havana 425 Pflanzen erhältlich ist und im wesentlichen die folgende, in SEQ ID NO: 12 wiedergegebene DNA-Sequenz aufweist:

GTC TCT GGT GGA GTT TGG GAC AGT TCA GTT GAA ACT AAT GCT ACT
GCT TCT CTC GTA AGT GAG ATG TGA

Das durch besagte DNA-Sequenz kodierte Peptidfragment, welches als 'Targeting'-Signal für die pflanzliche Vakuole fungiert und die folgende, in SEQ ID NO: 12 wiedergegebene Aminosäuresequenz aufweist, bildet ebenfalls einen Bestandteil der vorliegenden Erfindung:

Val Ser Gly Gly Val Trp Asp Ser Ser Val Glu Thr Asn Ala Thr
Ala Ser Leu Val Ser Glu Met

Ebenso beschrieben sind Derivate der oben näher bezeichneten DNA-Sequenzen, die diesen im wesentlichen homolog sind und noch die erfindungswesentlichen Eigenschaften aufweisen, d.h., die ein C-terminales Peptid kodieren, das als 'Targeting'-Signal für die pflanzliche Vakuole fungiert.

Eine DNA Sequenz ist einer zweiten DNA Sequenz dann im wesentlichen homolog, wenn wenigstens 60%, vorzugsweise wenigstens 80% und ganz besonders bevorzugt wenigstens 90% der aktiven Abschnitte der DNA Sequenzen einander homolog sind.

Bei besagten Derivaten der erfindungsgemässen DNA-Sequenzen kann es sich um natürlicherweise vorkommende Varianten oder Mutanten handeln oder aber insbesondere um solche, die gezielt mit Hilfe bekannter Mutationsverfahren herstellbar sind.

Unter einer Mutation sollen dabei sowohl die Deletion bzw. Insertion von einer oder mehreren Basen als auch ein Austausch von einer oder mehreren Basen oder aber eine Kombination dieser Massnahmen verstanden werden. Dies gilt insbesondere dann, wenn besagte Basensubstitution mit einer stillen Mutation einhergeht, die keinen Aminosäureaustausch zur Folge hat.
Beispiele solcher Mutanten, die ebenfalls ein Bestandteil der vorliegenden Erfindung sind, werden durch die im folgenden aufgelisteten und in SEQ ID NO: 3 bis 7 wiedergegebenen DNA-Sequenzen repräsentiert. Diese Mutanten sind mittels Oligonukleotid-vermittelter Mutagenese aus den in SEQ ID NO: 1 und 2 wiedergegebenen Stammsequenzen herstellbar und kodieren Peptid-Fragmente, die noch die gleichen 'Targeting'-Eigenschaften aufweisen wie die durch besagte Stammsequenzen kodierten Fragmente:
(a) GGA AAA GAT CTT TTA GTC GAT ACT ATG TAA
(b) GGA AAT GGA CTT TTA GTC AAT ACT ATG TAA
(c) GGA AAT GGA CTT TTA GTC CGT ACT ATG TAA
(d) A GAT CTT TTG GGA AAT GGA CTT TTA GTC GAT ACT ATG TAA
(e)

ATC GGT GAT CTT TTA GTC GAT ACT ATG TAA

Die Leerstellen der Sequenzen (a) bis (c) betreffen denjenigen Bereich der mutierten 'Targeting'-Seqeunz, der keine Unterschiede gegenüber der Ausgangssequenz aufweist.

Ebenso beschrieben sind Peptid-Fragmente, die von den oben genannten DNA-Sequenzen kodiert werden und die noch die gleichen 'Targeting'-Eigenschaften aufweisen wie die natürlicherweise vorliegenden, unmodifizierten Peptid-Fragmente, die durch besagte Stammsequenzen gemäss SEQ ID NO: 1 und 2 kodiert werden.

Besonders bevorzugt im Rahmen der Erfindung sind Peptid-Fragmente, die als 'Targeting'-Signal für die pflanzliche Vakuole fungieren und die folgenden, in SEQ ID NO: 3 bis 7 wiedergegebenen Aminosäuresequenzen aufweisen:
(a) Gly Lys Asp Leu Leu Val Asp Thr Met End
(b) Gly Asn Gly Leu Leu Val Asn Thr Met End
(c) Gly Asn Gly Leu Leu Val Arg Thr Met End
(d) Asp Leu Leu Gly Asn Gly Leu Leu Val Asp Thr Met End
(e)

Ile Gly Asp Leu Leu Val Asp Thr Met End

Die Leerstellen der Sequenzen (a) bis (c) betreffen denjenigen Bereich der mutierten 'Targeting'-Seqeunz, der keine Unterschiede gegenüber der Ausgangssequenz aufweist.

Diese beispielhafte Aufzählung ist in keiner Weise einschränkend, sondern sie dient lediglich dazu zu demonstrieren, dass Varianten der oben als bevorzugt genannten Sequenzen sehr einfach durch den Fachmann herstellbar sind, ohne dass dadurch die erfindungswesentliche Eigenschaft dieser Sequenzen verloren geht.

Ebenso beschrieben sind Fragmente oder Teilsequenzen, die aus den oben näher bezeichneten DNA Sequenzen oder aus Derivaten dieser DNA Sequenzen erhältlich sind und die noch die spezifischen Eigenschaften der Ausgangssequenzen aufweisen.

Besonders bevorzugt im Rahmen dieser Erfindung sind DNA-Fragmente, die aus der erfindungsgemässen DNA-Sequenz gemäss SEQ ID NO: 2 erhältlich sind und im wesentlichen die folgenden, in SEQ ID NO: 8 und 9 wiedergegebenen Nukleotid-Sequenzen aufweisen:

CTT TTA GTC GAT ACT ATG TAA

GGA CTT TTA GTC GAT ACT ATG TAA

Die oben wiedergegebenen DNA-Sequenzen kodieren Peptidfragmente, die als 'Targeting'-Signal für die pflanzliche Vakuole fungieren und die folgende, in SEQ ID NO: 8 und 9 wiedergegebene Aminosäure-Sequenaufweisen:

Leu Leu Val Asp Thr Met End

Gly Leu Leu Val Asp Thr Met End

Die genannten Peptidfragmente sind somit ebenfalls ein Bestandteil der vorliegenden Erfindung.

Einen weiteren Gegenstand der vorliegenden Erfindung bilden rekombinante DNA Moleküle worin eine DNA-Sequenz, die aus der 3'-terminalen Region eines Gens erhältlich ist, das für ein natürlicherweise vakuolär vorliegendes Proteinmolekül kodiert und worin besagte DNA Sequenz ein C-terminales Peptid [C-terminale Extension] kodiert, das für die gezielte Einschleusung ['Targeting'] eines assoziierten Genproduktes heterologen Ursprungs in die pflanzliche Vakuole verantwortlich ist, und wobei besagte DNA-Sequenz nicht die ca. 20 C-terminalen Aminosäuren des primären Translationsprodukts von Wild-Typ Osmotin kodiert, mit einem heterologen Gen operabel verknüpft ist. Die efindungsgemässen rekombinanten DNA Moleküle können mit in pflanzlichen Zellen aktiven Expressionssignalen und gegebenenfalls weiteren kodierenden und/oder nicht kodierenden Sequenzen der 3'- und/oder 5'-Region operabel verknüpft sein, sodass bei Transformation in einen pflanzlichen Wirt eine gezielte Einschleusung des Expressionsproduktes in die pflanzliche Vakuole erfolgt.

Dabei ist es vorteilhaft, wenn die exprimierbare DNA in der 5'-terminalen Region eine Sequenz enthält oder aber mit einer solchen Sequenz verknüpft wird, die für ein in der pflanzlichen Zelle funktionsfähiges, N-terminales Signalpeptid kodiert. Darüberhinaus können weitere Sequenzabschnitte im DNA Molekül vorliegen, welche für Peptidfragemente kodieren, die insgesamt dazu beitragen, die Kompetenz für eine Aufnahme in die Vakuole zu verbessern wie beispielsweise das von Matsuoka K und Nakamura K in der N-terminalen Extension des Sporamin aufgefundene Propeptidfragment [Matsuoka K und Nakamura K (1991)].

Weiterhin umfasst von der vorliegenden Erfindung sind Klonierungs-, Transformations- und Expressionsvektoren, die besagtes erfindungsgemässes rekombinantens DNA-Molekül enthalten sowie die mit besagten Vektoren transformierten Wirtsorganismen.

Einen weiteren Gegenstand dieser Erfindung bilden sog. 'Shuttle'-Vektoren oder binäre Vektoren, die besagtes erfindungsgemässes rekombinantens DNA-Molekül enthalten und die in der Lage sind sowohl in *E. coli* als auch in *A. tumefaciens* stabil zu replizieren.

Unter den Wirtsorganismen sind insbesondere pflanzliche Wirte ausgewählt aus der Gruppe bestehend aus pflanzlichen Protoplasten, Zellen, Kalli, Geweben, Organen, Zygoten, Embryonen, Pollen und/oder Samen bevorzugt sowie insbesondere auch ganze, vorzugsweise fertile Pflanzen, die mit besagter rekombinanter DNA transformiert sind. Ganze Pflanzen können dabei entweder direkt als solche mit dem erfindungsgemässen, rekombinanten DNA Molekül transformiert oder aber aus zuvor transformierten Protoplasten, Zellen und/oder Geweben *via* Regeneration gewonnen werden.

Ganz besonders bevorzugt sind transgene Pflanzen, insbesondere aber transgene fertile Pflanzen, die eines der erfindungsgemässen Konstrukte enthalten und bei denen das exprimierte Genprodukt, wie gewünscht, in der Vakuole vorliegt.

Ebenso umfasst von der vorliegenden Erfindung ist jegliches Vermehrungsgut einer transgenen Pflanze, wobei besagte transgene Pflanze entweder durch direkte Transformation mit einem der erfindungsgemässen, rekombinanten DNA Moleküle entstanden sein kann oder aber aus zuvor transformierten Protoplasten, Zellen, Kalli, Geweben, Organen, Zygoten, Embryonen, Pollen und/oder Samen *via* Regeneration gewonnen wird, ohne jedoch darauf beschränkt zu sein, und wobei das Vermehrungsgut ein erfindungsgemässes rekombinantes DNA Molekül enthält.

Unter Vermehrungsgut soll im Rahmen dieser Erfindung jegliches pflanzliche Material verstanden werden, das sich sexuell oder asexuell oder aber *in vitro* oder *in vivo* vermehren lässt, wobei Protoplasten, Zellen, Kalli, Gewebe, Organe, Eizellen, Zygoten, Embryonen, Pollen oder Samen, die von einer erfindungsgemässen transgenen Pflanze erhalten werden können, bevorzugt sind. Einen weiteren Gegenstand dieser Erfindung bildet die Nachkommenschaft besagter Pflanzen, sowie Mutanten und Varianten davon, einschliesslich derjenigen, die sich von Pflanzen ableiten, welche durch somatische Zellfusion, genetische Modifikation oder Mutantenselektion erhalten werden.

Weitere Gegenstände der vorliegenden Erfindung betreffen Verfahren
(a) zur Herstellung einer der erfindungsgemässen DNA-Sequenzen;
(b) zur Herstellung der erfindungsgemässen rekombinanten DNA-Moleküle, welche die obige erfindungsgemässe DNA-Sequenz in operabler Verknüpfung mit einer beliebigen, exprimierbaren DNA Sequenz heterologen Ursprung enthalten, wobei besagte DNA-Sequenz vorzugsweise unter der regulatorischen Konstrolle pflanzlicher Expressionssignale steht;
(c) zur Herstellung von Klonierungs-, Transformations- und/oder Expressionsvektoren, welche besagtes erfindungsgemässes rekombinantes DNA-Molekül enthalten;
(d) zur Herstellung transformierter Wirtsorganismen, insbesondere pflanzlicher Wirte ausgewählt aus der Gruppe bestehend aus pflanzlichen Protoplasten, Zellen, Kalli, Geweben, Organen, Zygoten, Embryonen, Pollen und/oder Samen sowie insbesondere auch ganze, vorzugsweise fertile Pflanzen;
(e) zur Herstellung von Vermehrungsgut ausgehend von transformiertem Pflanzenmaterial, insbesondere aber zur Herstellung von sexueller und asexueller Nachkommenschaft.

Ebenfalls beschrieben ist ein Verfahren zur zielgerichteten Einschleusung von Expressionsprodukten in die pflanzliche Vakuole, das im wesentlichen dadurch gekennzeichnet ist, dass man
(a) zunächst die für eine gezielte Einschleusung in die Vakuole verantwortliche DNA-Sequenz aus einer geeigneten Quelle isoliert oder diese mit Hilfe bekannter Verfahren synthetisiert;
(b) besagte DNA-Sequenz in das 3'-terminal Ende einer beliebigen exprimierbaren DNA-Sequenz operabel inseriert;
(c) das fertige Konstrukt in einen Pflanzenexpressionsvektor unter die Kontrolle von in Pflanzen aktiven Expressionssignalen einkloniert; und
(d) besagten Expressionsvektor in einen pflanzlichen Wirt transformiert und dort exprimiert.

Dabei ist es vorteilhaft, wenn die exprimierbare DNA in der 5-terminalen Region eine Sequenz enthält oder aber mit einer solchen Sequenz operabel verknüpft wird, die für ein in der pflanzlichen Zelle funktionsfähiges, N-terminales Signalpeptid kodiert. Darüberhinaus können weitere Sequenzabschnitte im DNA Molekül vorliegen, welche für Peptidfragemente kodieren, die insgesamt dazu beitragen, die Kompetenz für eine Aufnahme in die Vakuole zu verbessern wie beispielsweise das von Matsuoka K und Nakamura K in der N-terminalen Extension des Sporamin aufgefundene Propeptidfragment [Matsuoka K und Nakamura K (1991)].

Im Verlaufe der im Rahmen dieser Erfindung durchgeführten Untersuchungen hat es sich gezeigt, dass Proteine, die natürlicherweise eine der erfindungsgemässen 'Targeting'-Sequenzen enthalten und die daher normalerweise in die Vakuole eingeschleust werden, bei Verlust der erfindungsgemässen Signalsequenz in den extrazellulären Raum sezerniert werden.

Ein weiterer Gegenstand der Offenbarung betrifft daher ein Verfahren zur Ausschleusung von Proteinen, die natürlicherweise eine 'Targeting'-Sequenz enthalten und die daher normalerweise in eines der zellulären Kompartimente, insbesondere aber in die Vakuole, eingeschleust werden, in den Extrazellularraum der Pflanze, das im wesentlichen dadurch gekennzeichnet ist, dass man
(a) eine für ein solches Protein, insbesondere aber für eine Vakuolen-Protein, kodierende DNA-Sequenz isoliert;
(b) die für die Einschleusung in das jeweilige zelluläre Kompartiment verantwortliche 'Targeting'-Sequenz am C-terminalen Ende aus dem offenen Leseraster entfernt, z.B. durch Einfügen eines Stopp-Kodons unmittelbar vor der C-terminalen Extension oder durch Entfernen der C-terminalen Extension;
(c) besagte mutierte DNA-Sequenz in einen geeigneten Pflanzenexpressionsvektor einspleisst; und
(d) das fertige Konstrukt in einen pflanzlichen Wirt transformiert.

### Definitionen

In der folgenden Beschreibung werden eine Reihe von Ausdrücken verwendet, die in der rekombinanten DNA Technologie, sowie in der Pflanzengenetik gebräuchlich sind. Um ein klares und einheitliches Verständnis der Beschreibung und der Ansprüche sowie des Umfangs, der den besagten Ausdrücken zukommen soll, zu gewährleisten, werden die folgenden Definitionen aufgestellt.

Pflanzliches Material: In Kultur oder als solches lebensfähige pflanzliche Teile wie Protoplasten, Zellen, Kallus, Gewebe, Embryonen, Pflanzenorgane, Knospen, Samen, u.a. sowie ganze Pflanzen.

Pflanzenzelle: Strukturelle und physiologische Einheit der Pflanze, bestehend aus einem Protoplasten und einer Zellwand.

Protoplast: Aus Pflanzenzellen oder -geweben isolierte zellwandlose "nackte" Pflanzenzelle mit der Potenz, zu einem Zellklon oder einer ganzen Pflanze zu . regenerieren.

Pflanzengewebe: Gruppe von Pflanzenzellen, die in Form einer strukturellen und funktionellen Einheit organisiert sind.

Pflanzenorgan: Strukturelle und funktionelle Einheit aus mehreren Geweben, wie beispielsweise-Wurzel, Stamm, Blatt oder Embryo.

Heterologe(s) Gen(e) oder DNA: Eine DNA Sequenz, die ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA bezeichnet.

Homologe(s) Gen(e) oder DNA: Eine DNA Sequenz, die ein spezifisches Produkt oder Produkte kodiert oder eine biologische Funktion erfüllt und die aus der gleichen Spezies stammt, in welche das besagte Gen eingeschleust wird.

Synthetische(s) Gen(e) oder DNA: Eine DNA-Sequenz, die ein spezifisches Produkt oder Produkte kodiert oder eine biologische Funktion erfüllt und die auf synthetischem Wege hergestellt sind.

Pflanzen-Promotor: Eine. Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.

Terminations-Sequenz: DNA-Sequenz am Ende einer Transkriptions-Einheit, die das Ende des Transkriptionsvorganges signalisiert.

Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.

3'/5' nicht-translatierte Region: Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B. die Ribosomenbindungsstelle (5') oder das Polyadenylierungssignal (3').

DNA-Klonierungsvektor: Klonierungs-Vehikel, wie z.B. ein Plasmid oder ein Bakteriophage, das alle Signal-Sequenzen enthält, die für die Klonierung einer inserierten DNA in einer geeigneten Wirtszelle notwendig sind.

DNA-Expressions-Vektor: Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

DNA-Transfer-Vektor: Transfer-Vehikel, wie z.B. ein Ti-Plasmid oder ein Virus, das die Einschleusung von genetischem Material in eine geeignete Wirtszelle ermöglicht.

Mutanten, Varianten transgener Pflanzen: Spontan oder aber artifiziell, unter Anwendung bekannter Verfahrensmassnahmen, wie z.B. UV-Behandlung, Behandlung mit mutagenen Agentien etc. entstandener Abkömmling einer transgenen Pflanze, der noch die erfindungswesentlichen Merkmale und Eigenschaften der Ausgangspflanze aufweist.

Im wesentliche reine DNA Sequenz: Eine DNA Sequenz, die in im wesentlichen reiner Form aus einer natürlichen oder nicht-natürlichen Quelle isoliert wird. Eine solche Sequenz kann in einem natürlichen System vorliegen, wie beispielweise in Bakterien, Viren oder in pflanzlichen oder tierischen Zellen oder sie kann altemativ dazu in Form synthetischer DNA oder von cDNA zur Verfügung gestellt werden.
Im wesentlichen reine DNA wird in der Regel in Form eines Vektors isoliert, welcher die besagte DNA als Insert enthält. Im wesentlichen rein heisst, dass andere DNA Sequenzen nur in vernachlässigbarem Umfang vorliegen und beispielsweise weniger als 5%, vorzugsweise weniger als 1% und ganz besonders bevorzugt weniger als 0.1% ausmachen. Solche Sequenzen und die diese Sequenzen enthaltenden Vektoren liegen im allgemeinen in wässriger Lösung vor und zwar in einer Pufferlösung oder in einem der üblicherweise verwendeten Kulturmedien.

Im Rahmen der vorliegenden Offenbarung konnte erstmals eine konkrete DNA-Sequenz identifiziert und isoliert werden, die ein kurzes Peptidfragment kodiert, das für die gezielte Einschleusung eines beliebigen, assoziierten Genproduktes in die pflanzliche Vakuole verantwortlich ist. Als Ausgangsmaterial für die Isolierung besagter 'Targeting' DNA-Sequenzer besonders geeignet sind cDNA und/oder genomische DNA Klone von natürlicherweise vakuolär vorliegenden Proteinen, wie z.B. ein pflanzlicher Chitinase - oder Glucanaseklon.

Die vorliegende Offenbarung betrifft daher in erster Linie eine neue, im wesentlichen reine DNA Sequenz, welche aus der 3'-terminalen Region eines Gens erhältlich ist, das für ein natürlicherweise vakuolär vorliegendes Protein kodiert, und die in operabler Verknüpfung mit einer beliebigen, exprimierbaren DNA zu einem Genprodukt führt, das gezielt in die pflanzliche Vakuole eingeschleust wird, sowie Mutanten und Varianten davon.

Besonders bevorzugt im Rahmen dieser Erfindung ist dabei eine DNA Sequenz, die aus der 3'-terminalen Region eines pflanzlichen Chitinasegens erhältlich ist. Ebenfalls beschrieben ist eine DNA-Sequenz, die aus der 3'-terminale Region eines pflanzlichen Glucanasegens erhältlich ist.

Bei der Isolierung eines geeigneten Gens, insbesondere aber eines geeigneten Chitinase- oder Glucanasegens geht man vorzugsweise von genomischen oder cDNA Genbibliotheken aus, die nach gängigen, dem Fachman auf diesem Gebiet bestens bekannten Routineverfahren hergestellt werden können. Die grundsätzlichen Verfahren zur Herstellung von genomischen oder cDNA Genbibliotheken sind beispielsweise bei Maniatis *et al* (1982) im Detail beschrieben, während Angaben zur Umsetzung und Anwendung dieser Verfahren auf pflanzliche Systeme z.B. der Referenz von Mohnen (1985) entnommen werden können.

Genomische DNA sowie cDNA kann auf unterschiedliche Weise gewonnen werden. Genomische DNA beispielsweise kann mit Hilfe bekannter Verfahren aus geeigneten Zellen extrahiert und gereinigt werden.

In einer spezifischen Ausführungsform der vorliegenden Offenbarung geht man bei der Herstellung von cDNA in der Regel von mRNA aus, die aus ausgesuchten Zellen oder Geweben isoliert werden kann, insbesondere aber aus Zellen oder Geweben die bekanntermassen hohe Konzentrationen an natürlicherweise vakuolär vorliegenden Proteien, insbesondere aber hohe Chitinase- bzw. Glucanasekonzentrationen, aufweisen. Die isolierte mRNA kann dann im Rahmen einer reversen Transkription als Matritze für die Herstellung einer korrespondierenden cDNA verwendet werden.

Erfindungsgemäss besonders bevorzugt als Ausgangsmaterial für die Herstellung von cDNA sind pflanzliche Zellen oder Gewebe oder sonstiges geeignetes Pflanzenmaterial, das zuvor mit Hilfe geeigneter Massnahmen zur Produktion hoher Chitinasespiegel angeregt wurde. Dies lässt sich beispielsweise dadruch erreichen, dass man kultivierte Zellen oder Gewebe oder sonstiges geeignetes Pflanzenmaterial auf ein Hormon-freies Medium überimpft und dort über einen geeigneten Zeitraum hinweg kultiviert, der für die Induktion hoher Chitinasespiegel ausreichend ist.

Besonders bevorzugt im Rahmen dieser Erfindung ist ein Basismedium, das die von Linsmaier und Skoog (1965) vorgeschlagene Salz- und Thiamin-HCl Konzentration aufweist (LS-Medium).

Die Verfahren zur Isolierung von Poly (A⁺) RNA und zur Herstellung von cDNA sind dem Fachmann bekannt und werden in der Folge im Rahmen der Ausführungsbeispiele im Detail beschrieben.

Die extrahierten und gereinigten DNA Präparationen werden anschliessend für die nachfolgende Klonierung in Fragmente zerlegt. Die Fragmentierung der zu klonierenden genomischen DNA oder der cDNA auf eine für die Insertion in einen Klonierungs-Vektor geeignete Grösse kann entweder durch mechanisches Scheren oder aber vorzugsweise durch Schneiden mit geeigneten Restriktionsenzymen erfolgen. Geeignete Klonierungsvektoren, die bereits routinemässig für die Herstellung von genomischen und/oder cDNA Genbibliotheken verwendet werden umfassen beispielsweise Phagenvektoren, wie die λ-Charon Phagen oder aber bakterielle Vektoren, wie das *E. coli* Plasmid pBR322. Weitere geeignete Klonierungsvektoren sind dem Fachmann bekannt.

Aus den auf diese Weise hergestellten Genbibliotheken können dann im Rahmen eines Screening-Programmes geeignete Klone, die das gewünschte Gen, wie beispielsweise ein Chitinase- oder ein Glucanasegen, oder Teile davon enthalten, z.B. mit Hilfe geeigneter Oligonukleotidproben (Probenmolekül) aufgespürt und anschliessend isoliert werden. Zum Aufspüren geeigneter Klone stehen verschiedene Methoden zur Verfügung wie z.B. die differentielle Koloniehybridisierung oder die Plaquehybridisierung. Ebenso anwendbar sind immunologische Nachweismethoden, die auf einer Identifizierung der spezifischen Translationsprodukte beruhen.

Als Probenmolekül kann beispielsweise ein zuvor bereits isoliertes DNA Fragment aus dem gleichen oder aber einem strukturell verwandten Gen verwendet werden, das in der Lage ist, mit dem korrespondierenden Sequenzabschnitt innerhalb des zu identifizierenden, gewünschten Gens zu hybridisieren.

Sofern die Aminosäuresequenz des zu isolierenden Gens oder aber zumindest Teile dieser Sequenz bekannt sind, kann aufgrund dieser Sequenzinformation eine entsprechende korrespondierende DNA Sequenz entworfen werden. Da der genetische Kode bekanntlich degeneriert ist, können in der Mehrzahl der Fälle für ein und dieselbe Aminosäure verschiedene Kodons verwendet werden. Dies führt dazu, dass, abgesehen von wenigen Ausnahmefällen, in der Regel eine bestimmte Aminosäuresequenz von einer ganzen Reihe einander ähnlicher Oligonukleotide kodiert werden kann. Dabei ist jedoch zu beachten, dass nur ein Mitglied aus dieser Reihe von Oligonukleotiden tatsächlich mit der entsprechenden Sequenz innerhalb des gesuchten Gens übereinstimmt. Um von vorneherein die Anzahl möglicher Oligonukleotide zu begrenzen kann beispielsweise auf die von Lathe R *et al* (1985) aufgestellten Regeln für die Verwendung von Kodons zurückgegriffen werden, welche die Häufigkeit, mit der ein bestimmtes Kodon in eukaryontischen Zellen tatsächlich verwendet wird, berücksichtigen.

Aufgrund dieser Informationen lassen sich somit Oligonukleotidmoleküle entwerfen, die als Probenmoleküle für die Identifizierung und Isolierung geeigneter Klone verwendet werden können, indem man besagte Probenmoleküle in einem der zuvor beschriebenen Verfahren mit genomischer DNA oder cDNA hybridisiert.

Um die Nachweisbarkeit des gewünschten Gens, wie z.B. eines Chitinase- oder Glucanase-kodierenden Gens, zu erleichtern, kann das zuvor beschriebene DNA-Probenmolekül mit einer geeigneten, leicht nachweisbaren Gruppe markiert werden. Unter einer nachweisbaren Gruppe soll im Rahmen dieser Beschreibung jedes Material verstanden werden, das eine bestimmte, leicht identifizierbare, physikalische oder chemische Eigenschaft aufweist.

Solche Materialien finden insbesondere auf dem Gebiet der Immunoassays bereits eine breite Anwendung und sind in der Mehrzahl der Fälle auch in der vorliegenden Anmeldung verwendbar. Insbesondere seien an dieser Stelle enzymatisch aktive Gruppierungen genannt, wie z.B. Enzyme, Enzymsubstrate, Coenzyme und Enzyminhibitoren, desweiteren fluoreszierende und lumineszierende Agentien, Chromophore sowie Radioisotope, wie z.B. ³H, ³⁵S, ³²P, ¹²⁵I und ¹⁴C. Die leichte Nachweisbarkeit dieser Marker beruht einerseits auf ihren inhärent vorhandenen physikalischen Eigenschaften (z.B. Fluoreszenzmarker, Chromophore, Radioisotope), andererseits auf ihren Reaktions- und Bindungseigenschaften (z.B. Enzyme, Substrate, Coenzyme, Inhibitoren).

Weiterhin geeignet als Probenmolekül ist eine einzelsträngige cDNA, die sich von einer poly(A)+ RNA ableitet, welche ihrerseits aus einem zur Produktion hoher Chitinase- bzw. Glucanasespiegel induzierten Gewebe oder einer Zelle isoliert wird.

Allgemeine Verfahren betreffend Hybridisierung sind beispielsweise bei Maniatis T *et al* (1982) und bei Haymes BT *et al* (1985) beschrieben.

Diejenigen Klone innerhalb der zuvor beschriebenen Genbibliotheken, die in der Lage sind mit einem Probenmolekül zu hybridiseren und die sich mit Hilfe eines der oben erwähnten Nachweisverfahren identifizieren lassen, können dann weiter analysiert werden um Ausmass und Natur der kodierenden Sequenz im einzelnen zu bestimmten.

Ein alternatives Verfahren zur Klonierung von Genen, insbesondere aber von Chitinase- oder Glucanasegenen, beruht auf der Konstruktion einer Genbibliothek, die sich aus Expressionsvektoren aufbaut. Dabei wird in Analogie zu den zuvor bereits beschriebenen Verfahren zunächst genomische DNA, vorzugsweise aber cDNA, aus einer Zelle oder einem Gewebe isoliert, das in der Lage ist ein gewünschtes Genprodukt -im vorliegenden Fall Chitinase oder Glucanase- zu exprimieren und anschliessend in einen geeigneten Expressionsvektor eingespleisst. Die auf diese Weise hergestellten Genbibliotheken können dann mit Hilfe geeigneter Massnahmen, vorzugsweise unter Verwendung von Antikörpern, wie beispielsweise anti-Chitinase oder antiGlucanase Antikörpern, gescreent und diejenigen Klone ausgelesen werden, die das gewünschte Gen oder aber zumindest einenen Teil dieses Gens als Insert enthalten.

Mit Hilfe der zuvor beschriebenen Verfahren ist es somit möglich ein Gen, welches für eine natürlicherweise vakuolär vorliegendes Genprodukt kodiert, wie beispielsweise ein pflanzliches Chitinase- oder Glucanasegen, insbesondere aber ein basisches Chitinase- oder Glucanasegen aus Tabakpflanzen, zu isolieren, das in seiner C-terminalen Extension eine DNA-Sequenz besitzt, die in operabler Verknüpfung mit einem beliebigen heterologen Strukturgen zu einer gezielten Einschleusung des Genproduktes in die Vakuole des transformierten pflanzlichen Materials führt.

Zur weiteren Charakterisierung werden die auf die zuvor beschriebene Weise gereinigten und isolierten DNA Sequenzen einer Sequenzanalyse unterzogen. Dabei wird die zuvor isolierte DNA zunächst mit Hilfe geeigneter Restriktionsenzyme in Fragmente zerlegt und anschliessend in geeignete Klonierungsvektoren, wie z.B. die M13 Vektoren mp18 und mp19 einkloniert. Die Sequenzierung wird in 5' → 3' Richtung durchgeführt, wobei vorzugsweise die Dideoxynucleotid-Kettenterminationsmethode nach Sanger [Sanger *et al*, 1977] oder das Verfahrens nach Maxam and Gilbert [Maxam and Gilbert, 1980] Anwendung findet. Um Fehler bei der Sequenzierung zu vermeiden ist es vorteilhaft, wenn man beide DNA Stränge parallel sequenziert. Die Analyse der Nukleotid- und der korrespondierenden Aminosäuresequenz wird vorzugsweise Computer-unterstützt, unter Verwendung einer geeigneten, kommerziell erhältlichen Computersoftware [z.B. GCG Software der University of Wisconcin], durchgeführt.

Aus den auf die zuvor beschriebene Weise hergestellten DNA Klonen, insbesondere aber aus Chitinase- oder Glucanaseklonen, können dann sehr einfach, unter Anwendung allgemein bekannter Verfahrensmassnahmen, DNA Sequenzen isoliert werden, die ein Peptid kodieren, das für die gezielte Einschleusung eines assoziierten Genprodukts heterologen Ursprung in die pflanzliche Vakuole verantwortlich ist.

Besonders bevorzugt im Rahmen dieser Erfindung ist eine DNA-Sequenz, die in einer im wesentlichen reinen Form vorliegt und die beispielsweise aus dem 3'-terminalen Ende [C-terminale Extension] eines basischen Chitinasegens von *Nicotiana tabacum* L. c.v. Havana 425 Pflanzen erhältlich ist, und im wesentlichen die folgende, in SEQ ID NO: 2 wiedergegebene DNA-Sequenz aufweist:

AGG TCT TTT GGA AAT GGA CTT TTA GTC GAT ACT ATG TAA

Die oben wiedergegebene DNA-Sequenz kodiert ein Peptidfragment, das in operabler Verknüpfung mit einem Proteinmolekül als 'Targeting'-Signal für die pflanzliche Vakuole fungiert und das die folgende, in SEQ ID NO: 2 wiedergegebene Aminosäuresequenz aufweist:

Arg Ser Phe Gly Asn Gly Leu Leu Val Asp Thr Met

Das genannte Peptidfragment ist ebenfalls ein Bestandteil der vorliegenden Erfindung.

Weiterhin beschrieben ist eine DNA-Sequenz, die in einer im wesentlichen reinen Form vorliegt und die beispielsweise aus dem 3'-terminalen Ende [C-terminale Extension] eines basischen Glucanasegens von *Nicotiana tabacum* L. c.v. Havana 425 Pflanzen erhältlich ist, und im wesentlichen die folgende, in SEQ ID NO: 12 wiedergegebene DNA-Sequenz aufweist:

GTC TCT GGT GGA GTT TGG GAC AGT TCA GTT GAA ACT AAT GCT ACT
GCT TCT CTC GTA AGT GAG ATG TGA

Das durch besagte DNA-Sequenz kodierte Peptidfragment, das in operabler Verknüpfung mit einer exprimierbaren DNA als 'Targeting'-Signal für die pflanzliche Vakuole fungiert, weist die folgende, in SEQ ID NO: 12 wiedergegebene Aminosäuresequenz auf:

Val Ser Gly Gly Val Trp Asp Ser Ser Val Glu Thr Asn Ala Thr
Ala Ser Leu Val Ser Glu Met

Es versteht sich von selbst, dass diese nunmehr in ihrer Basenabfolge bekannten, DNA-Seqeuenzen nicht jedesmal wieder neu aus einem geeigneten Chitinase- bzw. Glucanasegen isoliert werden müssen, sondern dass diese selbstverständlich jederzeit sehr einfach mit Hilfe bekannter chemischer Verfahren synthetisch hergestellt werden können. Geeignete Verfahren zur Synthese kurzer DNA Oligonukleotide, wie z.B. die Phosphotriester- oder die Phosphitmethode sind dem Fachman bekannt. Heute sind die Oligonucleotidsynthesen zum grösstenteil mechanisiert und automatisiert, sodass kurze DNA-Fragmente innerhalb kurzer Zeit herstellbar sind.

Das gleiche gilt auch für die aus der Basensequenz unmittelbar ableitbaren Aminosäuresequenzen der korrespondierenden 'Targeting'-Peptide.

Durch Deletion, Insertion oder Austausch einer oder mehrerer Basenpaare in den oben genannten DNA-Sequenzen können daher sehr einfach Varianten oder Mutanten dieser Sequenzen hergestellt und auf ihre Tauglichkeit als 'Targeting'-Sequenz überprüft werden.

Im Rahmen der vorliegenden Beschreibung wird eine Vielzahl von Sequenzen offenbart, die sehr einfach aus einer natürlicherweise vorliegenden Ausgangssequenz mittels Mutagenese herstellbar sind.

Im einzelnen kann man dabei so vorgehen, dass man zunächst ein Gen identifiziert und isoliert, dass eine der oben genannten DNA-Sequenzen enthält. Dieses Gen, insbesondere aber die 3'-terminale 'Targeting'-Sequenz, kann dann nach Einspleissen in einen geeigneten Klonierungsvektor mit Hilfe bekannter Verfahrensmassnahmen modifiziert werden. Besonders geeignet zur Herstellung spezifischer Mutanten ist die sog. Oligonukleotid-vermittelte Mutagenese. Dabei werden kurze Oligonukleotid-fragmente synthetisiert, die der Wildtyp-Sequenz zwar im wesentlichen homolog sind, sich jedoch in einzelnen Nukleotiden von dieser unterscheiden. Bei besagten Unterschieden kann es sich um Insertionen, Deletionen, Inversionen oder um einen Austausch eines oder mehrerer Nukleotide oder aber um eine Kombination der zuvor erwähnten Verfahrensmassnahmen handeln. Diese mutierten Fragmente werden dann im Rahmen allgemein bekannter Verfahren gegen die homologen Gegenstücke auf dem Wildtypgen ausgetauscht. Das fertige Konstrukt kann dann, wie zuvor beschrieben, in einen geeigneten Pflanzenexpressionsvektor einkloniert und in eine Pflanze transformiert werden.

Die Mutagenisierung bestimmter DNA-Fragmerite kann aber auch vorzugsweise unter Anwendung der Polymerase Kettenreaktion ['polymerase chain reaction'; PCR] durchgeführt werden. Bei diesem *in vitro* Verfahren kommen chemisch synthetisierte Oligonukleotide zum Einsatz, die in der Regel aus den Randbereichen des zu mutierenden DNA-Fragmentes stammen und strangspezifisch sind. Unter geeigneten Bedingungen kommt es zu einer Hybridisierung der Oligonukleotide mit den komplementären Bereichen auf den durch Denaturierung erzeugten DNA-Einzelsträngen. Die auf diese Weise entstandenen doppelsträngigen Bereiche dienen als Primer für die sich anschliessende Polymerasereaktion.
Dabei können neben DNA-Polymerasen aus *E.coli* insbesondere hitzestabile Polymerasen aus thermophilen Bakterien wie z.B. *Thermus aquaticus* verwendet werden.

Die vorliegende Beschreibung beschränkt sich daher nicht auf die oben näher spezifizierte Basenabfolge, sondern offenbart darüberhinaus auch alle Mutanten und/oder Varianten dieser DNA Sequenzen, die durch Deletion bzw. Insertion von einer oder mehreren Basen oder aber insbesondere durch Austausch von einer oder mehreren Basen herstellbar sind und die noch die spezifischen, erfindungsgemässen Eigenschaften der Ausgangssequenzen aufweisen.

Besonders bevorzugt im Rahmen dieser Erfindung sind die folgenden Varianten, die aus den in SEQ ID NO: 1 und 2 wiedergegebenen Ausgangssequenzen durch Oligonukleotid-vermittelte Mutagenese herstellbar sind und die Peptide kodieren, welche noch die gleichen 'Targeting'-Eigenschaften aufweisen wie die durch besagte Ausgangssequenzen kodierten Peptide [SEQ ID NO: 3 bis 7]:
(a) GGA AAA GAT CTT TTA GTC GAT ACT ATG TAA
(b) GGA AAT GGA CTT TTA GTC AAT ACT ATG TAA
(c) GGA AAT GGA CTT TTA GTC CGT ACT ATG TAA
(d) A GAT CTT TTG GGA AAT GGA CTT TTA GTC GAT ACT ATG TAA
(e)

ATC GGT GAT CTT TTA GTC GAT ACT ATG TAA

Die Leerstellen der Sequenzen (a) bis (c) betreffen denjenigen Bereich der mutierten 'Targeting'-Seqeunz, der keine Unterschiede gegenüber der Ausgangssequenz aufweist.

Die auf die zuvor beschriebene Weise isolier- bzw. herstellbaren DNA-Sequenzen können jetzt dazu verwendet werden um homologe DNA Sequenzen gleicher Funktion aufzusprüren, indem man beispielsweise zunächst genomische oder cDNA Genbibliotheken herstellt und diese in der zuvor beschriebenen Weise, unter Verwendung der oben genannten DNA-Sequenzen als Probenmoleküle, auf das Vorhandensein homologer DNA Sequenzen untersucht, die in der Lage sind mit diesen Probenmolekülen zu hybridisieren.

Diese Verfahren zum Auffinden homologer DNA Sequenzen gleicher Funktion bilden ebenfalls einen Bestandteil der vorliegenden Offenbarung.

Ebenso sind Peptid-Fragmente beschrieben, die von den oben genannten DNA-Sequenzen kodiert werden und die noch die gleichen 'Targeting'-Eigenschaften aufweisen wie die natürlicherweise vorliegenden, unmodifizierten Peptid-Fragmente, die durch besagte, in SEQ ID NO: 1 und 2 wiedergegebenen Stammsequenzen kodiert werden.

Besonders bevorzugt sind Peptid-Fragmente, die als 'Targeting'-Signal für die pflanzliche Vakuole fungieren und die folgenden Aminosäuresequenzen aufweisen [SEQ ID NO: 3 bis 7]:
(a) Gly Lys Asp Leu Leu Val Asp Thr Met End
(b) Gly Asn Gly Leu Leu Val Asn Thr Met End
(c) Gly Asn Gly Leu Leu Val Arg Thr Met End
(d) Asp Leu Leu Gly Asn Gly Leu Leu Val Asp Thr Met End
(e)

Ile Gly Asp Leu Leu Val Asp Thr Met End

Die Leerstellen der Sequenzen (a) bis (c) betreffen denjenigen Bereich der mutierten 'Targeting'-Seqeunz, der keine Unterschiede gegenüber der Ausgangssequenz aufweist.

Ebenso beschrieben sind Fragmente oder Teilsequenzen, die aus den oben näher bezeichneten DNA Sequenzen oder aus Derivaten dieser DNA Sequenzen erhältlich sind und die noch die spezifischen Eigenschaften der Ausgangssequenzen aufweisen.

Besonders bevorzugt im Rahmen dieser Erfindung sind DNA-Fragmente, die aus der erfindungsgemässen DNA-Sequenz erhältlich sind und im wesentlichen die folgende, in SEQ ID NO: 8 und 9 wiedergegebene Nukleotid Sequenzen aufweisen:

CTT TTA GTC GAT ACT ATG TAA

GGA CTT TTA GTC GAT ACT ATG TAA

Die oben wiedergegebenen DNA-Sequenzen kodieren Peptidfragmente, die als 'Targeting'-Signal für die pflanzliche Vakuole fungieren und die folgende, in SEQ ID NO: 8 und 9 wiedergegebene Aminosäuresequenz aufweisen:

Leu Leu Val Asp Thr Met

Gly Leu Leu Val Asp Thr Met

Die genannten Peptidfragmente sind somit ebenfalls ein Bestandteil der vorliegenden Erfindung.

Im Rahmen der vorliegenden Erfindung konnte nunmehr erstmals gezeigt werden, dass die oben näher charakterisierten DNA-Sequenzen, die ein kurzes, C-terminales Peptid-Fragment kodieren, welches für die gezielte Einschleusung des damit natürlicherweise assoziierten Strukturgens verantwortlich ist, in der Lage sind auch in Verknüpfung mit heterologen Genen weiterhin ihre 'Targeting' Funktion zu erfüllen.

In einer spezifischen Ausführungsform der vorliegenden Erfindung wird dabei die genannte 'Targeting' DNA-Sequenz mit Hilfe der zuvor beschriebenen Methoden mit einem heterologen Gen, vorzugsweise mit einem heterologen Chitinasegen, das keine entsprechende 3'-terminale Sequenz aufweist, operabel verknüpft und in einen pflanzlichen Wirt transformiert. Innerhalb dieser transformierten Wirtspflanze wird dann ein Genprodukt exprimiert, das gezielt in die pflanzliche Vakuole eingeschleust wird.

Im einzelnen kann man dabei so vorgehen, dass man zunächst eine cDNA Genbibliothek aus geeignetem Pflanzenmaterial herstellt und aus besagter Genbibliothek mit Hilfe geeigneter Sondenmoleküle einen passenden Chitinase-cDNA Klon isoliert, der keine 3'-terminale Extension aufweist. Nach Einspleissen dieses cDNA Klones in einen geeigneten Klonierungsvektor kann besagter Klon durch Insertion bzw. Deletion von Restriktionsschnittstellen so modifiziert werden, dass eine operable Verknüpfung der erfindungsgemässen DNA 'Targeting'-Sequenz mit dem heterologen Chitinasegen erfolgen kann. Das fertige Konstrukt kann dann in einen geeigneten Pflanzenexpressionsvektor einkloniert werden, wobei es unter die regulatorische Kontrolle von in Pflanzen aktiven Expressionssignalen gelangt. Beispielhaft sei an dieser Stelle der Pflanzenexpressionsvektor pGY1 erwähnt, welcher den 35S Promotor des CaMV Virus enthält sowie dessen Terminationssequenzen, ohne dadurch jedoch den Gegenstand der vorliegenden Erfindung in irgeneiner Weise zu limitieren. Selbstverständlich kann auch jeder andere geeignete Vektor and dieser Stelle verwendet werden.

So ist es beispielsweise möglich, eine saure Chitinase aus Gurken, die kein C-terminales 'Signal-Peptid' besitzt und daher normalerweise in den extrazellulären Raum sezerniert wird, durch Verknüpfen mit einer der beschriebenen 'Targeting'-Sequenzen gezielt in die pflanzliche Vakuole einzuschleusen.

Ein weiterer Gegengstand der vorliegenden Erfindung betrifft daher die Konstruktion von chimären, rekombinanten DNA Molekülen, welche eine exprimierbare DNA, insbesondere aber ein Strukturgen, vorzugsweise ein heterologes Strukturgen, in operabler Verknüpfung mit der erfindungsgemässen DNA-Sequenz und mit in pflanzlichen Zellen aktiven Expressionssignalen, wie Promotor- und Terminationssequenzen, sowie gegebenenfalls mit weiteren kodierenden und/oder nicht kodierenden Sequenzen der 5'- und/oder 3'-Region enthalten.

Dabei ist es oft vorteilhaft zwischen der Promotorsequenz und der sich anschliessenden kodierenden DNA-Sequenz eine 'Leader'-Sequenz einzubauen, wobei die Länge der 'Leader'-Sequenz so gewählt wird, dass der Abstand zwischen dem Promotor und der 'Targeting' DNA-Sequenz für die Expression des jeweils assoziierten Strukturgens optimal ist.

Es ist weiterhin vorteilhaft, wenn das einzuschleusende DNA-Molekül eine Sequenz enthält oder aber in der 5'-terminalen Region mit einer solchen Sequenz verknüpft wird, die für ein in der pflanzlichen Zelle funktionsfähiges, N-terminales Signalpeptid kodiert. Darüberhinaus können weitere Sequenzabschnitte im DNA Molekül vorliegen, welche für Peptidfragemente kodieren, die insgesamt dazu beitragen, die Kompetenz für eine Aufnahme in die Vakuole zu verbessern wie beispielsweise das von Matsuoka K und Nakamura K in der N-terminalen Extension des Sporamin aufgefundene Propeptidfragment [Matsuoka K und Nakamura K (1991)].

Für die Verwendung in dem erfindungsgemässen Verfahren sind in erster Linie all diejenigen Strukturgene geeignet, die bei den transformierten pflanzlichen Zellen sowie den daraus sich entwickelnden Geweben und insbesondere aber den Pflanzen zu einem Schutzeffekt führen, wie z.B. zu einer erhöhten Resistenz gegenüber Pathogenen (beispielsweise gegenüber phytopathogenen Pilzen, Bakterien, Viren, etc.); einer Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc,), Insektiziden oder anderen Bioziden]; einer Resistenz gegenüber nachteiligen Umwelteinflüssen (beispielsweise gebenüber Hitze, Kälte, Wind, ungünstigen Bodenverhältnissen, Feuchtigkeit, Trockenheit, etc.).

Besonders hervorzuheben im Rahmen dieser Erfindung sind Strukturgene, die im Zusammenhang mit der Bekämpfung pflanzlicher Pathogene und Parasiten stehen.

Eine Resistenz gegenüber Insekten kann beispielsweise durch eine Gen übertragen werden, das ein Polypeptid kodiert, welches toxisch ist für Insekten und/oder deren Larven, wie z.B. das kristalline Protein von *Bacillus thuringiensis*.

Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen und sind daher normalerweise in Vakuolen bzw. 'Protein bodies' lokalisiert. Es konnte nachgewiesen werden, dass ein aus Sojabohnen isolierter und gereinigter Bowman-Birk Proteaseinhibitor die Darmprotease von *Tenebrio* Larven hemmt [Birk *et al* (1963)]. Das Gen, welches den Trypsininhibitor aus der Kuherbse kodiert, ist bei Hilder *et al* (1987) beschrieben.

Im Rahmen der vorliegenden Erfindung können nunmehr beliebige Proteaseinhibitoren verwendet werden, unabhängig von ihrer Herkunft [z.B. Proteaseinhibitoren aus nichtpflanzlichen oder rein synthetischen Quellen], indem man diese mit einem in der pflanzlichen Zelle funktionsfähigen, N-terminalen Signalpeptid und einer C-terminalen 'Targeting'-Sequenz operabel verknüpft. Dies führt dazu, dass die so modifizierten Proteaseinhibitoren gezielt in die Vakuole eingeschleust werden, wo sie optimal gespeichert werden können.

Ein Gen, das einen Proteaseinhibitor kodiert, kann in einem geeigneten Vektor unter die Kontrolle eines Pflanzenpromotors, insbesondere eines konstitutiven Promotors, wie z.B. des CaMV 35S Promotors, gebracht werden. Das Gen, beispielsweise die kodierende Sequenz des Bowman-Birk Proteaseinhibitors aus der Sojabohne, kann mit Hilfe der cDNA Klonierungsmethode erhalten werden. Eine weitere Möglichkeit zur Herstellung eines Proteaseinhibitors besteht in dessen synthetischer Herstellung, sofern dieser weniger als 100 Aminosäuren aufweist, wie z.B. der Trypsininhibitor der Limabohne. Die kodierende Sequenz lässt sich durch Zurückübersetzen der Aminosäuresequenz voraussagen. Zusätzlich werden an beiden Enden Restriktionsschnittstellen eingebaut, die für den jeweils gewünschten Vektor geeignet sind. Das synthetische Gen wird durch Synthese überlappender Oligonukleotidfragmente von 30 bis 60 Basenpaaren hergestellt, indem diese zunächst einer Kinasereaktion unterworfen, dann miteinander verknüpft [Maniatis *et al* (1982)] und schliesslich in einem passenden Vektor kloniert werden. Mit Hilfe der DNA Sequenzierung kann dann ein Klon, der das Insert in einer korrekten Orientierung aufweist, identifiziert werden. Für die Einschleusung in die Protoplasten kann isolierte Plasmid-DNA verwendet werden.

Ebenfalls zu erwähnen sind in diesem Zusammenhang hydrolytische Enzyme, die in der Lage sind, den Abbau der Zellwände von pflanzlichen Patogenen entweder selbst zu bewerkstelligen oder aber diesen in Verbindung mit anderen Substanzen im Sinne einer Synergie zumindest unterstützen.

Die Mehrzahl der Insekten beispielsweise besitzt ein Cuticularskelett, in dem lamellenförmig geschichtete Micelle aus Chitin in einer Grundsubstanz eingebettet sind. Auch zahlreiche pflanzenpathogene Pilze enthalten Chitin als integralen Bestandteil ihrer Hyphen- und Sporenstrukturen, so z.B. die Basidiomyceten (Brand- und Rostpilze), Ascomyceten und *Fungi Imperfecti* (einschliesslich *Alternaria* und *Bipolaris, Exerophilum turcicum, Colletotricum, Gleocercospora* und *Cercospora*). Chitinase ist in der Lage das Mycelwachstum bestimmter Pathogene *in-vitro* zu hemmen. Ein pflanzliches Organ oder Gewebe, das in der Lage ist die Chitinase konstitutiv oder aber als Antwort auf das Eindringen eines Pathogens zu exprimieren, könnte sich somit gegen den Angriff einer grossen Zahl verschiedener Pilze schützen.

. Die natürlicherweise in der Pflanze vorhandenen, endogenen Chitinasen liegen entweder extrazellulär (saure Chitinasen) oder innerhalb der Vakoule (basische Chitinasen) vor, sodass im Rahmen der vorliegenden Erfindung zwei alternative Vorgehensweisen denkbar sind.

Zum einen ist es möglich, die sauren Chitinasegene, denen die für die Einschleusung in die Vakuole verantwortliche Sequenz am C-terminalen Ende fehlt, unter Verwendung einer der der beschriebenen DNA Sequenzen so zu modifizieren, dass die Genprodukte gezielt in die Vakuole gelangen, wo sie die dort natürlicherweise vorhandenen, endogenen, basischen Chitinasen in ihrer Aktivität gegenüber eindringenden Pathogenen unterstützen können.

Die zweite Variante besteht darin, aus einem natürlicherweise vakuolär vorliegenden basischen Chitinasegen die C-terminale Extension mit Hilfe gentechnischer Verfahren aus dem offenen Leseraster zu entfemen oder aber diese zumindest zu inaktivieren, z.B. durch Einfügen eines Stopp Kodons unmittelbar vor der C-terminalen Extension. Dies führt dazu, dass das Genprodukt in den Interzcllularraum sezerniert wird, wo es in unmittelbaren Kontakt mit dem eindringenden Pathogen gelangen kann.

Ein weiterer Aspekt der vorliegenden Offenbarung betrifft somit rekombinante DNA Moleküle, die ein natürlicherweise vakuolär vorliegendes Strukturgen in operabler Verknüpfung mit in pflanzlichen Zellen aktiven Expressionssignalen enthalten, deren natürlicherweise im Gen vorliegende 3'-terminale 'Targeting'-Sequenz deletiert oder aber in anderer Weise inaktiviert ist. Diese Konstrukte liefern bei Transformation in einen pflanzlichen Wirt ein Expressionsprodukt, das keine funktionelle C-terminale Signalsequenz mehr enthält und damit in den Extrazellularraum der Pflanze sezerniert wird.

So ist z.B. ein rekombinantes DNA Molekül offenbart, das ein basisches Chitinasegen in operabler Verknüpfung mit in pflanzlichen Zellen aktiven Expressionssignalen enthält, dessen natürlicherweise im Gen vorliegende 3'-terminale 'Targeting'-Sequenz deletiert oder in anderer Weise inaktiviert ist und das daher bei Transformation in einen pflanzlichen Wirt ein Expressionsprodukt liefert, das keine funktionelle C-terminale Signalsequenz mehr enthält und damit in den Extrazellularraum der Pflanze sezerniert wird.

Ein weiteres Enzym, das im Abwehrmechanismus der Pflanze gegenüber Pathogenen vermutlich eine zentrale Rolle spielt, ist die β-1,3-Glucanase, deren Verwendung in Kombination mit einer 'Target' DNA-Sequenz ebenfalls beschrieben ist.

Weiterhin beschrieben ist daher ein rekombinantes DNA Molekül, das ein basisches Glucanasegen in operabler Verknüpfung mit in pflanzlichen Zellen aktiven Expressionssignalen enthält, dessen natürlicherweise im Gen vorliegende 3'-terminale 'Targeting'-Sequenz deletiert oder in anderer Weise inaktiviert ist und das daher bei Transformation in einen pflanzlichen Wirt ein Expressionsprodukt liefert, das keine funktionelle C-terminale Signalsequenz mehr enthält und damit in den Extrazellularraum der Pflanze sezerniert wird.

Ein weiterer Gegenstand dieser Erfindung betrifft die Verknüpfung der beschriebenen 'Targeting'-Sequenzen mit sog. lytischen Peptiden ['Lytic Peptids']. Dabei handelt es sich um natürliche oder synthetische Peptide mit antipathogener Aktivität, die in der Lage sind die Zellmembran von Pathogenen zu durchdringen, zu lysieren oder auf andere Weise zu schädigen. Vertreter solcher lytischer Peptide, die im Rahmen der vorliegenden Erfindung Verwendung finden können, sind sowohl aus tierischen [einschliesslich Insekten] als auch aus pflanzlichen und mikrobiellen Quellen bekannt und umfassen beispielsweise die Defensine, Cercopine, Thionine, Mellitine von Säugern, die Defensine, Magainine, Attacine, Dipterine, Sapecine, Caeruline, Xenopsine der Insekten sowie Hybride davon. Die Aminsäuresequenzen verschiedener lytischer Peptide sind in folgenden Publikationen wiedergegeben: WO 89/11291; WO 86/04356; WO 88/05826; US 4,810,777; WO 89/04371 sowie bei Bohlmann *et al* (1988), Selsted und Hartwig (1987) und Terry *et al* (1988).

Unter lytischen Peptiden sind im weitesten Sinne auch Verbindungen zu verstehen, deren Fähigkeit zur Pentetration, Lyse oder Schädigung der Zellmembranen auf einer enzymatischen Aktivität beruht, wie z.B. Lysozyme und Phospholipasen.

Weiterhin beschrieben ist eine kombinierte Expression von hydrolytischen und lytischen Peptiden mit anschliessender zielgerichteter Einschleusung in die Vakuole oder gegebenfalls den extrazellulären Raum. Darüberhinaus ist auch eine wechselseitige Anwendung von Expression und exogener Applikation denkbar, wobei insbesondere die lytischen Peptide für letzteren Verwendungszweck in Frage kommen, in Verbindung mit den üblicherweise für diesen Zweck verwendeten Hilfs- und/oder Zusatzstoffen.

Auch für eine Produktion gewünschter und nützlicher Verbindungen in der pflanzlichen Zelle als solcher oder als Bestandteil einer höher organisierten Einheit, wie z.B. einem Gewebe, Kallus, Organ, Embryo oder einer ganzen Pflanze, lässt sich die 'Targeting' DNA-Sequenzen in idealer Weise verwenden.

Gene, die ebenfalls im Rahmen der vorliegenden Erfindung Verwendung finden können, umfassen beispielsweise solche, die zu einer erhöhten Bildung von Reserve- oder Speicherstoffen in Blättern, Samen, Knollen, Wurzel, Stengeln, etc. oder in den 'Protein bodies' von Samen führen. Zu den wünschenswerten Substanzen, die von transgenen Pflanzen produziert werden können, zählen beispielsweise Proteine, Kohlehydrate, Aminosäuren, Vitamine, Alkaloide, Flavine, Riech- und Farbstoffe, Fette, etc..

Ebenso können Strukturgene mit der erfindungsgemässen DNA-Sequenz assoziiert werden, die pharmazeutisch akzeptable Wirksubstanzen, wie z.B. Hormone, Immunmodulatoren und andere physiologisch aktive Substanzen kodieren.

Zu den Genen, die im Rahmen dieser Erfindung in Betracht gezogen werden können, gehören daher bekannte Gene, ohne jedoch darauf beschränkt zu sein, beispielsweise pflanzenspezifische Gene wie das Zeingen aus Mais, das Aveningen aus Hafer, das Glutelingen aus Reis, etc., Säuger-spezifische Gene wie das Insulingen, das Somatostatingen, die Interleucingene, das t-PA Gen, etc., oder aber Gene mikrobiellen Ursprungs wie das NPT II Gen, etc. sowie synthetische Gene wie das Insulingen, etc.

Neben natürlicherweise vorkommenden Strukturgenen, die eine nützliche und wünschenswerte Eigenschaft kodieren, können im Rahmen dieser Erfindung auch Gene verwendet werden, die zuvor mit Hilfe chemischer oder gentechnologischer Methoden gezielt modifiziert wurden.

Weiterhin sind von dem breiten Konzept der vorliegenden Erfindung auch solche Gene umfasst, die vollständig durch chemische Synthese hergestellt werden. Als Gene oder DNA Sequenzen die im Rahmen der vorliegenden Erfindung Verwendung finden können, kommen somit sowohl homologe als auch heterologe Gen(e) oder DNA sowie synthetische Gen(e) oder DNA gemäss der im Rahmen der vorliegenden Erfindung gemachten Definition in Betracht. Als Beispiel für ein synthetisches Gen sein an dieser Stelle das Insulingen genannt.

Die im Rahmen der vorliegenden Erfindung verwendbaren DNA Sequenzen können ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA als auch die genomische DNA können aus verschiedenen Genen stammen. In jedem Fall können aber sowohl die genomische DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen oder Varietäten derselben Art oder verschiedenen Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, ist es vorteilhaft, wenn die kodierenden Gensequenzen zunächst in operabler Weise mit in pflanzlichen Zellen funktionsfähigen Expressions-Sequenzen verknüpft werden. Die hybriden Genkonstruktionen im Rahmen der vorliegenden Erfindung enthalten somit neben der 'Targeting' DNA-Sequenz ein oder mehrere Strukturgen(e) sowie in operabler Verknüpfung damit Expressions-Signale, die sowohl Promotor- und Terminator-Sequenzen als auch weitere regulatorische Sequenzen der 3' und 5' nicht-translatierten Regionen miteinschliessen.

Jeder Promotor und jeder Terminator, der in der Lage ist, eine Induktion der Expression einer kodierenden DNA-Sequenz (Strukturgen) zu bewirken, kann als Bestandteil der hybriden Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Cauliflower Mosaik Virus Gene (CaMV) oder aber homologe DNA-Sequenzen, die noch die charakteristischen Eigenschaften der genannten Expressionssignale aufweisen. Ebenfalls geeignet sind bakterielle Expressionssignale, insbesondere aber die Expressionssignale der Nopalin-Synthase-Gene (nos) oder der Octopin-Synthase-Gene (ocs) aus den Ti-Plasmiden von *Agrobacterium tumefaciens*.

Bevorzugt im Rahmen dieser Erfindung sind die 35S und 19S Expressions-Signale des CaMV Genoms oder deren Homologe, die mit Hilfe molekularbiologischer Methoden, wie sie z.B. bei Maniatis *et al*,(1982) beschrieben sind, aus besagtem Genom isoliert und mit der kodierenden DNA-Sequenz verknüpft werden können.

Unter Homologen der 35S und 19S Expressionssignale sind im Rahmen dieser Erfindung Sequenzen zu verstehen, die trotz geringer Sequenzunterschiede den Ausgangssequenzen im wesentlichen homolog sind und die nach wie vor die gleiche Funktion wie diese erfüllen.

Als Ausgangsmaterial für die 35S-Transkriptionskontroll-Sequenzen kann erfindungsgemäss z.B. das ScaI-Fragment des CaMV Stammes "S", das die Nukleotide 6808-7632 der Genkarte umfasst [Frank G *et al* (1980)], verwendet werden.

Die 19S Promotor- und 5' nicht-translatierte Region befindet sich auf einem Genomfragment zwischen der PstI Stelle (Position 5386) und der HindIII-Stelle (Position 5850) der CaMV Genkarte [Hohn *et al* (1982)]. Die entsprechende Terminator- und 3' nichttranslatierte Region liegt auf einem EcoRV/BglII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC *et al* (1981) beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche ein gewünschtes Genprodukt kodiert, verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose- 1,5-bisphosphat-Carboxylase aus Sojabohnen sowie den Promotor des Chlorophyll-a/b- Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, Cashmore A (1983)].

Ein weiterer Gegenstand der vorliegenden Erfindung betrifft somit rekombinante DNA Moleküle, die eine chimäre genetische Konstruktion enthalten, worin eine 'Target' DNA-Sequenz mit einer exprimierbaren DNA und mit in pflanzlichen Zellen aktiven Expressionssignalen sowie gegebenenfalls mit weiteren transkribierten und/oder nicht-transkribierten Sequenzen aus der 5'- und/oder 3'-Region, operabel verknüpft ist, sodass es bei Transformation in einen pflanzlichen Wirt zu einer gezielten Einschleusung der Expressionsprodukte in die pflanzliche Vakuole kommt.

Besonders bevorzugt sind rekombinante DNA Moleküle die eine chimäre genetische Konstruktion enthalten, worin eine 'Target' DNA-Sequenz mit einem Strukturgen operabel assoziiert ist, das den transformierten pflanzlichen Zellen sowie den daraus sich entwickelnden Geweben und insbesondere aber den Pflanzen einen Schutzeffekt gegenüber Pathogenen, Chemikalien sowie nachteiligen Umwelteinflüssen verleiht.

Ganz besonders bevorzugt im Rahmen dieser Erfindung sind rekombinante DNA Moleküle, die eine chimäre genetische Konstruktion enthalten, worin die 'Targeting' DNA-Sequenz mit einem Strukturgen operabel assoziiert ist, das Chitinase, insbesondere aber saure Chitinase, oder Glucanase, insbesondere aber saure Glucanase, in pflanzlichen Zellen exprimiert.

Ebenfalls umfasst von der vorliegenden Erfindung sind rekombinante DNA Moleküle, die eine chimäre genetische Konstruktion enthalten, worin die 'Targeting' DNA-Sequenz mit einem Strukturgen operabel assoziiert ist, das bei Expression in der transformierten pflanzlichen Zelle als solcher oder als Bestandteil einer höher organisierten Einheit ausgewählt aus der Gruppe bestehend aus einem Gewebe, Organ, Kallus, Embryo oder einer ganzen Pflanze, zu einer Einschleusung gewünschter und nützlicher Verbindungen in die pflanzliche Vakuole führt

Weiterhin beschrieben sind rekombinante DNA Moleküle, die eine chimäre genetische Konstruktion enthalten, worin ein Gen, welches ein natürlicherweise vakuolär vorliegendes Proteinmolekül kodiert, vorzugsweise aber ein basisches Chitinasegen, und ganz besonders bevorzugt ein basisches Chitinasegen aus Tabak- oder Gurkenpflanzen, so modifiziert ist, dass es in den Extrazellularraum sezemiert wird. Im einzelnen kann dies dadurch erreicht werden, dass man die natürlicherweise in besagten Genen, insbesondere aber in Chitinasegenen vorliegende C-terminale Extension, welche die für die Einschleusung in die Vakuole verantwortlichen DNA-Sequenzen enthält, mit Hilfe gentechnischer Verfahren entfernt bzw inaktiviert, beispielsweise durch Einfügen eines Stopp Kodons unmittelbar vor der C-terminalen Extension.

Weitere regulatorische DNA-Sequenzen, die für die Konstruktion chimärer Gene Verwendung finden können, umfassen beispielsweise Sequenzen, die in der Lage sind die Transkription einer assoziierten DNA-Sequenz in pflanzlichen Geweben im Sinne einer Induktion oder Repression zu regulieren.

So gibt es beispielsweise einzelne Pflanzengene, von denen bekannt ist, dass sie durch verschiedene interne und externe Faktoren wie Pflanzenhormone, Hitzeschock, Chemikalien, Pathogene, Sauerstoffmangel, Licht, Stress, etc. induziert werden.

Als ein Beispiel für eine Genregulation durch ein Pflanzenhormon soll hier die Abscisinsäure (ABS) erwähnt werden, von der bekannt ist, dass sie den während der späten Embryonalphase auftretenden Überschuss an mRNAs in Baumwolle induziert. Ein weiteres Beispiel liefert die Gibberellinsäure (GA3), die in Rizinussamen Malatsynthasetranskripte sowie in den Aleuronschichten von Gerste Isoenzyme der a-Amylase induziert.

Die Aktivität von Glucanase und Chitinase in Bohnenblättern kann durch Behandlung mit dem Stresshormon Ethylen markant erhöht werden. Für Chitinase wird dieser Induktionseffekt über den Promotor des Chitinasegens gesteuert, was durch Reportergenversuche unter Verwendung eines Promotors aus dem Chitinasegen von Bohnen (*Phaseolus vulgaris*) gezeigt werden konnte.

Die Regulation von Hitzeschock empfindlichen Proteingenen der Sojabohne wurde im Detail untersucht. Eine mehrstündige Behandlung der Pflanzen bei einer Temperatur von 40° C resultiert in der *de novo* Synthese von sogenannten Hitzeschock-Proteinen. Zahlreiche dieser Gene wurden inzwischen isoliert und ihre Regulation im einzelnen analysiert. Die Expression dieser Gene wird in erster Linie auf der Ebene der Transkription kontrolliert. Fusioniert man beispielsweise den Promotor des hps70 Gens mit dem Neomycinphosphotransferase II (NPT II) Gen, so kann das auf diese Weise entstandene chimäre Gen durch einen Hitzeschock induziert werden [Spena *et al*, 1985)].

Eine andere Klasse von in Pflanzen induzierbaren Genen beinhaltet die Licht-regulierten Gene, insbesondere das nukleär kodierte Gen der kleinen Untereinheit der Ribulose-1,5-bisphosphatcarboxylase (RUBISCO). Morelli *et al* (1985) haben nachgewiesen, dass die 5'-flankierende Sequenz eines RUBISCO-Gens aus der Erbse in der Lage ist, die Lichtinduzierbarkeit auf ein Reportergen zu übertragen, sofern dies in chimärer Form mit dieser Sequenz verknüpft ist. Diese Beobachtung konnte auch auf andere Licht-induzierte Gene ausgedehnt werden, wie z.B. das Chlorophyll a/b -Bindungsprotein.

Die Alkoholdehydrogenase-Gene (adh-Gene) des Mais waren Gegenstand intensiver Untersuchungen. Das adh1-s Gen aus Mais wurde isoliert und es wurde nachgewiesen, dass ein Teil der 5'-flankierenden DNA in der Lage ist, die Expression eines chimären Reportergens (z.B. Chloramphenicolacetyltransferase; CAT ) zu induzieren, wenn das vorübergehend transformierte Gewebe anaeroben Bedingungen ausgesetzt wurde [Howard *et al* (1987)]

Eine weitere Gruppe regulierbarer DNA-Sequerizen betrifft chemisch regulierbare Sequenzen, die z.B. in den PR-("pathogenesis related proteine") Proteingenen des Tabaks vorliegen und mit Hilfe chemischer Regulatoren induzierbar sind.

Die zuvor beispielhaft genannten regulierbaren DNA-Sequenzen können sowohl natürlichen als auch synthetischen Ursprungs sein oder aber aus einem Gemisch von natürlichen und synthetischen DNA-Sequenzen bestehen.

Es ist weiterhin vorteilhaft, wenn die exprimierbare DNA, insbesondere aber das einzuschleusende Strukturgen, eine Sequenz enthält oder aber in der 5'-terminalen Region mit einer solchen Sequenz verknüpft wird, die für ein in der pflanzlichen Zelle funktionsfähiges, N-terminales Signalpeptid kodiert. Dabei handelt es sich um ein Transportsignal, das am N-terminalen Ende von Proteinen zu finden ist, die über das Endomembransystem transportiert werden. Dieses Signalsequenz sorgt dafür, dass besagte Proteine zunächst ins Endoplasmatische Retikulum gelangen, wo das Signalpeptid proteolytisch vom Precursor-Protein abgespalten wird, sobald es seine Funktion erfüllt hat. Entsprechend seiner spezifischen Funktion wurde dieser Sequenz-Typ des Signal-Peptids im Verlaufe der Evolution bei allen lebenden Zellen in hohem Masse konserviert, unabhängig davon, ob es sich um Bakterien, Hefen, Pilze, Tiere oder Pflanzen handelt.

Darüberhinaus können noch weitere Sequenzabschnitte im DNA Molekül vorliegen, welche für Peptidfragemente kodieren, die insgesamt dazu beitragen, die Kompetenz für eine Aufnahme in die Vakuole zu verbessern wie beispielsweise das von Matsuoka K und Nakamura K in der N-terminalen Extension des Sporamin aufgefundene Propeptidfragment [Matsuoka K und Nakamura K (1991)].

Weiterhin sind somit chimäre genetische Konstruktionen beschrieben, die neben der in operabler Verknüpfung mit einem Strukturgen oder sonstigen exprimierbaren DNA Sequenzen vorliegenden 'Targeting'-Sequenz weitere regulatorische DNA Sequenzabschnitte enthalten, die z.B. eine gezielte Induktion oder Repression der Genexpression ermöglichen.

Die verschiedenen Sequenzabschnitte können mit Hilfe an sich bekannter Methoden miteinander zu einer vollständigen kodierenden DNA-Sequenz verknüpft werden. Geeignete Methoden schliessen beispielsweise die *in vivo* Rekombination von DNA-Sequenzen, die homologe Abschnitte aufweisen sowie die *in vitro* Verknüpfung von Restriktionsfragmenten mit ein.

Als Klonierungsvektoren verwendet man im allgemeinen Plasmid- oder Virus-(Bakteriophagen)-Vektoren mit Replikations- und Kontrollsequenzen, die von Spezies stammen, die mit der Wirtszelle kompatibel sind.

Der Klonierungsvektor trägt in der Regel einen Replikationsursprung, insbesondere einen Replikationsursprung, der in *E. coli*, in *Agrobacterium* oder in beiden funktionsfähig ist, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise Resistenzen gegen Ampicillin, Tetracyclin, Hygromycin, Kanamycin, Methotrexat, G418 und Neomycin, ohne das diese beispielhafte Aufzählung eine Limitierung des Erfindungsgegenstandes darstellt.

Als Wirtszellen kommen im Rahmen dieser Erfindung Prokaryonten in Frage, einschliesslich bakterieller Wirte, wie z.B. *A.tumefaciens, E.coli, S.typhimurium* und *Serratia marcescens*, sowie Cyanobakterien. Ferner können auch eukaryontische Wirte wie Hefen, mycelbildende Pilze und Pflanzenzellen im Rahmen dieser Erfindung verwendet werden.

Das Einspleissen der erfindungsgemässen hybriden Genkonstruktion in einen geeigneten Klonierungsvektor erfolgt mit Hilfe von Standardmethoden wie sie z.B. bei Maniatis *et al* (1982) beschrieben sind.

Dabei wird in der Regel der Vektor und die einzuspleissende DNA Sequenz zunächst mit geeigneten Restriktions-Enzymen geschnitten. Geeignete Restriktionsenzyme sind z.B. solche, die Fragmente mit glatten Enden liefern, wie z.B. SmaI, HpaI und EcoRV, oder aber Enzyme, die kohäsive Enden bilden, wie z.B. EcoRI, SacI und BamHI.

Sowohl Fragmente mit glatten Enden wie auch solche mit kohäsiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der *E.coli* DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Desoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Im Rahmen der vorliegenden Erfindung werden bei der Konstruktion der Chitinasemutanten verschiedene Teile der jeweils verwendeten Chitinaseklone miteinander verknüpft. Um dieses Ziel erreichen zu können, müssen diese Teile kompatibel sein, d.h. es müssen geeignete Restriktionsschnittstellen in die verschiedenen Sequenzen eingefügt werden. In einer spezifischen Ausführungsform dieser Erfindung werden daher vorzugsweise die im folgenden aufgelisteten Restriktionsschnittstellen verwendet, die alle zu den gleichen kohäsiven Enden führen:
BamHI [G/GATCC]
BclI [T/GATCA]
BglII [A/GATCT]

Der Leseraster wird vorzugsweise jeweils so gewählt, dass GAT als Kodon für Aspartat fungiert.

Die Klonierungsvektoren und die mit diesen Vektoren transformierte Wirtszelle werden in der Regel dazu verwendet, die Kopienzahl der darin einklonierten Konstrukte zu erhöhen. Mit einer erhöhten Kopienzahl ist es möglich, den Vektor, der die hybride Genkonstruktion trägt, zu isolieren und z.B. für die Einschleusung der chimären Gensequenz in die pflanzliche Zelle vorzubereiten.

In einem weiteren Verfahrensschritt werden diese Plasmide dazu verwendet, die ein gewünschtes Genprodukt kodierenden Strukturgene oder aber nicht-kodierende DNA-Sequenzen mit regulatorischer Funktion, in die pflanzliche Zelle einzuschleusen und gegebenenfalls in das Pflanzengenom zu integrieren.

Weitertin beschrieben ist die Herstellung von pflanzlichen Rezipienten-Zellen, welche besagtes Strukturgen oder sonstige wünschenswerte Gene bzw. Genfragmente oder sonstige nützliche DNA-Sequenzen in ihr Genom eingebaut enthalten.

Die Einschleusung der chimären genetischen Konstruktion erfolgt vorzugsweise in pflanzliche Protoplasten mit Hilfe bekannter Gen-Transfer-Verfahren.

Es existieren mittlerweile eine Reihe von sehr effizienten Verfahren für die Einführung von DNA in Pflanzenzellen, basierend auf der Verwendung von Gentransfer-Vektoren oder auf direkten Gentransfer-Verfahren.

Eine Möglichkeit besteht beispielsweise darin, dass man Pflanzenzellen mit Viren oder mit *Agrobacterium* in Kontakt bringt. Dies kann durch Infektion sensitiver Pflanzenzellen oder durch Co-Cultivierung von Protoplasten, die sich aus Pflanzenzellen ableiten, bewerkstelligt werden. Das Cauliflower Mosaik Virus (CaMV) kann im Rahmen dieser Erfindung ebenso als Vektor für die Einschleusung der erfindungsgemässen chimären genetischen Konstruktion in die Pflanze verwendet werden. Das gesamte virale DNA-Genom von CaMV wird dabei in ein bakterielles Elternplasmid integriert unter Ausbildung eines rekombinanten DNA Moleküls, das in Bakterien vermehrt werden kann. Nach erfolgter Klonierung wird das rekombinante Plasmid mit Hilfe von Restriktionsenzymen entweder zufällig oder an ganz spezifischen, nicht essentiellen Stellen innerhalb des viralen Teils des rekombinanten Plasmids gespalten, z.B. innerhalb des Gens, das für die Uebertragbarkeit des Virus durch Blattläuse kodiert, und die hybride Genkonstruktion wird in diese Schnittstelle einkloniert.

Ein kleines Oligonukleotid, ein sog. Linker, der eine einzige, spezifische Restriktionserkennungsstelle besitzt, kann ebenfalls integriert werden. Das so modifizierte rekombinante Plasmid wird erneut kloniert und durch Einspleissen der hybriden Genkonstruktion in eine nur einmal vorkommende Restriktionsstelle weiter modifiziert.

Der modifizierte virale Anteil des rekombinanten Plasmids kann dann aus dem bakteriellen Eltern-Plasmid ausgeschnitten und für die Inokulation der Pflanzenzellen oder der gesamten Pflanzen verwendet werden.

Eine andere Methode zur Einschleusung der chimären Genkonstruktion in die Zelle bedient sich der Infektion der Pflanzenzelle mit *Agrobacterium tumefaciens* und/oder *Agrobacterium rhizogenes*, welches mit besagter Genkonstruktion transformiert ist. Die transgenen Pflanzenzellen werden anschliessend unter geeigneten, dem Fachmann bekannten Kultivierungsbedingungen kultiviert, so dass sie Schösslinge und Wurzeln ausbilden und letztlich ganze Pflanzen resultieren.

Eine weitere Möglichkeit zur Transformation pflanzlichen Materials besteht in einer gemischten Infektion unter Verwendung von sowohl *Agrobacterium rhizogenes* als auch transformiertem *Agrobacterium tumefaciens* wie es von Petit *et al* (1986) für die Transformation von Karotten beschrieben ist. Das Mischungsverhältnis muss dabei so aufeinander abgestimmt werden, dass die aufgrund der *A. rhizogenes* Transformation gebildeten Würzelchenkolonien auch einen hohen Anteil an *A. tumefaciens* Ti-Plasmiden enthalten. Dies kann z.B. dadurch erreicht werden, dass man *A. rhizogenes* und *A. tumefaciens* in einem Mischungsverhältnis von 1:1 bis 1:100, vorzugsweise aber in einem Mischungsverhältnis von 1:10 in bekannter Weise gemeinsam auf das Pflanzenmaterial appliziert. Die transgenen Pflanzen werden anschliessend unter geeigneten, dem Fachmann bekannten Kultivierungsbedingungen kultiviert, sodass sie Schösslinge und Wurzel ausbilden und letztlich ganze Pflanzen resultieren.
Das Vermischen der beiden *Agrobacterium* Spezies erfolgt vorteilhafterweise erst kurz vor der eigentlichen Inokulation des zu transformierenden pflanzlichen Materials.

Die erfindungsgemässe chimäre Genkonstruktion lässt sich somit beispielsweise mit Hilfe des Ti-Plasmids von *Agrobacterium tumefaciens* oder des Ri-Plasmids von *Agrobacterium rhizogenes* in geeignete Pflanzenzellen überführen. Das Ti-Plasmid bzw. das Ri-Plasmid wird im Verlaufe der Infektion durch *Agrobacterium* auf die Pflanze übertragen und stabil ins Pflanzengenom integriert.

Sowohl Ti-Plasmide als auch Ri-Plasmide besitzen zwei Regionen, die für die Herstellung transformierter Zellen essentiell sind. Eine davon, die Transfer-DNA (T-DNA) Region, wird auf die Pflanze übertragen und führt zur Induktion von Tumoren. Die andere, die virulenzverleihende (vir) Region, ist nur für die Ausbildung, nicht aber für die Aufrechterhaltung der Tumoren essentiell.
Die Transfer-DNA Region kann in ihren Dimensionen durch Einbau der chimären Genkonstruktion vergrössert werden, ohne dass die Uebertragungsfähigkeit beeinträchtigt wird. Durch Entfemen der tumorverursachenden Gene und durch Einbau eines selektionierbaren Markers kann das modifizierte Ti- bzw. Ri-Plasmid als Vektor für den Transfer der erfindungsgemässen Genkonstruktion in eine geeignete Pflanzenzelle verwendet werden.

Die vir-Region bewirkt den Transfer der T-DNA Region von *Agrobacterium* auf das Genom der Pflanzenzelle, unabhängig davon, ob die T-DNA-Region und die vir-Region auf demselben Vektor oder auf verschiedenen Vektoren innerhalb derselben *Agrobacterium*-Zelle vorliegen. Eine vir-Region auf einem Chromosom induziert ebenso den Transfer der T-DNA von einem Vektor in eine Pflanzenzelle.

Im Rahmen dieser Erfindung wird daher bevorzugt ein System zur Uebertragung einer T-DNA-Region von *Agrobacterium* in Pflanzenzellen verwendet, bei dem die vir-Region und die T-DNA-Region auf verschiedenen Vektoren liegen. Ein solches System ist unter dem Begriff "binäres Vektor-System" bekannt und der die T-DNA enthaltende Vektor wird entsprechend als ein "binärer Vektor" bezeichnet.

Jeder T-DNA enthaltende Vektor, der in Pflanzenzellen übertragbar ist und der eine Selektion der transformierten Zellen erlaubt, ist für die Verwendung im Rahmen dieser Erfindung geeignet.

Besonders bevorzugt im Rahmen dieser Erfindung ist ein 'Shuttle'-Vektor, der die erfindungsgemässe chimäre genetische Konstruktion zwischen der Linken (LB) und Rechten Bordersequenz (RB) einkloniert enthält und der in der Lage ist sowohl in *E. coli* als auch in *A. tumefaciens* stabil zu replizieren.

Durch Anwendung neu entwickelter Transformationstechniken lassen sich mittlerweile *in vitro* im Prinzip auch solche Pflanzenspezies transformieren, die keine natürlichen Wirtspflanzen für *Agrobacterium* sind. So sind beispielsweise monokotyle Pflanzen, insbesondere aber die Getreidearten sowie verschiedene Gräser, keine natürlichen Wirte für *Agrobacterium*.

Es gibt nämlich in der Zwischenzeit vermehrt Hinweise darauf, dass sich auch Monokotyledonen mit *Agrobacterium* transformieren lassen, so dass unter Verwendung von neuen experimentellen Ansätzen, die jetzt verfügbar werden, auch Getreide und Gräser-Spezies einer Transformation zugänglich werden [Grimsley NH *et al* (1987)].

Bevorzugt im Rahmen dieser Erfindung ist die sog. Blattscheiben-Transformation ("Leaf Disk Transformation") unter Verwendung von *Agrobacterium* [Horsch *et al* (1985)]. Sterile Blattscheiben einer geeigneten Zielpflanze werden dabei mit *Agrobacterium* Zellen, die eine der erfindungsgemässen chimären Genkonstruktionen enthalten, inkubiert und anschliessend in oder auf ein geeignetes Nährmedium überführt. Besonders geeignet und daher im Rahmen dieser Erfindung bevorzugt sind LS Medien, die durch Zusatz von Agar verfestigt sind und die mit einem oder mehreren der üblicherweise verwendeten pflanzlichen Wachstumsregulatoren, insbesondere aber solchen ausgewählt aus der Gruppe der Auxine bestehend aus a-Naphthylessigsäure, Picloram, 2,4,5-Trichlorphenoxyessigsäure, 2,4-Dichlorphenoxyessigsäure, Indol-3-buttersäure, Indol-3-milchsäure, Indol-3-bemsteinsäure, Indol-3-essigsäure, p-Chlorphenoxyessigsäure sowie aus der Gruppe der Cytokinine bestehend aus Kinetin, 6-Benzyladenin, 2-Isopentenyladenin und Zeatin, angereichert sind. Die bevorzugte Konzentration der Auxine und Cytokinine liegt in einem Bereich von 0.1 mg/l und 10 mg/l.

Nach mehrtägiger Inkubation, vorzugsweise aber nach 2 bis 3tägiger Inkubation bei einer Temperatur von 20°C bis 40°C, vorzugsweise von 23°C bis 35°C und ganz besonders bevorzugt von 25°C und diffusem Licht werden die Blattscheiben zur Sprossinduktion auf ein geeignetes Medium tranferriert. Besonders bevorzugt zur Selektion der Transformanten ist ein LS Medium, das kein Auxin, dafür aber ein Cytokinin enthält, und mit einer Selektivsubstanz versetzt ist. Die Kulturen werden im Licht gehalten und in geeigneten Abständen, vorzugsweise aber in einwöchigen Intervallen auf frisches Medium übertragen. Sich entwickelnde grüne Sprosse werden ausgeschnitten und in einem Medium, das eine Bewurzelung der Sprosse induziert, weiterkultiviert. Besonders bevorzugt im Rahmen dieser Erfindung ist ein LS Medium, das kein Auxin und kein Cytokinin enthält, zur Selektion der Transformanten aber mit einer Selektivsubstanz versetzt ist.

Neben der *Agrobacterium*-vermittelten Transformation sind im Rahmen dieser Erfindung für die Einschleusung der erfindungsgemässen Genkonstruktionen in pflanzliches Material auch direkte Transformationsverfahren anwendbar.

So kann das genetische Material, das in einem Vektor enthalten ist, beispielsweise mit Hilfe rein physikalischer Massnahmen direkt in die pflanzliche Zelle eingeschleust werden, wie z.B. durch Mikroinjektion unter Verwendung fein ausgezogener Mikropipetten [Neuhaus *et al* (1987)] oder durch Beschuss der Zellen mit Mikroprojektilen, die mit der transformierenden DNA beschichtet sind ["Microprojectile Bombardment"; Wang Y-C *et al* (1988)].

Weitere Möglichkeiten für den direkten Transfer von genetischem Material in die pflanzliche Zelle bestehen in der Behandlung der Protoplasten mit die Plasmamembran modifizierenden Verfahrensmassnahmen, wie z.B. der Polyethylenglykol Behandlung, der Hitzeschockbehandlung oder der Elektroporation sowie einer Kombination dieser Verfahrensmassnahmen [Shillito *et al* (1985)].

Bei der Elektroporations-Technik werden pflanzliche Protoplasten zusammen mit Plasmiden, die die hybride Genkonstruktion enthalten, elektrischen Impulsen hoher Feldstärke ausgesetzt. Dies führt zu einer reversiblen Permeabilitätserhöhung von Biomembranen und ermöglicht damit die Einschleusung der Plasmide. Elektroporierte pflanzliche Protoplasten emeuern ihre Zellwand, teilen sich und bilden Kallusgewebe. Die Selektion der transformierten Pflanzenzellen kann mit Hilfe der zuvor beschriebenen phaenotypischen Marker erfolgen.

Ein weiteres Verfahren für die direkte Einführung von genetischem Material in Pflanzenzellen, das auf rein chemischen Verfahrensmassnahmen beruht und eine sehr effiziente und schnelle Durchführung der Transformation ermöglicht, ist bei Negrutiu I *et al* (1987) sowie bei Goodall G *et al* beschrieben.

Ebenfalls geeignet für die Transformation pflanzlichen Materials ist der direkte Gentransfer unter Verwendung der Co-Transformation (Schocher RJ *et al* 1986).

Bei der Co-Transformation handelt es sich um eine Methode, die auf der gleichzeitigen Aufnahme und Integration verschiedener DNA-Moleküle (nicht-selektionierbares und selektionierbares Gen) ins pflanzliche Genom beruht, und die daher das Auffinden von Zellen, die mit nicht selektierbaren Genen transformiert sind, ermöglicht.

Die zuvor gegebene beispielhafte Auflistung möglicher Transformationsverfahren erhebt keinen Anspruch auf Vollständigkeit und soll den Erfindungsgegenstand in keiner Weise einschränken.

Diejenigen Zellklone, die die hybride Genkonstruktion in ihr Genom eingebaut enthalten, werden mit Hilfe üblicher Selektions-, Screening- und Nachweismethoden ausgelesen und für die Regeneration transgener Pflanzen verwendet.

Die Regeneration von in Kultur gehaltenen Protoplasten zu ganzen Pflanzen ist z.B. bei Potrykus I und Shillito RD (1986) beschrieben.

Die Regenerationsverfahren unterscheiden sich von Pflanzenspezies zu Pflanzenspezies. Im allgemeinen aber werden die Protoplasten in einem der bekannten Kulturmedien zur Teilung und Zellwandbildung angeregt. Es entstehen schliesslich Kalluskulturen, welche durch Behandlung mit bestimmten Wirkstoffen, wie z.B. Auxinen und Cytokininen zur Wurzel- bzw. Sprossbildung induziert werden können.

Die so gewonnenen Pflänzchen können anschliessend in Erde übertragen und in gleicher Weise wie normale Sämlinge weiterkultiviert werden.

Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wiederholbar.

Die regenerierten transgenen Pflanzen, welche ein in der pflanzlichen Zelle exprimierbares Strukturgen der zuvor beschriebenen hybriden Genkonstruktion als integralen Bestandteil des pflanzlichen Genoms enthalten, kann vegetativ vermehrt werden, vorzugsweise in Form steriler Spross-Kulturen.

Die stabile Integration eines funktionsfähigen exprimierbaren Gens ins pflanzliche Genom der regenerierten transgenen Pflanzen wird anhand der mitotischen Stabilität des integrierten Gens sowie aufgrund des Verhaltens als Mendelsches Merkmal während der Meiose und unter Verwendung der "Southern blot" Analyse (Southern EM, 1975) verifiziert.

Ebenfalls umfasst vom breiten Konzept dieser Erfindung ist somit transgenes pflanzliches Material, ausgewählt aus der Gruppe bestehend aus Protoplasten, Zellen, Kalli, Geweben, Organen, Samen, Embryonen, Pollen, Eizellen, Zygoten, etc. sowie insbesondere auch ganzen, fertilen Pflanzen, das mit Hilfe der zuvor beschriebenen Verfahren transformiert worden ist und die erfindungsgemässe rekombinante DNA in exprimierbarer Form enthält. Ebenfalls beschrieben sind Verfahren zur Herstellung besagten transgenen pflanzlichen Materials.

Das Verfahren zur Herstellung von transformiertem pflanzlichem Material, welches ein Genprodukt enthält, das gezielt in die pflanzliche Vakuole eingeschleust wird, ist im wesentlichen dadurch charakterisiert, dass man:
(a) zunächst die für die für eine gezielte Einschleusung in die Vakuole verantwortliche DNA-Sequenz aus einer geeigneten Quelle isoliert oder diese mit Hilfe bekannter Verfahren synthetisiert;
(b) besagte DNA-Sequenz in das 3'-terminale Ende einer beliebigen exprimierbaren DNA-Sequenz operabel inseriert;
(c) das fertige Konstrukt in einen Pflanzenexpressionsvektor unter die Kontrolle von in Pflanzen aktiven Expressionssignalen einkloniert; und
(d) besagten Expressionsvektor mit Hilfe bekannter Verfahren in pflanzliches Material transformiert und dort exprimiert.

Handelt es sich bei der zu exprimierenden DNA nicht um ein Strukturgen, das bereits natürlicherweise ein N-terminales Signalpeptid kodiert, so ist es vorteilhaft, zusätzlich eine DNA Sequenz, die für ein solches Signalpeptid kodiert, operabel in die 5'-terminalen Region der zu exprimierenden DNA zu inserierten.

Ebenfalls beschrieben ist ein Verfahren zur Herstellung von transformiertem pflanzlichem Material, welches ein Genprodukt enthält, das gezielt in den extrazellulären Raum sezerniert wird, das im wesentlichen dadurch charakterisiert ist, dass man:
(a) eine für ein solches Genprodukt kodierende DNA-Sequenz isoliert;
(b) die für die Einschleusung in das jeweilige zelluläre Kompartiment verantwortliche 'Targeting'-Sequenz vom C-terminalen Ende entfernt;
(c) besagte deletierte DNA-Sequenz in einen geeigneten Pflanzenexpressionsvektor einspleisst; und
(d) das fertige Konstrukt in pflanzliches Material transformiert.

Bevorzugt im Rahmen dieser Erfindung sind transgene Pflanzen, einschliesslich ihrer sexuellen und asexuellen Nachkommenschaft, die aus pflanzlichem Material, ausgewählt aus der Gruppe bestehend aus Protoplasten, Zellen, Kalli, Geweben, Organen, Samen, Embryonen, Pollen, Eizellen, Zygoten, etc. regenerierbar sind und eines der erfindungsgemässen rekombinanten DNA-Moleküle enthalten.

Ebenfalls bevorzugt sind transgene Pflanzen einschliesslich ihrer sexuellen und asexuellen Nachkommenschaft, die gegenüber dem Wildtyp einen signifikant erhöhten Prtiteingehalt in der pflanzlichen Vakuole und/oder im Extrazellularraum aufweisen.

Besonders bevorzugt sind transgene Pflanzen einschliesslich ihrer sexuellen und asexuellen Nachkommenschaft, die gegenüber dem Wildtyp einen signifikant erhöhten Chitinasegehalt in der pflanzlichen Vakuole ausweisen.

Ebenfalls besonders bevorzugt sind transgene Pflanzen einschliesslich ihrer sexuellen und asexuellen Nachkommenschaft, die gegenüber dem Wildtyp einen signifikant erhöhten Chitinasegehalt im Extrazellularraum der Pflanze ausweisen.

Unter den Begriff der asexuellen und/oder sexuellen Nachkommenschaft transgener Pflanzen fallen definitionsgemäss im Rahmen dieser Erfindung somit auch alle Mutanten und Varianten die mit Hilfe bekannter Verfahren, wie z.B. durch Zellfusionen oder Mutantenselektion gewonnen werden können, und welche noch die charakteristischen Eigenschaften der transformierten Ausgangspflanze aufweisen, sowie sämtliche Kreuzungs-und Fusionsprodukte mit dem transformierten Pflanzenmaterial.

Ein weiterer Gegenstand dieser Erfindung betrifft das Vermehrungsgut transgener Pflanzen.

Unter Vermehrungsgut transgener Pflanzen soll im Rahmen dieser Erfindung jedes beliebige pflanzliche Material verstanden werden, das sich auf sexuellem oder asexuellem Wege bzw. *in-vivo* oder *in-vitro* vermehren lässt. Als besonders bevorzugt sind im Rahmen dieser Erfindung in erster Linie Protoplasten, Zellen, Kalli, Gewebe, Organe, Samen, Embryonen, Pollen, Eizellen, Zygoten anzusehen, sowie jedes beliebige andere Vermehrungsgut das von transgenen Pflanzen erhalten werden kann.

Pflanzenteile, wie z.B. Blüten, Stengel, Früchte, Blätter, Wurzeln, die von transgenen Pflanzen oder deren Nachkommen stammen, die zuvor mit Hilfe des beschriebenen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen *Angiospermae* und *Gymnospermae*.

Unter den *Gymnospermae* sind von besonderem Interesse die Pflanzen der Klasse *Coniferae*.

Unter den *Angiospermae* sind neben den Laubbäumen und Sträuchern von besonderem Interesse Pflanzen der Familien *Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Malvaceae* oder *Gramineae* und der Ordnung *Leguminosae* und hier vor allem der Familie *Papilionaceae*. Bevorzugt sind Vertreter der Familien *Solanaceae, Cruciferae* und Gramineae.

Zu den Zielkulturen im Rahmen der vorliegenden Erfindung zählen beispielsweise auch jene, ausgewählt aus der Reihe: *Fragaria, Lotus, Medicago, Onobrychis, Trifolium, Trigonella, Vigna, Citrus, Linum, Geranium, Manihot, Daucus, Arabidopsis, Brassica, Raphanus, Sinapis*, *Atropa*, *Capsicum*, *Datura*, *Hyoscyamus*, *Lycopersion*, *Nicotiana*, *Solanum, Petunia, Digitalis, Majorana, Cichorium, Helianthus, Lactuca, Bromus, Gossypium, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, Ipomoea, Passiflora, Cyclamen, Malus, Prunus, Rosa, Rubus, Populus, Santalum, Allium, Lilium, Narcissus, Ananas, Arachis, Phaseolus* und *Pisum.*

Aufgrund neuer Enwicklungen auf dem Gebiet der *in vitro* Kultivierung von Pflanzen, vornehmlich im Bereich der Pflanzenregeneration, ist es inzwischen möglich, auch bei Vertretern aus der Familie der Gramineae, ausgehend von pflanzlichen Protoplasten, ganze Pflanzen zu regenerieren. Beispiele von erfolgreich durchgeführten Regenerationsexperimenten bei Gramineen sind u.a. bei Yamada Y *et al* (1986) für Reisprotoplasten, bei Rhodes *et al* (1988) und Shillito RD *et al* (1989) für Maisprotoplasten sowie bei Horn *et al* (1988) für *Dactylis glomerata* Protoplasten beschrieben.

Es können im Rahmen der vorliegenden Erfindung daher auch die folgenden Pflanzen verwendet werden: *Lolium, Zea, Triticum, Sorghum, Saccharum, Bromus, Oryzae, Avena, Hordeum, Secale* und *Setaria.*

Ein weiterer bevorzugter Gegenstand dieser Erfindung betrifft somit transgene Pflanzen aus der Gruppe der *Graminaceae* einschliesslich ihrer sexuellen und asexuellen Nachkommenschaft, die aus pflanzlichem Material, ausgewählt aus der Gruppe bestehend aus Protoplasten, Zellen, Kalli, Geweben, Organen, Samen, Embryonen, Pollen, Eizellen, Zygoten, etc. regenerierbar sind und eines der erfindungsgemässen rekombinanten DNA-Moleküle enthalten.

Die reifen Pflanzen, die aus transformierten Pflanzenzellen herangezogen wurden, werden zur Samenproduktion mit sich selbst gekreuzt. Einige der Samen enthalten die Gene, die für eine nützliche und wünschenswerte Eigenschaft kodieren in einem Verhältnis, das genau den etablierten Gesetzen der Vererbung gehorcht. Diese Samen können zur Produktion transgener Pflanzen verwendet werden.

Homozygote Linien können durch wiederholte Selbstbestäubung und Herstellung von Inzuchtlinien gewonnen werden. Diese Inzuchtlinien können dann wiederum zur Entwicklung von Hybriden verwendet werden. Bei diesem Verfahren wird eine Inzuchtlinie, die besagtes Fremdgen enthält, mit einer anderen Inzuchtlinie zur Produktion gekreuzt.

Nach der allgemeinen Beschreibung der vorliegenden Erfindung soll nun zum besseren Verständnis auf spezifische Ausführungsbeispiele Bezug genommen werden, die zum Zwecke der Illustration in die Beschreibung mitaufgenommen werden, und die keinen limitierenden Charakter haben, es sei denn, es wird speziell darauf hingewiesen.

In der im folgenden im Detail beschriebenen spezifischen Ausführungsform der vorliegenden Erfindung wird eine basische Chitinase sowie eine nicht verwandte Klasse III Chitinase [Gurkenchitinase], die natürlicherweise in den extrazellulären Raum sektretiert wird und keine Homologie zu Klasse I oder II Chitinasen aufweist, mit und ohne 'Targeting'-Sequenz in *Nicotiana sylvestris* Pflanzen exprimiert und ihre Lokalisation innerhalb der pflanzlichen Zelle bestimmt.

Der Nachweis für die Notwendigkeit der C-terminalen Extension der natürlicherweise vakuolär vorliegenden Proteine für eine zielgerichtete Lokalisation in die Vakuole sowie für die prinzipielle Möglichkeit zur gezielten Ausschleusung besagter Proteine in den extrazellulären Raum, lässt sich anhand einer basischen Chitinase sowie einer basischen Glucanase aus Tabak führen. Dabei werden die 7 bzw. 22 C-terminalen Aminosäuren, welche die C-terminale Extension dieser natürlicherweise vakuolär vorliegenden Proteine ausmachen, entfernt bzw inaktiviert, indem man mittels Oligonucleotid-Mutagenese ein geeignetes Stopp Codon an der entsprechenden Stelle innerhalb der kodierenden Sequenz plaziert. Die führt in der Folge zu einer Ausschleusung der mutierten Chitinase bzw. Glucanase in den extrazellulären Raum.

Um dagegen zu demonstrieren, dass besagte 'Targeting'-Sequenz darüberhinaus in der Lage ist, ein assoziiertes Proteinmolekül gezielt in die Vakuole zu dirigieren, wird die C-terminale Sequenz einer basischen Chitinase aus Tabak mit einer sauren Chitinase aus Gurkenblätter verknüpft. Dabei geht man so vor, dass zunächst die 5'-nichtkodierende Sequenz aus einer basischen Tabakchitinase sowie die ebenfalls in diesem Bereich lokalisierte, für ein Signalpeptid kodierende Sequenz mit der die reife Grukenchitinase kodierenden Sequenz verknüpft wird. Anschliessend wird das 3'-Ende dieses Konstrukts über ein Linkerfragment eine 9 Aminosäuren kodierende Sequenz aus der C-terminalen Extension des Tabakchitinasegens angehängt.
Die zuvor beschriebenen Kontrukte werden dann unter die Kontrolle eines starken CaMV 35 S Promotors gebracht und mittels *Agrobacterium*-Transformation in *Nicotiana sysvestris* Pflanzen eingeschleust.

Nach erfolgter Transformation werden transgene Pflanzen, welche eine starke Chitinaseexpression zeigen, selektiert und für die Analyse der Chitinaselokalisation verwendet. Es werden Protoplasten hergestellt und die spezifischen Aktivitäten der Chitinase in Homogenaten und Protoplasten verglichen.

Zur Bestimmung der subzellulären Lokalisation der intrazellulären Chitinase werden Vakuolen aus Protoplasten isoliert.

### NICHTLIMITIERENDE AUSFÜHRUNGSBEISPIELE

### Allgemeine rekombinante DNA-Techniken

Da viele der in dieser Erfindung genutzten rekombinanten DNA-Techniken Routine für den Fachmann sind, ist eher hier eine kurze Beschreibung dieser allgemein gebrauchten Techniken enthalten als jedesmal dort, wo sie erscheinen. Bis darauf, wo extra darauf hingewiesen wird, sind alle diese Verfahren in der Referenz von Maniatis e*t a*l (1982) beschrieben.

### A. Schneiden mit Restriktionsendonukleasen

Typischerweise sind etwa 50 bis 500 µg/ml DNA in dem Reaktionsansatz enthalten, in der vom Hersteller, New England Biolabs, Beverly, MA., empfohlenen Pufferlösung. 2 bis 5 Einheiten von Restriktionsendonukleasen werden für jedes µg DNA hinzugefügt und der Reaktionsansatz bei der vom Hersteller empfohlenen Temperatur für eine bis drei Stunden inkubiert. Die Reaktion wird durch 10 minütiges Erhitzen auf 65°C oder durch Extraktion mit Phenol, gefolgt durch Präzipitation der DNA mit Ethanol, beendet. Diese Technik wird auch auf den Seiten 104 bis 106 der Maniatis e*t a*l (1982)-Referenz beschrieben.

### B. Behandlung der DNA mit Polymerase, um glatte Enden zu erzeugen

50 bis 500 µg/ml DNA-Fragmente werden zu einem Reaktionsansatz hinzugefügt, in dem vom Hersteller, New England Biolabs, empfohlenen Puffer. Der Reaktionsansatz enthält alle vier Desoxynukleotidtriphosphate in Konzentrationen von 0.2 mM. Die Reaktion erfolgt während 30 Minuten bei 15°C und wird dann durch 10 minütiges Erhitzen auf 65°C beendet. Für Fragmente, die durch Schneiden mit Restriktionsendonukleasen erhalten werden, welche 5'-hervortretende Enden erzeugen, wie EcoRI und BamHI, wird das grosse Fragment, oder Klenow-Fragment, der DNA-Polymerase benutzt. Für Fragmente, die durch Endonukleasen erhalten werden, welche 3'-hervometende Enden erzeugen, wie PstI und SacI, wird die T4-DNA-Polymerase benutzt. Die Verwendung dieser zwei Enzyme wird auf den Seiten 113 bis 121 der Maniatis e*t a*l (1982)-Referenz beschrieben.

### C. Agarose-Gelelektrophorese und Reinigung von DNA-Fragmenten von Gelen

Die Agarose-Gelelektrophorese wird in einem horizontalen Apparat durchgeführt, wie auf den Seiten 150 bis 163 der Maniatis et al.-Referenz beschrieben. Der benutzte Puffer ist der dort beschriebene Tris-Borat- puffer. Die DNA-Fragmente werden durch 0.5 µg/ml Ethidiumbromid gefärbt, das entweder im Gel- oder Tankpuffer während der Elektrophorese vorhanden ist oder nach der Elektrophorese hinzugefügt wird. Die DNA wird durch Beleuchtung mit langwelligem Ultraviolettlicht sichtbar gemacht. Wenn die Fragmente vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis e*t a*l (1982) auf Seite 170 beschriebenen leicht verändert.

Als Alternative kann die DNA aus der Agarose mit Hilfe des Geneclean Kits (Bio 101 Inc., La Jolla, CA, USA) isoliert werden.

### D. Hinzufügen von synthetischen Linkerfragmenten an DNA-Enden

Wenn es erwünscht ist, eine neue Endonukleaseschnittstelle an das Ende eines DNA-Moleküls anzufügen, wird das Molekül gegebenenfalls zuerst mit DNA-Polymerase behandelt, um glatte Enden zu erzeugen, wie im obigen Abschnitt beschrieben. Etwa 0.1 bis 1.0 µg dieses Fragments wird zu etwa 10 ng phosphorylierter Linker-DNA gegeben, die von New England Biolabs bezogen wurde, in einem Volumen von 20 bis 30 µl mit 2 µl T4 DNA-Ligase von New England Biolabs, und 1mM ATP in dem vom Hersteller empfohlenen Puffer. Nach einer Inkubation über Nacht bei 15°C wird die Reaktion durch 10 minütiges Erhitzen auf 65°C beendet.

Der Reaktionsansatz wird auf etwa 100 µl in einem Puffer verdünnt, der richtig für die Restriktionsendonuklease ist, die die synthetische Linkersequenz schneidet. Ungefähr 50 bis 200 Einheiten dieser Endonuklease werden hinzugefügt. Die Mischung wird 2 bis 6 Stunden bei der angemessenen Temperatur inkubiert, dann wird das Fragment einer Agarose-Gelelektrophorese unterworfen und gereinigt wie oben beschrieben. Das resultierende Fragment wird nun Enden haben mit Endigungen, welche durch Schneiden mit der Restriktionsendonuklease erzeugt wurden. Diese Enden sind gewöhnlich kohäsiv, so dass das resultierende Fragment nun leicht an andere Fragmente mit den gleichen kohäsiven Enden geknüpft werden kann.

### E. Entfernen von 5'-terminalen Phosphaten von DNA-Fragmenten

Während der Plasmidklonierungsschritte vermindert die Behandlung des Vektorplasmids mit Phosphatase die Rezirkularisation des Vektors (diskutiert auf Seite 13 der Maniatis e*t a*l-Referenz). Nach Schneiden der DNA mit der richtigen Restriktionsendonuklease wird eine Einheit alkalische Phosphatase aus dem Darm von Kälbern hinzugefügt, die von Boehringer-Mannheim, Mannheim, erworben wurde. Die DNA wird eine Stunde bei 37°C inkubiert und anschliessend zweimal mit Phenol extrahiert und mit Ethanol gefällt.

### F. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15°C durchgeführt. Wenn DNA-Fragmente mit glatten Enden verknüpft werden sollen, werden sie inkubiert wie oben, bis darauf, dass die Menge der T4 DNA-Ligase auf 2 bis 4 Einheiten erhöht wird.

### G. Die Transformation von DNA in E. coli

*E. coli* Stamm HB101 wird für die meisten Experimente verwendet. DNA wird mit dem Kalziumchloridverfahren, wie es von Maniatis *et al* (1982), Seiten 250 bis 251, beschrieben wurde, in E. *coli* eingeführt.

### H. Screening von E.coli auf Plasmide

Nach der Transformation werden die resultierenden Kolonien von *E. coli* auf das Vorhandensein des gewünschten Plasmids durch ein schnelles Plasmidisolationsverfahren geprüft. Zwei gebräuchliche Verfahren werden auf den Seiten 366 bis 369 der Maniatis *et al* (1982)-Referenz beschrieben.

### I. Isolierung von Plasmid-DNA in grossem Massstab

Verfahren zur Isolierung von Plasmiden aus *E.coli* in grossem Massstab werden auf den Seiten 88 bis 94 der Maniatis *et al* (1982)-Referenz beschrieben.

### J. Klonierung in M13 Phagenvektoren

Die folgende Beschreibung ist so zu verstehen, dass die doppelsträngige replikative Form der Phage M13-Abkömmlinge für Routineverfahren, wie Schneiden mit Restriktionsendonuklease, Verknüpfen etc., benutzt wird.

Enzyme können, wenn nicht extra darauf hingewiesen wird, von Boehringer, Biolabs (BRL), bezogen werden. Sie werden entsprechend den Angaben des Herstellers verwendet, es sei denn, es wird gesondert darauf hingewiesen.

### K. Southern blot-Analyse

Die extrahierte DNA wird dabei zunächst mit Restriktionsenzymen behandelt, anschliessend einer Elektrophorese in einem 0,8%igen bis 1%igen Agarose-Gel unterworfen, auf eine Nitrozellulosemembran [Southern EM (1975)] transferiert und mit der nachzuweisenden DNA, welche zuvor einer Nick Translation unterworfen wurde (DNA-spezifische Aktivitäten von 5 x 10⁸ bis 10 x 10⁸ c.p.m./µg), hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNA wird durch Schwärzung eines Röntgenfilms während 24 bis 48 Stunden sichtbar gemacht.

### L. Western Blot Analyse

Nach einer SDS-Polyacrylamidgelelektrophorese werden die Proteine elektrophoretisch auf einen Nitrocellulose- oder Nylonfilter übertragen. Dieser Filter wird dann zunächst mit einem blockierenden Agens (z.B. 5% Magermilchpulver in PBS: Milch-PBS) vorbehandelt. Anschliessend wird der Filter mehrere Stunden mit einem Antiserum inkubiert, das mit der jeweils nachzuweisenden Verbindung (im vorliegenden Fall: Chitinase) reagiert. Der auf diese Weise vorbehandelte Filter wird mehrmals mit Milch-PBS gewaschen und dann mit einem kommerziell erhältlichen sekundären Antikörper, der mit einem Enzym gekoppelt ist [z.B. Peroxidase gekoppelte Ziegen-anti-Kaninchen Antikörper (BIORAD), 1:2000 verdünnt in Milch-PBS], inkubiert. Der Filter wird nochmals in PBS gewaschen und anschliessend gemäss den Angaben des Herstellers [BIORAD] mit Chloronaphtol und Wasserstoffperoxid angefärbt. Weiter Details sind in Sambrook *et al* (1989) angegeben.

### M. Generelle Techniken für die Generierung, Reinigung und automatische Sequenzierung von Peptiden,

Reduktion und Alkylierung: Gereinigtes, lyophylisiertes Protein wird in 6 M Guanidin-HCl enthaltend 1 M Tris-HCl (pH 8.6) und 10 mM EDTA gelöst. Dithiothreitol (DDT) wird bis zu einer Endkonzentration von 20 mM, 4-Vinylpyridin bis zu einer Endkonzentration von 50 mM zugegeben. Dieser Ansatz wird anschliessend unter Stickstoff 1.5 Stunden inkubiert. Das pyridylethylierte Material wird dann über eine HPLC [Aquapore Phenylsäule (2.1 x 10 cm, Brownlee)] entsalzt. Die Säule wird mit einem linearen, von 5% bis 80% reichenden Gradienten eines Acetonitril:Isopropanol Gemisches (1:1) in 0.1 % Trifluoressigsäure (TFA) eluiert.

Cyanogenbromid Spaltung und Verdauung mit Pygroglutamataminopeptidase: Die Cyanogenbromidspaltung wird *in situ* gemäss Simpson und Nice (1984)durchgeführt. Eine Verdauung mit Pyroglutamataminopeptidase [Boehringer Mannheim] kann gemäss Allen (1981)durchgeführt werden.

LysC Verdauung: Das Protein wird mit Endoproteinase Lys-C [Boehringer Mannheim] in 0.1 M Tris-HCl (pH 8.5) über einen Zeitraum von 24 Stunden bei Raumtemperatur verdaut, unter Verwendung eines Enzym:Substrat Verhältnisses von 1:10. Die resultierenden Peptide werden mit Hilfe einer HPLC [Aquapore C-8 Säule (1 x 22 cm, Brownlee)] isoliert. Als Elutionsmittel dient ein linearer Acetonitril:Isopropanol (1:1) Gradient (0% bis 60%) in 0.1% TFA.

Trypsin Verdauung: Die Verdauung von Protein mit Trypsin [Cooper] wird in 0.1 M Ammoniumbicarbonat (pH 8.2) enthaltend 0.1 M Calciumchlorid bei einer Temperatur von 37°C und einem Enzym:Substrat Verhältnis von 1:100 durchgeführt. Die Inkubationsdauer beträgt 5 Stunden. Die resultierenden Peptide werden über eine HPLC abgetrennt [vergl. Abschnitt C, oben].
Altemativ dazu kann die Verdauung auch bei einem Enzym:Substrat Verhältnis von 1:50 in 0.1 M Tris-HCL (pH 8.5) bei einer Temperatur von 37°C durchgeführt werden. Die Inkubationsdauer beträgt in diesem Fall 24 Stunden. Die resultierenden Peptide werden über eine HPLC [Vydac C-18 Säule (2.1 x 150 mm)] unter Verwendung eines linearen (0% bis 60%) Acetonitril:Isopropanol (1:1) Gradienten in 0.1% TFA aufgetrennt.

Sequenzierung: Der automatisierte Edman Abbau wird mit Hilfe eines Applied Biosystems 470A Gasphasen Sequenzierers durchgeführt. Die Identifizierung der Phenylthiohydantoin (PTH) Aminosäuren erfolgt unter Verwendung eines Applied Biosystems 129A PTH Analysators.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.

### I. Herstellung von cDNA- und genomischen Genbibliotheken aus Tabak

### Beispiel 1: Pflanzenmaterial

*Nicotiana tabacum* L. c.v. Havana 425 Pflanzen werden entweder im Gewächshaus oder aber ausgehend von Oberflächen-sterilisierten Samen herangezogen. Die Oberflächensterilisation der Samen wird folgendermassen durchgeführt:
20 bis 30 Samen werden in ein Sieb mit einer Porengrössen von ca. 200 µm gegeben und in 10 ml einer 10%igen, kommerziell erhältlichen Bleichlösung (NaOCl) inkubiert. Die Samen werden anschliessend mehrmals mit sterilem destilliertem Wasser abgespült.

In den folgenden Ausführungsbeispielen wird vorrangig eine klonierte Linie parenchymatischen Markgewebes (N) verwendet, die gemäss Eichholz *et al* (1983) aus *Nicotiana tabacum* L. c.v. Havana 425 Pflanzen isoliert werden kann.

### Beispiel 2: Gewebekultur

Das Tabakgewebe wird auf einem Basismedium kultiviert, das eine Salz- und Thiamin-HCl-Konzentration nach Linsmeier und Skoog (1965) (LS) aufweist und durch Zusatz von 10 g/l Agar verfestigt ist. Als weiteren Zusatz enthält dieses Basismedium einen pH-Indikator, wie beispielsweise 5 mg/l Chlorphenolrot. Weitere Medien, die auf diesem LS Basismedium aufbauen und in den sich anschliessenden Ausführungsbeispielen zur Anwendung kommen, enthalten als weitere Zusätze z.B. Kinetin (1.4 µM) [Cytokinin--Medium] oder a-Naphthylessigsäure (10.7 µM) [Auxin-Medium] oder aber ein Gemisch aus Kinetin und a-Naphtylessigsäure [Auxin/Cytokinin-Medium (Medium A)].

Die Selektivmedien B und C enthalten kein a-Naphtylessigsäure, dafür aber eine Selektivsubstanz, mit deren Hilfe die Transformanten aus der grossen Anzahl nicht transformierter Zellen und Gewebe ausgelesen werden können. Die genaue Zusammensetzung dieser Selektivmedien ist in Abschnitt VII (Medien) wiedergegeben.

Die aus *Nicotiana tabacum* L. c.v. Havana 425 Pflanzen isolierte Stamm-Linie (275N) wird in Intervallen von 21 Tagen auf einem Auxin/Cytokinin-Medium (10 ml) subkultiviert, d.h. jeweils auf neues Medium (10 ml) überimpft und bei 25'C im Licht kultiviert.

Zur Induktion hoher Chitinase-Spiegel in den kultivierten Geweben werden diese von dem Auxin/Cytokinin-Medium auf ein Hormon-freies Basismedium überimpft. Weitere Einzelheiten zur Kultivierung von Pflanzengeweben und zur Induktion von Chitinase sind bei Felix und Meins (1985)beschrieben.

### Beispiel 3: Herstellung von Tabakprotoplasten

100 ml einer 2 Tage alten Tabak Zellsuspension gemäss Beispiel 1 wird mit einem gleichen Volumen einer doppelt konzentrierten Enzymlösung gemischt. Die Enzymlösung enthält die folgenden Bestandteile:

| | |
|---|---|
| Cellulase R.10 Onozuka® (Yakult Honsha, Tokyo, Japan) | 10.0 g/l |
| Macerase (Pektinase from *Rhizopus sp.,* Behring Diagnostics, La Jolla, CA) | 2.5 g/l |
| Pectolyase Y-23® (Seishin Pharm. Co., Tokyo, Japan) | 1.0 g/l |
| CaNO₃. 4H₂O | 1.45 g/l |
| MgSO₄. 7H₂O | 0.5 g/l |
| NH₄H₂PO₄ | 0.23 g/l |
| KNO₃ | 1.2 g/l |
| D-Mannit (pH 5.70) | 73.0 g/l |

Obige Enzymlösung wird zentrifugiert und durch ein 0.2 mm Filter sterilisiert.
Der Ansatz bestehend aus Tabak-Suspensionskultur und Enzymlösung wird 5 Stunden bei Zimmertemperatur auf einer Rundschüttelmaschine (40 Upm) vorsichtig bewegt. In bestimmten Zeitabständen werden aus diesem Ansatz Proben entnommen und mikroskopisch analysiert. Die enzymatische Verdauung wird solange fortgesetzt, bis ca. 80% der Zellen in spärische Protoplasten umgewandelt sind. Die Inkubationsgefässe werden dann von der Schüttelmaschine genommen. Man lässt die Zell-/Protoplastensuspension absetzten und entfernt die obere Hälfte des Mediums, die keine Zellen oder Protoplasten enthält durch Absaugen mit einer Pipette und verwirft diese. Der Rest wird in 50 ml Zentrifugenröhrchen überführt und 10 Minuten bei 500 Upm in einer klinischen Zentrifuge (Modell HN-SII, IEC) zentrifugiert. Die Protoplasten-haltigen Pellets werden in einer Rinse I-Lösung [verlgeiche Abschnitt VII] resuspendiert und anschliessend erneut 10 Minuten bei 1'000 Upm zentrifugiert.

Die Bande mit den Protoplasten befindet sich am oberen Rand des Zentrifugenröhrchens. Die Protoplastenfraktion wird gesammelt und anschliessend erneut in Rinse I-Lösung gewaschen. Die Protoplasten werden in ein Zentrifugenröhrchen zurücküberführt und bis zur weiteren Verwendung in Eis aufbewahrt.

### Beispiel 4: Konstruktion einer cDNA Genbibliothek

Die cDNA-Genbibliotek wird ausgehend von poly(A)+ RNA hergestellt, welche aus einer klonierten Linie parenchymatischen Markgewebes von *Nicotiana tabacum* L. c.v. Havana 425 Pflanzen (siehe Beispiel 1) erhältlich ist. Das Tabakgewebe wird zuvor gemäss Beispiel 2 zur Produktion hoher Chitinasespielgel angeregt, indem man dieses auf einem hormonfreien LS-Basismedium kultiviert.

### 4.1. Isolierung von Gesamt-RNA

Die Aufbereitung von Gesamt-RNA erfolgt im wesentlichen nach dem bei Lagrimini LM *et al* (1987) beschriebenen Verfahren.

In flüssigem Stickstoff tiefgefrorenes Tabakgewebe wird zunächst in einem Mörser grob zerstossen und dann in einen geeigneten Homogenisations-Puffer [(1) 7.56 M Guanidin HCl, 0.73 M Mercaptoethanol, 18.9 mM Natriumacetat pH 5.0; oder (2) 4% (w/v) SDS, 0.1 M Tris-HCl pH 7.8 (1 Volumenteil) + 80% Phenol (v/v), 0.1% (w/v) Hydroxyquinolin, 0.1 M Tris-HCl pH 7.8 (1 Volumenteil); oder (3) gemäss Lagrimini LM *et al*, (1987)] gegeben (2.5 ml Puffer pro Gramm Gewebe). Nach Zugabe eines gleichen Volumensteils Phenol wird dieser Ansatz homogenisiert, z.B in einem Polytron Homogenisator. Anschliessend wird ein halbes Volumen Chloroform zugesetzt und die Emulsion für etwa 15 Minuten vorsichtig durchmischt. Die verschiedenen Phasen werden dann über eine Zentrifugation (10,400 g für 10 Minuten) voneinander getrennt; die wässrige Phase wird verworfen. An dieser Stelle können wahlweise weitere Extraktionsschritte angeschlossen werden, z.B. in Form einer zusätzlichen Phenol-Chloroform Extraktion oder einer zweimaligen Extraktion mit einem Gemisch aus Phenol:Chloroform:Isoamylalkohol (25:24:1). An die Extraktion schliesst sich der Präzipitationsschritt an. Dieser erfolgt durch Zugabe von 0.3 M Natriumacetat und 2.5 Volumenteilen Ethanol. Das Präzipitat wird durch Zentrifugation gesammelt (10.400 g für ca. 15 Minuten) und in 2 ml sterilem Wasser resuspendiert. Nach Zugabe von Lithiumchlorid in einer Endkonzentration von 3 M wird der gesamte Ansatz bei 4°C über Nacht inkubiert. Das Präzipitat wird dann erneut durch Zentrifugation gesammelt und das gebildete Pellet mit eingekühltem Ethanol gewaschen. Das Pellet wird anschliessend getrocknet und in 500 µl sterilem Wasser resuspendiert. Die Konzentration der Gesamt-RNA in dieser Präparation wird spektrophotometrisch bestimmt.

Altemativ zu dem zuvor beschriebenen Verfahren kann die Gesamt-RNA auch aus Kallusgewebe isoliert werden. Auch in diesem Fall kommen die zuvor beschriebenen Verfahrensschritte zur Anwendung, wobei als Ausgangsmaterial jedoch in Würfel geschnittenes Kallusgewebe (ca. 3 mm) verwendet wird, das vor dem Homogenisations-Schritt zunächst in flüssigem Stickstoff tiefgefroren und anschliessend in einem vorgekühlten Mörser zu einem freinen Pulver zerstossen wird.

### 4.2. Isolierung polyadenylierter RNA

Poly(A)+ RNA wird mit Hilfe einer Oligo-d(T) Cellulosechromatographie (Collaborative Research, Lexington, MA, USA) gemäss an sich bekannter Verfahren isoliert [siehe z.B. Mohnen (1979)].

Oligo-d(T) Cellulose wird zunächst 15 Minuten in 20 Volumenteilen 2.0 M NaCl gewaschen, anschliessend in sterilem Wasser suspendiert und auf eine Säule (1.5 cm Durchmesser und 20 cm Länge) gepackt. Die Säule wird mit einer Pufferlösung (440 mM NaCl, 0.9 mM EDTA, 9 mM Tris-HCl, pH 7.5; oder 0.5 M NaCl, 10 mM Tris-HCl, pH 7.5) gewaschen bis das Eluat einen pH-Wert zwischen 7.0 und 7.6 aufweist. Anschliessend werden RNA Lösungen mit einem RNA-Anteil zwischen 0.6 mg und 6.0 mg RNA in einem Volumen von 4.0 ml auf eine Endkonzentration von 1 mM EDTA und 10 mM Piperazin-1,4-bis(2-ethansulfonsäure), pH 7.5 eingestellt. Die RNA wird durch 5 minütiges Erhitzen bei 70°C und anschliessendes Abkühlen auf Eis denaturiert. Die gesamte Lösung wird dann mit 0.1 Volumenteilen 4 M NaCl auf einen Wert von 0.36 M NaCl eingestellt. Die RNA Lösung wird auf die Säule gegeben und die nicht polyadenylierte RNA [poly (A)- RNA] mit der oben erwähnten Pufferlösung eluiert. Die Absorption der Eluate wird mit Hilfe eines Spectrophotometers (Hitachi Modell 100-40, Hitachi, Tokyo, Japan) sowie eines angeschlossenen W+W Rekorders (Scientific Instruments, Basel, Schweiz) bestimmt. Sobald die Absorption die Basislinie erreicht hat, wird die an die Säule gebundene poly (A)+ RNA mit 1 mM EDTA, 10 mM Tris-HCl pH 7.5 eluiert. Die Eluate werden in eine Lösung aus 0.5 mM EDTA und 0.3 M Natriumacetat pH 5.0 gegeben und durch Zugabe von 2.5 Volumenteilen Ethanol präzipitiert. Die RNA wird anschliessend durch Zentrifugation bei 83'000 x g (30 bis 45 Minuten) gesammelt, unter Stickstoff getrocknet und in 1 mM EDTA, 10 mM Tris, pH 7.5 oder in Wasser resuspendiert.

### 4.3. Konstruktion und Selektion von cDNA Klonen

Poly(A)+ RNA wird mit Hilfe einer präparativen Ultrazentrifugation (17 Stunden bei 57'000 g) über einen 5-25%igen (w/v) Saccharosegradienten (1 mM EDTA; 10 mM Tris-HCl, pH 7.5) in einzelne Fraktionen zerlegt. Die Fraktionen, welche die Infomation für Chitinase enthalten, lassen sich anhand einer *in vitro* Translation unter Verwendung von anti-Chitinase Antikörpern identifizieren. Diese werden dann vereinigt und für die weitere Aufarbeitung verwendet.

Das Vorgehen für die Herstellung von anti-Chitinase Antikörpern ist dem Fachmann bekannt und kann beispielsweise gemäss dem bei Shinshi *et al* (1985) bzw. bei Mohnen (1985) beschriebenen Verfahren durchgeführt werden. Dabei werden im wesentlichen Emulsionen mit gereinigten Chitinasepräparationen in komplettem Freunds Adjuvans in drei Monate alte weibliche Kaninchen (New Zealand white rabbit) injiziert. Weitere Injektionen folgen nach einer bzw. zwei Wochen sowie anschliessend in monatlichen Intervallen. Die Blutentnahme erfolgt jeweils 7 bis 10 Tage nach der Injektion, wobei die Serumproben bei -20°C aufbewahrt werden. Immunglobulin G wird über eine Affinitätschromatographie an einer Protein A-Sepharose C1 4B Säule (Pharmacia) gereinigt, anschliessend lyophilisiert und bei -20°C aufgehoben.

Die Synthese doppelsträngiger DNA ausgehend von der poly(A)+ RNA Matritze, die Klonierung dieser doppelsträngigen DNA in pBR322, die differentielle Koloniehybridisierung und die Plasmid Isolierung werden im wesentlichen gemäss den Vorschriften und der Beschreibung bei Maniatis *et al* (1982) durchgeführt.

Für die Synthese von ca. 0.8 µg doppelsträngiger DNA werden 3 µg der zuvor isolierten und mit Chitinase mRNA angereicherten poly(A)+ RNA mit reverser Transkriptase (Life Sciences, St. Petersburg, FL) sowie DNA Polymerase I (New England Biolabs, Beverly, MA) inkubiert. Die so gebildete cDNA wird mit Hilfe der 'Homopolymeric dC-dG Tailing' Methode, die es erlaubt die cDNA und die Vektor DNA mit komplementären kohäsiven Enden zu versehen und die bei Maniatis *et al* (1982) [Seite 217 - 219] im einzelnen beschrieben ist, in die PstI Schnittstelle von pBR322 eingespleisst. Der mit PstI linearisierte umd mit oligo-dC Enden versehene Vektor pBR322 ist kommerziell erhältlich.

Das resultierende rekombinante Plasmid wird dann für die Transformation kompetenter *E.coli* DH1 Zellen verwendet. Die Klonierung in Plasmiden ist bei Maniatis *et al* (1982) auf den Seiten 242-246 und 391 im Detail beschrieben.

Die auf diese Weise konstruierte cDNA Bibliothek, die in Form von Bakterienkolonien auf Agarplatten vorliegt, wird dann zunächst mit Hilfe der differentiellen Koloniehybridisierung unter Verwendung radioaktiv markierter cDNA gescreent.

Im Rahmen der differentiellen Koloniehybridisierung werden von den Bakterienkolonien Duplikate auf Nitrocellulosefiltern hergestellt, indem man die Filter auf die Agarplatte auflegt und dann vorsichtig wieder abzieht. Die Original-Agarplatte wird für die spätere Identifizierung positiver Kolonien aufbewahrt. Die mit Bakterienkolonien behafteten Filter werden anschliessend auf ein Nährmedium gelegt und dort belassen, bis die Kolonien zu ca. 2 mm grossen Klonen ausgewachsen sind. Die Filter werden dann mit Natronlauge behandelt, was zu einer Lyse der Bakterienzellen und zur Denaturierung und Fixierung der bakteriellen DNA auf dem Filter führt. Nach pH-Neutralisierung werden die Filter mehrmals gewaschen, getrocknet und schliesslich bei einer Temperatur von 80°C unter Vakuum "gebacken", wodurch die DNA kovalent an den Filter gebunden wird.

Die Filter werden 2 mal hintereinander mit radioaktiv markierten (nicht-klonierten) cDNA Sonden hybridisiert. Bei diesen cDNA Sonden handelt es sich um poly(A)+ RNA aus Tabakgewebe, das zuvor zur Produktion von Chitinase induziert wurde (siebentägige Inkubation auf Basalmedium ohne Hormonzusätze) sowie um poly(A)+ RNA aus nicht-induziertem Tabakgewebe (sieben tägige Inkubation auf Auxin-Cytokinin Medium). Vielversprechende cDNA Klone, die stärker mit der cDNA aus induziertem Gewebe reagieren als mit der Kontroll-DNA aus nicht-induziertem Gewebe, werden dann mit Hilfe des 'Hybrid-select' Translationsverfahrens gemäss der Beschreibung bei Mohnen *et al* (1985) und Mohnen (1985) weiter analysiert. Die Plasmid DNA wird durch einminütiges Kochen in 0.2 M - 0.3 M NaOH, 3 M NaCl denaturiert und anschliessend auf Eis abgekühlt. Die Hybridisierungsreaktion wird auf quadratischen BA/85 Nitrocellulosefiltern (Schleicher und Schüll, Dassel, FRG) mit 200 µg bis 250 µg Gesamt-RNA pro Filter, durchgeführt. Die auf den Filtern hybridisierte RNA wird eluiert, mit Ethanol präzipitiert und in 10 µl Wasser gelöst und mit Hilfe der *in vitro* Translation (mit Hilfe eines kommerziell erhältlichen Weizenkeimextraktes) analysiert. Die radioaktiv markierten Produkte werden mit Antikörpern gegen das gewünschte Protein gefällt und durch SDS-Polyacrylamid-Gelelektrophorese analysiert.

### 4.4. cDNA Klon pCHN48

Die cDNA Genbibliothek wird gemäss Maniatis *et al* (1982) mit Hilfe der Kolonie- bzw. Plaque-Hybridisierung gescreent. Als DNA Probe dient der bei Shinshi *et al* (1987) beschriebene cDNA Klon pCHN50, der die gesamte, das reife Protein-kodierende DNA Sequenz enthält, dem aber die komplette N-terminale Signalpeptidsequenz fehlt. Man erhält auf diese Weise verschiedene Klone unterschiedlicher Länge. Der längste dieser Klone wird ausgewählt und einer Nukleotidsequenzanalyse unterzogen. Dieser Klon mit der Bezeichnung pCHN48 besitzt ein 1.14 Kb umfassendes Insert mit 7 Adenosinen am Poly(A) Ende, einen einzigen grossen Leserahmen ('open reading frame') von 987 Nukleotiden Länge, entsprechend einem Polypeptid von 329 Aminosäuren sowie einem Nukleotid aus der 5'-nicht-translatierten Region. Die aus der DNA Sequenz der kodierenden Region ableitbare Aminosäuresequenz stimmt mit der Sequenz für die ersten 20 N-terminalen Aminosäuren bekannter Chitinasen aus Tabak überein.

### Beispiel 5: Konstruktion einer genomischen Genbibliothek

### 5.1. Isolierung chromosomaler Tabak-DNA

Zur Lysierung der Protoplasten werden 100 ml eisgekühlte Protoplastensuspension mit 400 ml eines eisgekühlten TENP-Puffers (vgl. Abschnitt VII) vermischt. Die Kerne werden aus diesem Lysat mit Hilfe einer 10 minütigen Zentrifugation in einer IEC klinischen Zentrifuge bei 2'000 Upm abgetrennt. Das so erhältliche Pellet wird in 500 ml eingekühltem TENP-Puffer resuspendiert und, wie zuvor beschrieben, erneut pelletiert. Anschliessend werden 8 CsCl Gradientenröhrchen vorbereitet, wobei in jedem der Röhrchen ein Zellpellet aus ca. 12.5 ml Protoplastensuspension zu 26 ml 10fach konzentrierten TE Puffers (vgl. Abschnitt VII) zugegeben wird. Zur Lyse der Zellkerne wird diesem Ansatz 5 ml einer 20%igen (w/v) Natriumlaurylsarkosin Lösung zugesetzt sowie 32.2 g CsCl und 2.89 ml Ethidumbromidlösung (EtBr, 10 mg/ml). Der gesamte Ansatz wird leicht durchmischt um das CsCl zu lösen.

Die so gewonnenen Lysate werden in Polyallomer-Röhrchen (Beckman VTi50, 39 ml Röhrchen) übertragen und bei 45'000 Upm und einer Temperatur von 20°C 16 Stunden lang in einem VTi50 Rotor (Beckman) zentrifugiert. Im UV-Licht fluoreszierende Banden werden mit Hilfe von 3 ml Spritzen mit 16 Gauge Nadeln aus dem Gradienten abgezogen und gesammelt. Die weitere Aufarbeitung der DNA erfolgt durch Wiederholung der zuvor beschriebenen CsCl/EtBr Gleichgewichtszentrifugation. Die fluoreszierenden Banden werden erneut gesammelt und anhaftendes EtBr wird in 6 aufeinanderfolgenden Extraktionsschritten unter Verwendung von Isopropanol, der mit 20 x SSC (vergl. Abschnitt VII) gesättigt ist (gleiches Volumen), entfernt. Aus dieser auf die zuvor beschriebene Weise gereinigten Gradientenlösung wird die DNA ausgefällt, indem man nacheinander 2 Volumenteile destilliertes Wasser, 0.1 Volumenteile 3.0 M Natriumacetat, pH 5.4 und 2 Volumenteile Ethanol zugibt. Die fadenförmige DNA wird mit Hilfe einer Pasteurpipette aus der Lösung heraus aufgewickelt, in 70% Ethanol gewaschen und mit einem gleichen Volumenteil Chloroform weiter extrahiert. Die DNA wird durch Zugabe von 0.1 Volumenteilen 3 M Natriumacetat, pH 5.4 und 2.0 Volumenteilen Ethanol präzipitiert. Der DNA Faden wird erneut aufgewickelt, in 70% Ethanol gewaschen und 5 Minuten an der Luft getrocknet. Anschliessend wird er in einem Gesamtvolumen von 7 ml TE-Puffer (vergl. Abschnitt VII) gelöst und bis zur weiteren Verwendung bei 4°C aufbewahrt.

### 5.2. Konstruktion einer genomischen λ Genbibliothek

Die zuvor isolierte Tabak DNA wird mit dem Restriktionsenzym Sau3A verdaut und 20 Stunden über einen 10% - 40%igen Saccharosegradienten in einem SW41 Rotor (Beckman) bei 20'000 Upm aufgetrennt. Die aus diesem Gradienten erhältlichen Fraktionen werden mit Hilfe einer Gelelektrophorese (0.5% Agarosegel in TBE Puffer) analysiert. Diejenigen Fraktionen, die Fragmente der richtigen Grösse aufweisen, werden gepoolt. Die DNA wird mit 1/10 Volumenteil 3 M Natriumacetat (pH 4.8) un 2 Volumenteilen Ethanol ausgefällt und mit BamHI-verdauter, Phosphatase-behandelter λ EMBL3 DNA (Stratagen; La Jolla, CA) ligiert. Die Verknüpfungsreaktion wird gemäss den Angaben des Herstellers durchgeführt, wobei jeweils Reaktionsansätze von 5 µl verwendet werden, die 1 µg λ-Vektor-DNA sowie 0.1 µg der einzubauenden Tabak DNA enthalten. Der Einbau der aus dieser Verknüpfungsreaktion resultierenden DNA in die Köpfe von λ-Phagen wird mit Hilfe des Gigapack Plus Kits von Stratagene gemäss den Angaben des Hersteller durchgeführt. Die Phagenausbeute nach Infektion von *E.coli* CES201 (Glover DM,) beträgt ca. 2 x 10⁶ Phagen pro µg inserierter DNA.

### 5.3. Screening der Genbibliotheken und Isolierung genomischer Klone

Die genomische Genbibliothek wird gemäss Maniatis *et al* (1982) mit Hilfe der Kolonie- bzw. Plaque-Hybridisierung gescreent. Als DNA Probe dient der bei Shinshi *et al* (1987) beschriebene cDNA Klon pCHN50 sowie der in Abschnitt 4.4. isolierte Klon pCHN48.

Die auf diese Weise erhältlichen Rekombinanten werden gereinigt und mit Hilfe der Southern Blot-Analyse teilweise charakterisiert. Einer dieser Klone, der mit den cDNA Probenmolekülen pCHN50 und pCHN48 ein sehr starkes Hybridisierungssignal erkennen lässt und gemäss Southern Blot-Analyse das komplette Chitinasegen enthält, wird für die weiteren Untersuchungen ausgewählt und erhält die Bezeichnung λCHN17.

### Beispiel 6: Der genomische Tabak-Chitinase Klons λCHN17

### 6.1 DNA-Sequenzierung und Sequenzanalyse

Nach Verdauung mit dem Restriktionsenzym HindIII erhält man ein 5.4 Kb umfassendes DNA Fragment, welches das komplette Chitinasegen enthält und mit einem Fragment gleicher Grösse aus Tabak DNA korrelliert. Ein Teil dieses Fragments, das 3850 Bp umfasst und die gesamte kodierende Sequenz beinhaltet, wird anschliessend sequenziert.

Restriktionsfragmente werden in geeigneten Klonierungsvektoren, wie z.B, M13mp18 oder M13mp19 [Yanisch-Perron *et al* (1985)] kloniert und in beiden Richtungen mit Hilfe der Dideoxynukleotid Methode ['Dideoxynucleotide chain-termination method'; Sanger *et al* (1977)] sequenziert. Die ermittelten Nukleotid- und Aminosäuresequenzen werden mit Hilfe des Computers unter Verwendung einer Genetics Computer Group Software [Devereux *et al* (1984)] weiter analysiert.

Die komplette DNA Sequenz des basischen Chitinasegens aus Tabak, das die Bezeichnung Gen 48 erhält, ist in SEQ ID NO: 10 wiedergegeben. Ein Vergleich dieser Sequenz mit den cDNA Klonen pCHN48 und pCHN50 macht deutlich, dass innerhalb der kodierenden Region von Gen 48 zwei intervenierende Sequenzabschnitte (Intron) zwischengeschaltet sind. Das erste dieser Introns umfasst 274 Bp und befindet sich zwischen dem 1. und 2. Nukleotid des für Glycin-kodierenden Kodons 148. Das zweite Intron ist 269 Bp lang und befindet sich zwischen dem 2. und 3. Nukleotid des Histidin-kodierenden Kodons 199. Beide Introns besitzen in Übereinstimmung mit anderen, bekannten pflanzlichen und tierischen Introns Consensus-Spleissstellen, die eine Donor- und eine Akzeptorsequenz (GTAAGTC und ACAG) aufweisen. Darüberhinaus besitzen beide Introns die Sequenz CT(G/A)A(C/T), 33 Nukleotide vom 3' Border entfernt, die Ähnlichkeiten zu tierischen Consensussequenzen (PyTPuAPy) aufweist. Diese Consensussequenzen sind an der Ausbildung schleifenförmiger Intermediärprodukte im Rahmen der Excission beteiligt.

Die Nukleotidsequenz der Exons von Gen 48 ist identisch mit der DNA Sequenz der kodierenden Region von Klon pCHN48.

### Beispiel 7: Herstellung einer c-DNA Genbibliothek aus Gurkenblättern

### 7.1 Reinigung und Proteinsequenz der Gurkenchitinase

Ein durch Pathogene induzierbares Chitinaseprotein wird aus infizierten Gurkenblättern gemäss der bei Métraux (1988) beschriebenen Methode isoliert. Aus dieser homogenen Proteinpräparation werden dann Peptidfragmente anhand allgemein bekannter Verfahren generiert. Die Aminosäuresequenzen dieser Peptidfragmente sind im folgenden summarisch wiedergegeben:

Amino-Terminus Ala Gly Ile Ala Ile Tyr Trp Gly Gln Asn Gly
Asn Glu Gly Ser Leu Ala Ser Thr Cys Ala Thr
Gly Asn Tyr Glu Phe Val Asn Ile Ala Phe Leu

LysC Peptide (Lys) Asn Phe Gly Gln Val Ile Leu Ser Ala Ala
Pro Gln Cys Pro Ile Pro Asp Ala His Leu Asp
Ala Ala Ile Lys
(Lys) Thr Gly Leu Phe Asp Ser Val Trp Val Gln
Phe Tyr Asn Asn Pro Pro Cys Met Phe Ala Asp
Asn Ala Asp Asn Leu Leu Ser
(Lys) Leu Tyr Met Gly Leu Pro Ala Ala Arg Glu
Ala Ala Pro Ser Gly Gly Phe Ile Pro Ala Asp
(Lys) Ala Ser Ser Asn Tyr Gly Gly Val Met Leu
Trp Ser Lys

CNBR Peptide (Met) Phe Ala Asp Asn Ala Asp Asn Leu Leu Ser
(Met) Gly Leu Pro Ala Ala Arg Glu Ala Ala Pro
Ser Gly Gly Phe Ile Pro Ala Asp Val Leu Ile
Ser Gln Val Leu Pro Thr Ile

Trpyptische
Peptide Val Leu Leu Ser Ile Gly Gly Gly Ala
Thr Gly Leu Phe Asp ? Val
Leu Tyr Met Gly Leu Pro Ala Ala
Ala Ser Ser Asn Tyr Gly Gly Val
Ala Phe Asp Asn Gly Tyr

### 7.2 Herstellung einer cDNA Bibliothek aus TNV-infizierten Gurken blättern

Gurkenblätter werden mit Tabak Nekrosis Virus (TNV) infiziert. 5 Tage nach der Infektion wird die RNA gemäss obiger Beschreibung isoliert [vergl. Beispiel 4.1].

Polyadenylierte RNA [Poly(A)+ RNA] wird mit Hilfe von Standardverfahren [vergl. Abschnitt 4.2] isoliert und für die Herstellung einer cDNA Genbibliothek verwendet. Diese wird im wesentlichen gemäss dem bei Gubler und Hoffman (1983) beschriebenen Verfahren in einem λ Zap Klonierungsvektor [STRATAGEN] hergestellt.

### 7.3 Isolierung von cDNA Klonen, die eine Gurkenchitinase kodieren

Zwei Regionen aus den zuvor bestimmten Proteinsequenzen werden ausgewählt für die Herstellung von Oligonukleotidproben. Die synthetisierten Oligonukleotidproben decken alle möglichen Kombinationen von mRNA ab, die in der Lage sind die ausgewählten Peptide zu kodieren:

                G  T        A
Probe 1:    5' - CCATTCTGNCCCCAGTA - 3'

G   G   G   G   C
Probe 2:   5′ - GGATTATTATAAAATTGNACCCA - 3′

Etwa 300'000 Plaques werden von der zuvor konstruierten cDNA Bibliothek ausplattiert. Duplizierte Abdrücke dieser Plaques werden mit einem ³²P-markierten Oligonukleotidgemisch 1 (Probe 1) oder 2 (Probe 2) getestet. Plaques die mit beiden Proben positive Resultate erbringen werden isoliert. Die Isolierung der Plaques sowie die automatische Excision werden gemäss den Angaben des Herstellers [Stratagene Lambda Zap Laboratory Manual, Stratagen, San Diego, USA] durchgeführt.

Ein geeigneter positiver Klon wird in das Bluescript' Plasmid eingespleisst und erhält die Bezeichnung pBSCucCht5. Die Sequenz dieses c-DNA Klons kann durch Dideoxy-Sequenzierung bestimmt werden [siehe SEQ ID NO 11].

### II. Konstruktion von Chitinase- und Glucanasemutanten

### II.1. Chitinasemutanten

### Beispiel 8 Einfügen neuer Restriktionsschnittstellen

Bei die Konstruktion der Chitinasemutanten werden verschiedene Teile der jeweils verwendeten Chitinaseklone miteinander verknüpft. Um dieses Ziel erreichen zu können müssen diese Teile kompatibel sein, d.h. es müssen geeignete Restriktionsschnittstellen in die verschiedenen Sequenzen eingefügt werden. Im folgenden werden die nachfolgend aufgelisteten Restriktionsschnittstellen verwendet, die alle zu den gleichen kohäsiven Enden führen:
BamHI [G/GATCC]
BclI [T/GATCA]
BglII [A/GATCT]

Der Leseraster wird jeweils so gewählt, dass GAT als Kodon für Aspartat fungiert.

### Beispiel 9 Konstruktion von Mutanten des Tabakchitinasegens

Durch Einspleissen des EcoRI/HindIII Inserts aus pCHN87, einem subklonierten Fragment des genomischen Chitinase-Klons CHN17 [zur Herstellung vergl. Abschnitt 12.2], in das Plasmid pTZ18R erhält man pTRCH1. Das Plasmid pTZ18R kann von der Firma PHARMACIA kommerziell bezogen werden.

Das Insert des cDNA Klons pCHN48 [vergl. Abschnitt 4.4], das die gesamte cDNA Sequenz enthält, wird durch partielle Verdauung mit PstI isoliert und in das Plasmid pUC8 einkloniert. Der so entstandene Klon erhält die Bezeichnung pCHN6.

Das grosse PstI Fragment aus pCHN6 wird in das Plasmid pUC18 einkloniert, wobei zwei verschieden Klone entstehen [pUCH2 und pUCH3], die sich in der Orientierung des in den Polylinker eingespleissten Inserts unterscheiden.

Das EcoRI/HindIII Fragment aus pUCH2 wird anschliessend isoliert und in das Plasmid pTZ18U eingespleisst. Man erhält aus diese Weise das Plasmid pTUCH2. Das Plasmid pTZ18U kann ebenfalls bei der Firma PHARMACIA kommerziell bezogen werden.

Das EcoRI/HindIII Fragment aus pUCH3 wird ebenfalls isoliert und in das Plasmid pTZ18R einkloniert. Der so gebildete Klon erhält die Bezeichnung pTRCH3.

### Beispiel 10 Oligonucleotid-vermittelte Mutagenese

Für die Oligonucleotid-vermittelte Mutagenese werden die folgenden Oligonukleotide mit Hilfe gängiger, dem Fachmann bekannter Methoden, synthetisiert:

#1 CTGCCTCGGC TGATCA ATGTGG

#2 TTTGGAAATT GACTC TTAGTCG

#3 CCAG AGATCT TTTGGGAAATGG

#4 GACTTTTAGTCAATACTATGTAA

#5 GACTTTTAGTCCGTACTATGTAA

#6 CTTTTGGAAA AGATCT TTTAGTCG

#7 CCGCTCTTC GGATCC GGCTGG

#8 CAGCATCGGΔ TGATCA GGAAGCTC

#9 CATCT TCTAGA TTTAGTCTC

Die gegenüber dem Wildtyp veränderten Nukleotide sind unterstrichen und fettgedruckt.
Die Restriktionsschnittstellen sind durch einen Leerschlag hervorgehoben.
Die Deletion in Oligonukleotid #8 ist durch Δ wiedergegeben.
Die Insertion in den Oligonukleotiden #3 und #8 ist kursiv wiedergegeben.

### 10.1 Mutagenese der Tabakchitinaseklone

Zur Herstellung von Einzelstrang-Plasmiden werden die Ausgangsplasmide in einen dam Stamm von *E. coli* eingeführt, durch Infektion einer Übemachtkultur dieser Bakterien mit dem Helferphagen M13KO7 [PHARMACIA]. Diese Plasmide können anschliessend sehr einfach mit Hilfe von Ammoniumacetat und Polyethylenglykol (PEG 6000) aus dem Kulturüberstand ausgefällt und mit Phenol/Chloroform und Chloroform extrahiert werden. Nach erneuter Fällung mit Ethanol wird die isolierte DNA für die eigentliche Mutagenese verwendet [DNA aus ca. 5 ml Kultur je Mutagenese].

200 pMol eines jeden Oligonukleotids werden in 30 µl 0.1 M Tris-HCl (pH 7.5), 10 mM MgCl₂, 6 mM Dinitrothreitol (DTT), 2 mM ATP und 5 Einheiten T4 Polynukleotidkinase für einen Zeitraum von 45 Minuten bei einer Temperatur von 37°C phosphoryliert. Durch anschliessende 10 minütige Inkubation bei 60°C wird die Reaktion gestoppt.

Die einzelsträngige DNA wird mit 2.5 µl phosphoryliertem Oligonukleotid und 1 µl Lösung A [0.2 M Tris-HCl (pH 7.5), 0.1 M MgCl₂, 0.5 M NaCL, 10 mM DTT] in einem Endvolumen von 30 µl gemischt und zunächst 5 Minuten bei 80°C und anschliessend 20 Minuten bei Raumtemperatur inkubiert.

Jedem Röhrchen werden dann 13 µl der folgenden Mischung zugegeben:

| | |
|---|---|
| 18.5 µl | H₂O |
| 2.0 µl | Lösung B [0.2 M Tris-HCl (pH 7.5), 0.1 M MgCl₂, 0.1 DTT] |
| 2.0 µl | 10 mM ATP |
| 2.0 µl | dNTP-Gemisch (je 10 mM dATP, dCTP, dGTP, dTTP) |
| 1.0 µl | Klenow Fragment |
| 0.5 µl | T4 DNA Ligase |

Diese Mischung wird 30 Minuten bei einer Temperatur von 32°C und anschliessend nochmals 2 bis 16 Stunden bei Raumtemperatur inkubiert.

Ein Viertel dieses Ansatzes wird in kompetente *E. coli* mutS Zellen (reparaturdefizienter Stamm) transformiert. Diese Zellen werden 3 bis 6 Stunden in 2 x L- oder 2 x TY-Medium bei 37°C geschüttelt. Anschliessend wird die Plasmid-DNA mit Hilfe einer minipräparativen Methode, die dem Fachmann auf diesem Gebiet geläufig ist, isoliert.
Die isolierte DNA wird dann in kompetente *E. coli* DH5a Zellen transformiert, die auf einem Ampicillin-haltigen Medium ausplattiert werden. Die Mutanten könne dann sehr einfach durch Koloniehybridiserung unter Verwendung des radioaktiv markierten mutagenen Oligonukleotids identifiziert werden. Zusätzlich kann eine Restriktionsanalyse durchgeführt werden, da alle Mutanten aufgrund der Mutation eine Schnittstelle hinzugewonnen oder aber verloren haben.

### 10.1.2 pTRCH4

Plasmid pTRCH1 wird mit Hilfe von Oligonukleotid #1 mutagenisiert. Das neu entstandene Plasmid wird mit pTRCH4 bezeichnet. Im Rahmen dieser Mutation wird eine BclI Restriktionsschnittstelle am ersten Kodon der für die reife Chitinase kodierenden DNA Sequenz eingefügt, wobei die kodierte Aminosäure von Glutaminsäure zu Asparaginsäure verändert wird [Glu1 → Asp1]

### 10.1.3 pTRCH6

Plasmid pTRCH3 wird mit Hilfe von Oligonukleotid #2 mutagenisiert. Das neu entstandene Plasmid wird mit pTRCH6 bezeichnet. Im Rahmen dieser Mutation wird Kodon 300 der für die reife Chitinase kodierenden DNA Sequenz von Glycin zu einem Stop-Kodon verändert (Gly300 → Stop300), gleichzeitig wird eine HinfI Restriktionsschnittstelle eingefügt.

### 10.1.4 pTUCH6

Das HindIII/PvuII Fragment von pTRCH3 und das PvuII/HindIII Fragment von pTRCH6, welche die obige Mutation enthalten, werden in den zuvor mit HindIII/EcoRI geschnitten Vektor pTZ18U eingespleisst. Auf diese Weise erhält man das Plasmid pTUCH6.

### 10.1.5 pTUCH7

Plasmid pTUCH2 wird mit Hilfe von Oligonukleotid #3 mutagenisiert. Das neu entstandene Plasmid wird mit pTUCH7 bezeichnet. Im Rahmen dieser Mutation wird im Bereich von Kodon 295/296 der für die reife Chitinase kodierenden DNA Sequenz eine BglII Restriktionsschnittstelle eingefügt. Da sich diese Restriktionsschnittstelle in einem anderen Leseraster befindet als dies bei den anderen bisher beschriebenen Mutanten der Fall ist, wird bei Kodon 297 ein G inseriert. Man erhält auf diese Weise wieder einen kompatiblen Leseraster.

### 10.1.6 pTUCH8

Plasmid pTUCH2 wird mit Hilfe von Oligonukleotid #4 mutagenisiert. Das neu entstandene Plasmid wird mit pTUCH8 bezeichnet. Im Rahmen dieser Mutation wird Kodon 304 der für die reife Chitinase kodierenden DNA Sequenz von Asparaginsäure zu Asparagin [Asp304 → Asn304] verändert. Dies führt zum Verlust einer TaqI Schnittstelle.

### 10.1.7 pTUCH9

Plasmid pTUCH2 wird mit Hilfe von Oligonukleotid #5 mutagenisiert. Das neu entstandene Plasmid wird mit pTUCH9 bezeichnet. Im Rahmen dieser Mutation wird Kodon 304 von Asparaginsäure zu Arginin [Asp304 → Arg304] verändert, was ebenfalls zum Verlust einer TaqI Schnittstelle führt.

### 10.1.8 pTUCH10

Plasmid pTUCH2 wird mit Hilfe von Oligonukleotid #6 mutagenisiert. Das neu entstandene Plasmid wird mit pTUCH10 bezeichnet. Im Rahmen dieser Mutation wird Kodon 299 von Asparagin zu Lysin [Asn299 → Lys299] verändert und Kodon 300 von Glycin zu Asparaginsäure [Gly300 → Asp300]. Gleichzeitig wird eine BglII Schnittstelle eingefügt.

### 10.2 Mutagenese der Gurkenchitinase Klone

Die Mutagenese der Gurkenchitinase Klone wird analog zu dem in Beispiel 11.1 beschriebenen Verfahren durchgeführt.

Das EcoRI Insert aus dem Gurkenchitinase cDNA Klon pBSCucCht5 [vergl. Abschnitt 7] wird in das Plasmid pTZ18U einkloniert. Das auf diese Weise konstruierte Plasmid erhält die Bezeichnung pTUCU2.

### 10.2.1 pTUCU4

Plasmid pTUCU2 wird mit Hilfe von Oligonukleotid #7 mutagenisiert. Das neu entstandene Plasmid wird mit pTUCU4 bezeichnet. Im Rahmen dieser Mutation wird eine BamHI Restriktionsschnittstelle am letzten Kodon der für das Signalpepetid kodierenden DNA Sequenz eingefügt, wodurch das nachfolgende Kodon so verändert wird, dass es nunmehr für die Aminosäure Prolin anstelle von Alanin kodiert [Ala -1 → Pro -1]

### 10.2.2 pTUCU5

Plasmid pTUCU2 wird mit Hilfe von Oligonukleotid #8 mutagenisiert. Das neu entstandene Plasmid wird mit pTUCU5 bezeichnet. Im Rahmen dieser Mutation wird eine BclI Restriktionsschnittstelle im Bereich des Stop-Kodon der Gurken-Chitinase eingefügt. Dadurch wird das Stop-Kodon in ein Asparaginsäure Kodon umgewandelt [Stop268 → Asp268]

### 10.2.3 pTUCU6

Plasmid pTUCU2 wird mit Hilfe von Oligonukleotid #9 mutagenisiert. Das neu entstandene Plasmid wird mit pTUCU6 bezeichnet. Im Rahmen dieser Mutation wird eine XbaI Restriktionsschnittstelle im Bereich der 3'-nicht-kodierenden Sequenz der cDNA Sequenz eingefügt [im Bereich der Nukleotide 981-986 von Klon pBSCucCht5], wodurch ein Einklonieren in den Vektor pGY1 möglich wird.

### II.2 Glucanasemutante

### Beispiel 11: PCR-vermittelte Mutagenese

Das für die erste Aminosäure der C-terminalen Extension kodierende Basentriplett [GTC = Valin] wird unter Anwendung der PCR-Methode in das Stoppkodon TGA umgewandelt.

Die Verfahren zur Durchführung einer PCR Mutagenese sind dem Fachmann auf diesem Gebiet bestenst bekannt. Sie sind beispielsweise in den folgenden Referenzen beschrieben: Wang *et al* (1989); Innis *et al* (1990) und Erlich (1989).
Darüberhinaus kann ein entsprechender PCR-Kit von PERKIN-ELMER CETUS [Norwalk, Conn., USA] oder anderen Herstellern kommerziell bezogen und entsprechenden den darin enthaltenen Instruktionen durchgeführt werden.

### 11.1.: Konstruktion von pCIB1005BΔVTP

Dieses Konstrukt kann ausgehend von dem Plasmid pCIB 1005B, das bei der 'American Type Culture Collection' [ATCC] in Rockville, Maryland, USA unter der Nummer ATCC 40770 hinterlegt und dessen Konstruktion in EP-A 0,392,225 im Detail beschrieben ist, hergestellt werden.

Plasmid pCIB1005B kodiert eine komplette, chimäre, basische Prepro-β-1,3-Glucanase aus Tabak mit vollständiger N-terminaler Signalsequenz und einer 22 Aminosäuren umfassenden C-terminalen Extension, in einem CaMV 35S Expressionsvektor.
Plasmid pCIB1005BΔVTR, dessen Herstellung im folgenden beschrieben wird, kodiert die gleiche Glucanase, wobei jedoch die 22 Aminosäuren umfassende, C-terminale Extension durch Einfügen eines Stopp-Kodons am Ende der für das reife Protein kodierenden Sequenz funktionell deletiert ist.

Im Rahmen der PCR Amplifikation werden die folgenden Oligonukleotide verwendet:

Oligo1:	5'-	GGG ACA CAC GTG CAC CTT	- 3'

Oligo2: 5'- CTG TCC CAA ACT CCA CCA GAT CAC CCA AAG TTG
... ATA TTA TAT T . - 3'

Oligo3: 5'- AAT ATA ATA TCA ACT TTG GGT GAT CTG GTG GAG
TTT GGG ACA G - 3'

Oligo4: 5'- GCC TCC CCT TCA TCG TCC - 3'

Oligonukleotid 1 [Oligo1] korrespondiert zu einer Sequenz in der kodierenden Region der Glucanase-DNA die stromaufwärts ['upstream'] der dort befindlichen XhoI Schnittstelle lokalisiert ist.

Oligonukleotid 3 [Oligo3] umfasst die Region im Bereich des 3'-Endes der für das reife Glucanaseprotein kodierenden Sequenz gefolgt von dem einzuführenden Stopp-Kodon sowie einem Teil der für die C-terminale Extension kodierenden Sequenz.

Oligonukleotid 2 [Oligo2] weist eine zu Oligo3 korrespondierende Sequenz auf, jeoch in anti-sense Orientierung.

Oligonukleotid 4 [Oligo4] umfasst eine Sequenz die in der tml 3'- Region von pCIB1005B lokalisiert ist, stormabwärts ['downstream'] der dort befindlichen SacI Schnittstelle.

Es werden drei PCR Reaktionen durchgeführt:
(1) PCR1 mit Oligo1 und Oligo2 als Primer und pCIB1005B als Template;
(2) PCR2 mit Oligo2 und Oligo4 als Primer und pCIB1005B als Template;
(3) PCR3 mit Oligo1 und Oligo4 als Primer und den Produkten der PCR1 und PCR2 Reaktion als Template. Die ersten beiden Zyklen der PCR3 Reaktion werden nur mit den Templates durchgeführt, erst anschliessenden werden die Primer zugegeben.

Das Produkt der PCR3 Reaktion wird mit SacI und XhoI verdaut und über ein Agarosegel aufgetrennt. Die 725 Bp entsprechende Bande wird aus dem Gel ausgeschnitten und gereinigt.

Anschliessend wird Plasmid pCIB1005B ebenfalls mit XhoI und SacI verdaut und über Nacht mit dem verdauten und gereinigten PCR3 Fragment in einer Ligasereaktion verknüpft. Das Ligationsgemisch wird dazu verwendet um kompetente *E.coli* DH5a Zellen zu transformieren. Die Selektion der Transformanten erfolgt durch Ampicillin. Die Plasmide pCIB1005B und pCIB1005BΔVTP können aus Übemacht-Kulturen transformierter Zellen isoliert und für die anschliessende Transfektion von *Nicotiana plumbaginifolia* Protoplasten verwendet werden. Die Korrektheit der auf diese Weise erhältlichen Konstrukte wird durch Sequenzanalyse bestätigt.

### III. Einbau der Chitinasemutanten in ein Pflanzen-Expressionsplasmid

### Beispiel 12 Konstruktion von Plasmid pSCH10

Plasmid pSCH10 enthält die für Tabak-Chitinase kodierende Sequenz aus dem genomischen Klon CHN17 sowie dem cDNA Klon CHN48, eingespleisst im Vektorplasmid pGY1. Flankien wird diese Sequenz vom 35S Promotor des CaMV Virus sowie von dessen Terminationssequenzen. Bei diesem Konstrukt handelt es sich somit um die Wildtyp-Chitinasekonstruktion. Diese wird im folgenden im Detail beschrieben.

### 12.1 Konstruktion von Plasmid pGY1

Das Plasmid pGY1 ist abgeleitet von dem bei Pietrzak *et al* (1986) beschriebenen pflanzlichen Expressionsvektor pDH51. Im Plasmid pGY1 ist die NcoI Schnittstelle des Ausgangsplasmids pDH51 gegen eine XhoI Schnittstelle ersetzt.

Das Plasmid pDH51 wird mit NcoI geschnitten und die überstehenden Enden werden mit Klenow Polymerase aufgefüllt. Die nunmehr glatten Enden werden dann mit einem XhoI Linker (CCTCGAGG) ligiert.

### 12.2 Konstruktion von Plasmid pSCH10

Plasmid pSCH10 enthält die Chitinase-kodierende Sequenz, die 5'-transkribierte nicht-kodierende Sequenz sowie 21 stromaufwärts der Transkriptionsstartstelle gelegene Basenpaare des Chitinasegens 48 sowie eine 31 Bp umfassende nicht-kodierende Sequenz, eingespleisst in die BamH1/PstI Klonierungsstelle des CaMV 35S Pflanzenexpressionsvektors pGY1.

Die zur Herstellung von pSCH10 notwendigen Verfahrensschritte sind im folgenden näher beschrieben:
(1) Der genomische Klon λCHN17, der das Chitinasegen 48 enthält, wird mit HindIII geschnitten. Das resultierende 5.6 Kb umfassende HindIII Fragment wird isoliert und in die HindIII Klonierungsstelle des Plasmids pUC8 eingespleisst, unter Bildung von Plasmid pCHN65.
(2) Plasmid pCHN65 wird mit EcoRI geschnitten und das resultierende 2.5 Kb umfassende Fragment, welches das 5'-Ende der Chitinase-kodierenden Region enthält, wird ebenfalls in pUC8 einkloniert. Das entstehende Plasmid erhält die Bezeichnung pCHN68.
. (3) Plasmid pCHN68 wird mit PstI verdaut und unter Verlust eines 0.5 Kb umfassenden PstI/EcoRI Fragmentes religiert (mit sich selbst verknüpft). Das resultierende Plasmid wird mit pCHN74 bezeichnet.
(4) Um die 5'-nicht kodiertende Region des Transkriptes von Gen 48 hinter eine BamHI Schnittstelle zu klonieren, wird das Plasmid pCHN74 zunächst mit HphI verdaut, anschliessend durch Einwirkung von T4 DNA Polymerase mit glatten Enden versehen und schliesslich mit PstI geschnitten. Das HphI/PstI Fragment wird isoliert und in das mit HincII/PstI verdaute Plasmid pUC8 [Vieira & Messing (1982)] eingespleisst, unter Bildung von pCHN87.
   Nach Sequenzierung lässt sich anhand der DNA Sequenz zeigen, dass während des letzten Verfahrensschrittes eine Base der 1/2 HincII Schnittstelle verlorengegangen ist.
(5) pCHN87 wird mit EcoRI und HindIII verdaut und in den Klonierungsvektor pUC9 [Vieira & Messing (1982)] eingespleisst. Das resultierende Plasmid erhält die Bezeichnung pCHN88.
(6) Das 1 Kb umfassende PstI Fragment des Tabak cDNA Klones 48 (pCHN 48) wird in die PstI Klonierungsstelle von Plasmid pUC8 eingespleisst unter Bildung von pCHN78.
(7) Das PstI Insert von Plasmid pCHN78 wird durch Schneiden des Plasmids mit dem Restriktionsenzym PstI freigesetzt und in die PstI Stelle von pCHN88 eingespleisst, wodurch die komplette, für Chitinase kodierende DNA Sequenz wiederhergestellt wird. Das resultierende Plasmid erhält die Bezeichnung pCHN89.
(8) Das Plasmid pCHN89 wird anschliessend vollständig mit BamHI und teilweise mit PstI verdaut. Das resultierende 1.5 Kb umfassende Fragment wird in den pflanzlichen Expressionsvektor pGY1, der zuvor ebenfalls mit BamHI und PstI geschnitten wurde, einkloniert, unter Bildung von pSCH10. Durch diesen Klonierungsschritt wird die Chitinase-kodierende DNA Sequenz zwischen den CaMV Promotor und die CaMV Terminationssequenz plaziert.

### Beispiel 13 Einbau der Chitinasemutanten in das pflanzliche Expressionsplasmid pSCH10

### 13.1 Einbau der Tabak-Chitinasemutanten

Das PstI Fragment von Plasmid pSCH10 wird durch die folgenden PstI Fragment ersetzt:
PstI Fragment von pTUCH6 → pSCM3
PstI Fragment von pTUCH7 pSCM25
PstI Fragment von pTUCH8 → pSCM22
PstI Fragment von pTUCH9 pSCM23
PstI Fragment von pTUCH10 → pSCM24

Diese Plasmide enthalten nunmehr mutierte 3'-terminale 'Targeting'-Sequenzen, welche die folgende Nukleotid-Sequenzen aufweisen:

pSCH10: 5'- GGA AAT GGA CTT TTA GTC GAT ACT AGT TAA -3'

pSCM24: 5'- GGA AAA GAT CTT TTA GTC GAT ACT ATG TAA -3'

pSCM22: 5'- GGA AAT GGA CTT TTA GTC AAT ACT ATG TAA -3'

pSCM23: 5'- GGA AAT GGA CTT TTA GTC CGT ACT ATG TAA -3'

Die gegnüber dem Wildtyp-Plasmid [pSCH10] veränderten (mutierten) Nukleotide sind fettgedruckt und unterstrichen.

### 13.2 Einbau der Gurken-Chitinasemutanten

### 13.2.1 Plasmid pSCU1

Das EcoRI/BclI Fragment aus dem Plasmid pSCM1 und das BamHI/EcoRI Fragment aus pTUCU4 werden in das zuvor mit EcoRI geschnittene Plasmid pTZ18U einkloniert. Es entsteht das Plasmid pTUCU7.

Das EcoRI/NsiI Fragment aus dem Plasmid pTUCU7 und das NsiI/EcoRI Fragment aus pTUCU6 werden in das zuvor mit EcoRI geschnittene Plasmid pTZ18U einkloniert. Es entsteht das Plasmid pTUCU11.

Plasmid pTUCU11 wird dann mit BamHI und XbaI verdaut und das BamHI/XbaI Fragment isoliert. Dieses wird anschliessend in das zuvor mit BamHI/XbaI geschnittene Plasmid pGY1 eingespleisst, wobei das Plasmid pSCU1 entsteht.

### 13.2.2 Plasmid pSCU3

Das EcoRI/NsiI Fragment aus dem Plasmid pTUCU7 und das NsiII/EcoRI Fragment aus pTUCU5 werden anschliessend in das zuvor mit EcoRI geschnittene Plasmid pTZ18U einkloniert. Man erhält aus diese Weise das Plasmid pTUCU10.
Plasmid pSCU3 entsteht durch Einklonieren des XhoI/BclI Fragmentes von Plasmid pTUCU10 und des BglII/PstI Fragmentes von Plasmid pTUCU7 in das zuvor mit XhoI/PstI geschnittene Plasmid pGY1.

### 13.2.3 Plasmid pSCU6

Durch Einklonieren des XhoI/BclI Fragmentes aus pTUCU10 und des BglII/PstI Fragmentes aus pTUCH10 in das zuvor mit XhoI/PstI geschnittene Plasmid pGY1 gelangt man zu Plasmid pSCU6.

Die Plasmide pSCU3 und pSCU6 enthalten nunmehr im Gegensatz zum Kontrollplasmid pSCU1 3'-terminale 'Targeting'-Sequenzen, welche die folgende Nukleotid-Sequenzen [5'→ 3'] aufweisen:

Tabakchitinassegen
TTG GGA AAT GGA CTT
pSCU3: ATC GGT GAT CTT TTA GTC GAT ACT ATG TAA
Gurkenchitinasegen Tabakchitinasegen
pSCU6: ATC GGT GAT CTT TTA GTC GAT ACT ATG TAA
BclI/BglII Ligation

Die gegnüber dem Wildtyp veränderten (mutierten) Nukleotide sind fettgedruckt und unterstrichen.
Die *via* Insertion neu eingefügten Nukleotide sind kursiv angegeben.

### Beispiel 14 Einspleissen der pSCM- und pSCU-Konstrukte in einen binären Pflanzenvektor

### 14.1 Konstruktion von Plasmid pCIB200

Die Konstruktion dieses binären Vektors geht aus von dem bei Schmidhauser und Helinski (1985) beschriebenen Plasmid pTJS75, einem Abkömmling von RK2 (An G *et al*, 1985), das einen weiten Wirtsbereich umfasst und ein Tetrazyklinresistenzgen aufweist. Dieses Plasmid wird mit dem Restriktionsenzym NarI geschnitten und anschliessend mit dem AccI Fragment von pUC4K [Vierra und Messing (1982)], welches das NptI-Gen trägt, verknüpft. Das auf diese Weise gebildete Plasmid pTJS75kan, das neben dem Tetrazyklinresistenzgen nunmehr auch das NptI-Gen beinhaltet, wird dann mit dem Restriktionsenzym SalI verdaut.

Gleichzeitig wird das Plasmid pCIB7, das bei Rothstein SJ *et al* (1987) beschrieben ist, mit EcoRV geschnitten und das resultierende EcoRV Fragment, welches die linke und rechte T-DNA Grenzsequenz aus dem Ti-Plasmid von *Agrobacterium tumefaciens* sowie ein chimäres Nos/NptII Gen und die pUC Polylinkerregion enthält, wird mit XhoI-Linkem verknüpft.

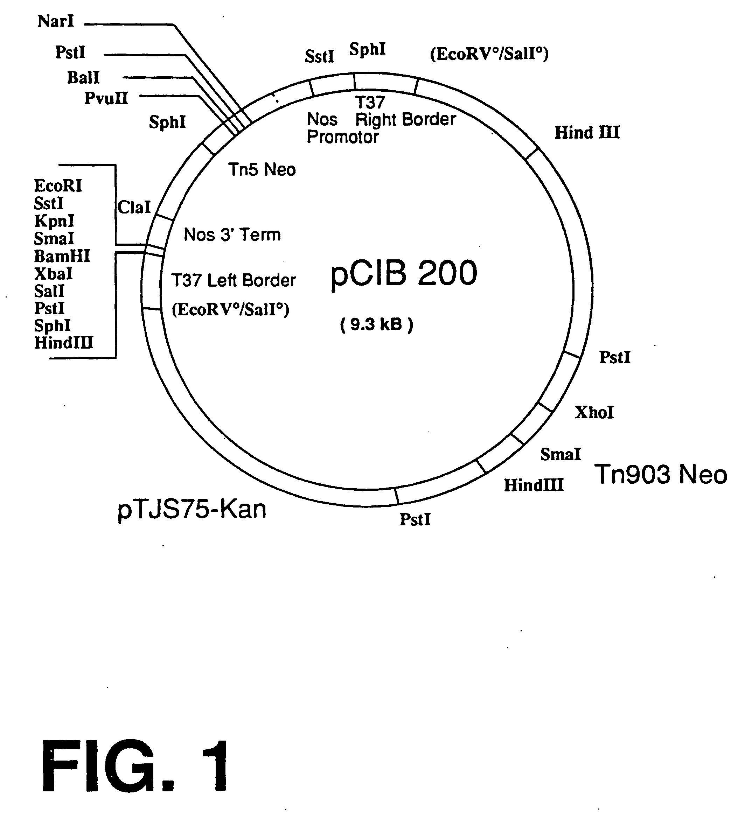

Das resultierende Konstrukt wird dann mit XhoI verdaut und in die SalI Schnittstelle des Plasmids pTJS75kan einkloniert. Das resultierende Plasmid, dessen Genkarte in Figur 1 wiedergegeben ist, erhält die Bezeichnung pCIB200.

### 14.2 Einklonieren der EcoRI-Fragmente in pCIB200

Plasmid pCIB200 wird zunächst mit EcoRI geschnitten. Anschliessend werden die folgenden EcoRI Fragmente in das geschnittene pCIB200 einkloniert:
EcoRI Fragment aus Plasmid pSCH10 → pSCH12
EcoRI Fragment aus Plasmid pSCM3 → pSCM13
EcoRI Fragment aus Plasmid pSCU1 → pSCU11
EcoRI Fragment aus Plasmid pSCU3 → pSCU13

### IV. Transformation in Tabak Protoplasten

Die zuvor beschriebenen Plasmide werden *in vitro* unter Verwendung eines 'Transient Expression Systems' in *Nicotiana plumbaginifolia* Protoplasten getestet.

### Beispiel 15 Herstellung und Kultivierung von Nicotiana plumbaginifolia Protoplasten

Die Herstellung und Kultivierung von *Nicotiana plumbaginifolia* Protoplasten erfolgt mit Hilfe allgemein bekannter Verfahren, analog der für Tabak [vergl. Abschnitt 3] beschriebenen Vorgehensweise. Eine detaillierte Beschreibung findet sich bei Shillito und Potrykus (1987)oder bei Negrutiu *et al*, 1987.

### Beispiel 16 Transformation der Protoplasten

Die Transformation der *Nicotiana plumbaginifolia* Protoplasten wird gemäss dem bei Negrutiu *et al* (1987) beschriebenen Verfahren durchgeführt.

Die gemäss obigem Verfahren hergestellten Protoplasten werden nach dem letzten Reinigungsschritt in einer Lösung suspendiert, die folgende Zusammensetzung aufweist:

| | |
|---|---|
| Mannit | 0.4 M |
| CaCl₂ | 15-30 mM |
| MES | 0.1% (w/v) |

Die Protoplastendichte liegt bei 1.6 - 2 x 10⁶/ml.

3 ml dieser Protoplastensuspension werden zunächst in 15 ml Zentrifugen-Röhrchen mit 5 µg Plasmid DNA vermischt, die in Form einer sterilen, wässrigen Suspension vorliegt [Paszkowski *et al* (1984)]. Wenige Minuten später gibt man zu dieser Mischung 0.3 ml einer Polyethylenglycollösung [40% (w/v) PEG 6000 in 0.4 M Mannit, 0.1 M Ca(NO₃)₂, pH 7.0] zu und vermischt diesen Ansatz vorsichtig. Einige Minuten später werden 4 ml K3-Medium [Z Pflanzenphysiol, 78: 453-455 (1976); Shillito *et al,* (1981)] zugegeben und die Röhrchen für 24 Stunden liegend im Dunkeln bei einer Temperatur von 25°C bis 27°C inkubiert.

Um die Pelletiereigenschaften der Protoplasten zu verbessern, kann man anschliessend ein W5-Salzlösung [Negrutiu *et al*, 1987] [5.4 ml] zusetzen. Die Protoplasten werden dann bei geringer Geschwindigkeit [ca. 60 -100 x g] zentrifugiert. Ein Aliquot des Überstandes wird für die spätere Analyse der Chitinaseaktivität entnommen, der Rest wird verworfen. Die abgetrennten Protoplasten werden im restlichen Medium resuspendiert und in Eppendorf-Röhrchen überführt, wo das Volumen annäherungsweise bestimmt wird. Aliquots dieser Suspension werden für die Analyse der Chitinaseaktivität sowie das Western Blotting verwendet.

Die Transformation der *Nicotiana plumbaginifolia* Protoplasten mit Plasmid pCIB1005B und pCIB1005BΔVTP kann auch nach dem von Goodall *et al* (1990) modifizierten 'Negrutiu'-Verfahren durchgeführt werden.

### V. TRANSFORMATION UND HERSTELLUNG TRANSGENER PFLANZEN

### Beispiel 17.1 Transformation von Agrobacterium tumefaciens mit binären Vektoren

Die zuvor in Beispiel 14 beschriebenen binären Vektoren werden unter Anwendung des folgenden Verfahrens in den *Agrobacterium tumefaciens* Stamm LB4404 transformiert. *A. tumefaciens* LBA 4404 enthält ein deletiertes Ti Plasmid, dem die T-DNA Region fehlt, das aber nach wie vor eine intakte vir-Region aufweist [Hoekema *et al* (1983)].

*Agrobacterium tumefaciens* Stamm LB4404 wird bei einer Temperatur von 30°C in einer über Nacht Kultur in 5 ml MG/L Medium [vergl. Abschnitt VIII] herangezogen. Zu dieser 5 ml über Nacht Kultur werden dann 250 ml MG/L Medium zugegeben und der ganze Ansatz wird gut durchmischt, bis eine optische Dichte von OD=0.6 (bei 600 nm) erreicht ist. Die Zellen werden dann über eine Zentrifugation bei 8'000 g gesammelt und in 5 ml MG/L Medium resuspendiert. 200 µl dieser Zellsuspension werden mit 0.2 µg bis 1 µg binärer Plasmid DNA in MG/L Medium inkubiert, wobei dieser Ansatz nach leichtem Durchmischen sofort in einem Trockeneis/Ethanol Bad tiefgefroren wird. Nach 5 Minuten wird das Röhrchen in ein 37°C Wasserbad gegeben und dort für 5 Minuten belassen. Danach werden 2 ml MG/L Medium zugegeben. Diese Suspension wird dann für 2 bis 3 Stunden in einem 30°C Wasserbad inkubiert. Anschliessend werden die Zellen über eine Zentrifugation gesammelt. Die Zellen werden in einem kleinen Volumen MG/L Medium resuspendiert und dann auf Selektivmedien (MG/L Platten mit 100 µg/ml Gentamycin) ausplattiert. Nach 2 bis 3 Tagen bei 30°C erscheinen die ersten Kolonien.

### Beispiel 17.2 Transformation von Agrobacterium tumefaciens mit binären Vektoren

In einer alternativen Ausführungsform werden die zuvor in Beispiel 14 beschriebenen binären Vektoren durch eine triparentale Kreuzung [Rogers SG *et al* (1986)], unter Verwendung eines *E. coli* Helferstammes, der ein Plasmid mit einer tra-Funktion besitzt, in den *Agrobacterium tumefaciens* Stamm LBA4404 überführt. Als *E.coli* Helferstamm kann z.B. *E. coli* BHB1011 verwendet werden, der das Plasmid pRK2013 enthält, das die für den Transfer der binären Vektoren notwendigen tra-Funktionen besitzt.

*A. tumefaciens* LBA4404 wird über Nacht bei 28°C in LB Medium mit 20 mg/l Rifampicin und 500 mg/l Streptomycin herangezogen.
*E. coli* BHB1011 und die *E. coli* Stämme mit den binären Vektoren wrden über Nacht bei 37°C in LB Medium mit 25 mg/l Kanamycin herangezogen.
Je 1 ml dieser Kulturen wird bei 8000 g abzentrifugiert, in 1 ml sterilem Wasser oder 10 mM MgSO₄ gewaschen, wieder abzentrifugiert und in 100 µl Wasser oder einer MgSO₄ Lösung resuspendiert. Eine Platte mit einem LB Festmedium wird in vier Sektoren unterteilt. In diesen Sektoren werden Tropfen der drei Bakterienkulturen übereinander aufgetragen und zwar so, dass in drei der vier Sektoren die drei möglichen Kombinationen zweier Kulturen gemischt werden. Diese dienen als Kontrollen. Im vierten Sektor dagegen werden all drei Kulturen zusammengemischt. Nach dem Eintrocknen der Tropfen werden diese Platten über Nacht bei 28°C inkubiert. Danach wird von jedem Sektor eine Probe entnommen und in Wasser oder MgSO₄ Lösung suspendiert. Von diesen Suspensionen werden Verdünnungen hergestellt und auf LB-Platten mit 20 mg/l Rifampicin, 500 mg/l Streptomycin und 25 mg/l Kanamycin ausplattiert und für zwei bis drei Tage bei ca. 28°C inkubiert. Kolonien, die bei hoher Verdünnung der triparentalen Kreuzung wachsen [bei ähnlicher Verdünnung der Kontrollkreuzungen darf nichts wachsen] werden durch wiederholtes ausplattieren einzelner Kolonien von eventuell noch vorhandenen Elternbakterien befreit.

### Beispiel 18 Blattscheiben- ('Leaf-disk') Transformation von N. sylvestris und N. tabacum

Die Blattscheiben-Transformation wird im wesentlichen gemäss dem bei Horsch *et al*, (1985) beschriebenen Verfahren durchgeführt.
*A. tumefaciens* LBA 4404 (pCIB200; pSCH12; pSCM13; pSCU11; pSCU13) wird über Nacht in einem Glutamat-Salz Medium, das mit 20 mg/l Rifampicin, 500 mg/l Streptomycin und 25 mg/l Kanamycin angereichert und auf einen pH-Wert von 5.6 eingestellt ist, bei einer Temperatur von 28°C herangezogen. In dieser über Nacht-Kultur, die ca. 3.3 x 10⁸ Zellen enthält, werden sterile Blattscheiben (5 mm bis 10 mm Druchmesser) von *N. sylvestris* oder *N. tabacum* cv. Havana 425 für 5 Minuten inkubiert. Sie werden dann aus der Kultur entnommen und auf sterilen Papierhandtüchern trockengetupft, bevor sie dann in Petrischalen mit einem Durchmesser von 100 mm überführt werden, die eine Nährkultur enthalten.

Diese Nährkultur besteht aus einem mit 1% Agar (DIFCO) verfestigten Basismedium (30 ml) nach Linsmeier und Skoog (1965), das als weitere Zusätze einen pH Indikator (Chlorphenolrot; 5 mg/l)sowie die Pflanzenwuchsstoffe Kinetin (0.3 mg/l) und a-Naphthylessigsäure (2 mg/l) enthält. Dieses Agar-Medium (Medium A) wird mit 1 ml bis 2 ml einer 2 Wochen alten Suspensionskultur von S275N Zellen, die sich aus Markgewebe von *N. tabacum* cv. Havana 425 (Eichholz et al, 1983) ableitet, überschichtet und mit einem Filterpapier (#1 Whatman filter paper) überdeckt. Auf dieses Filterpapier werden dann die Blattscheiben aufgebracht.

Nach 48 Stunden werden die Explantate zur Sprossinduktion auf ein Selektivmedium der gleichen Zusammensetzung gegeben, das darüberhinaus aber noch 350 mg/l Cefotaxim sowie 100 mg/l Kanamycin enthält (Medium B) und bei 25°C und diffusem Licht (80 bis 100 µEinstein) inkubiert. Ko-kultiviertes Kontrollgewebe wird auf das gleiche Medium ohne Kanamycin überimpft. Die Explantate werden wöchentlich auf frisches Medium B transferriert.

4 bis 8 Wochen nach der Ko-Kultivierung werden die sich aus den Explantaten entwickelnden grünen Sprosse geerntet und in 50 ml Gefässen auf 25 ml Medium C (Festmedium mit 0.6% Phytagar) überführt. Das gesamte Gewebe wird bei einer Temperatur von 24°C bis 28°C bei einer Beleutungsintensität von 80 bis 100 µEinstein kultiviert. Nach 1 bis 2 Wochen erfolgt die Bewurzelung der Sprosse.

### VI. ANALYSE DES TRANSGENEN PFLANZENMATERIALS

### (A) Indirekter Nachweis der Vakuolenlokalisation

### Beispiel 19 Analyse der transformierten Protoplasten

Die Analyse der transformierten Protoplasten wird gemäss den in den Abschnitten 17 und 20.3 im Detail beschriebenen Verfahren durchgeführt.

### 19.1: Chitinaseaktivität

Wie anhand der Aktivitätsdaten aus Tabelle 1 ersichtlich ist, besitzen die Kontrollprotoplasten zwar eine endogene Chitinaseaktivität. Im Überstand hingegen lässt sich nur eine geringe Aktivität nachweisen.

Im Gegensatz dazu zeigen alle Konstrukte der Tabak-Chitinase eine deutlich erhöhte Gesamtaktivität sowohl im Pellet als auch im Überstand. Die insgesamt erhöhte Chitinaseaktivität in allen getesteten Überstandsproben lässt sich vermutlich auf eine Überladung des zellulären Sortiersystems zurückführen. Dennoch sind deutliche Unterschiede erkennbar:

Bei den Konstrukten pSCH10, pSCM22, pSCM23 und pSCM24 liegt die Chitinaseaktivität im Pellet 3-4 mal höher als die in den Kontrollen gemessenen Aktivitäten. Beim pSCM3 Konstrukt dagegen ist die Chitinaseaktivität im Pellet lediglich um 35% erhöht.

Bei den Gurkenchitinase Konstrukten pSCU1, pSCU3 und pSCU6 findet man im Pellet lediglich eine um 10-20% erhöhte Chitinaseaktivität, während die Aktivität im Überstand um den Faktor 7 bis 8 (bzw. 4 für pSCU3) höher liegt im Vergleich zu den Kontrollen.

Die Ergebnisse der Tabelle 1 werden durch die Western Blot Resultate bestätigt. Die Western Blot Daten für die Gurkenchitinase Konstrukte z.B. zeigen deutlich, dass im Falle von pSCU1 (Wildtyp) nur sehr wenig Chitinase in den transformierten Protoplasten vorhanden ist, während in den mit pSCU3 und pSCU6 transformierten Protoplasten sehr hohe Chitinasekonzentrationen vorliegen.

### 19.2: Glucanaseaktivität

Die mit den Kontrollprotoplasten erzielten Ergebnisse [siehe Tabelle 10] zeigen, dass die β-1,3-Glucanase aus *Nicotiana plumbaginifolia* in der Zelle zurückgehalten wird.

Ein vergleichbares Ergebniss lässt sich bei Verwendung des intakten Konstrukts [pCIB1005B] erzielen, wobei in diesem Fall lediglich ein stärkeres Signal in den Protoplastenextrakten zu beobachten ist. Dies zeigt, dass auch die Tabak-Glucanase in den Protoplasten zurückgehalten wird und somit korrekt in das zelluläre Kompartiment dirigiert wird.

Bei Verwendung des Konstrukt mit deletierter bzw. inaktivierter C-terminaler Extension [pCIB1005BΔVTP] dagegen wird die β-1,3-Glucanase ins Kulturmedium sezerniert.

### Beispiel 20 Analyse der transformierten Pflanzen

Die gemäss Beispiel 18 transformierten Pflanzen werden mit Hilfe der im folgenden beschriebenen Verfahren analysiert.

### 20.1 Extraktion der Interzellulären Flüssigkeit (ICF)

Die Extraktion intercellulärer Flüssigkeit aus pflanzlichem Gewebe kann gemäss der bei Parent und Asselin ( 1984) beschriebenen Methode durchgeführt werden.
Dabei werden zunächst Blätter von regenerierten transgenen Pflanzen gesammelt und in Stücke von 4 bis 5 cm² zerschnitten. Diese werden dann im Vakuum jeweils für 30 Sekunden unter leichem Schütteln mit einem starken Überschuss kaltem (ca 4°C) Puffer infiltriert. Besagter Puffer weist vorteilhafterweise die folgende Zusammensetzung auf:

| | |
|---|---|
| Tris-HCl [pH 7.8] mit 0.5 M Saccharose | 25.0 mM |
| MgCl₂ | 10.0 mM |
| CaCl₂ | 10.0 mM |
| Phenylmethylsulfonylfluorid (PMSF) | 0.5 mM |
| 2-Mercaptoethanol | 5.0 mM |

Altemative dazu kann auch ein 50 mM Citratpuffer (pH 5.5) verwendet werden. Auch ein Arbeiten bei Zimmertemperatur ist möglich.

Beim Entfernen des Vakuums dringt der Puffer in die Blätter ein. Die Blattstückchen werden anschliessend vorsichtigt abgetrocknet und in eine 20 ml Spritze überführt. Diese wird in ein Zentrifugenröhrchen eingehängt und 10 Minuten bei niedrigen Geschwindigkeiten (ca. 1000 x g) und bei niedriger Temperatur (4°C) zentrifugiert.

### 20.2 Extraktion der Intrazellulären Proteinfraktion

Die gemäss Abschnitt 20.1 behandelten Blattstückchen werden anschliessend im gleichen Puffer homogenisiert. Die groben Partikel werden durch Zentrifugation abgetrennt.

### 20.3 Bestimmen der Chitinaseaktivität

Die Proteinkonzentration wird in beiden Extrakten mit Hilfedes BIORAD Protein Assay, der auf dem Bradford-Verfahrens basiert, bestimmt. Dieses beruht auf der Farbänderung eines Farbstoffes in Abhängigkeit von der Proteinkonzentration.

Die Chitinaseaktivität kann mit Hilfe eines radiometrischen Assays unter Verwendung von radioaktiv markiertem [tritiiertem] Chitin ermittelt werden [Boller *et al* (1983)]. Die Ergebnisse für die Tabakchitinase-Transformanten sind in Tabelle 2, für die Grukenchitinase-Transformanten in Tabelle 3 wiedergegeben.

Aus den Ergebnissen der hier durchgeführten Transformationsversuche mit Protoplasten und ganzen Pflanzen lässt sich somit zusammenfassend feststellen, dass das erfindungsgemässe Peptidfragment am C-terminalen Ende der basischen Chitinase aus Tabak für die gezielte Einschleusung der Chitinase in die Vakuole der Pflanze verantwortlich ist.
Darüberhinaus machen die Ergebnisse mit pSCM22, pSCM23 und pSCM24 deutlich, dass eine gewisse Variation innerhalb der Aminosäuresequenz am C-terminalen Ende erlaubt ist, ohne dass dadurch die Targeting Funktion dieser Sequenz verloren geht. Fehlt die 3'-terminale 'Targeting'-Sequenz [pSCM3; pSCM13] so wird ein Gossteil der ansonsten vakuolär vorliegenden Chitinase in den Extrazellularraum sezerniert.

Weiterhin kann anhand der vorliegenden Ergebnisse gezeigt werden, dass die erfindungsgemässe DNA-Sequenz auch bei operabler Verknüpfung mit einem heterologen Gen [Gurkenchitinasegen; pSCU3; pSCU6; pSCU13] als 'Targeting'-Signal fungiert, indem das natürlicherweise sezernierte Chitinase-Protein in der Pflanzenzelle und dort mit grosser Wahrscheinlichkeit innerhalb der Vakuole zurückgehalten wird.

### (B) Direkter Nachweis der Vakuolenlokalisation

### Beispiel 21.1: Protoplastenisolation (adaptiert nach Muller et al., 1983):

Blätter transgener *N.sylvestris-*Pflanzen werden in Stücke von 1-1.5 g geschnitten und in Petrischalen in ein K3M Osmotikum gelegt K3M enthält die halbe Konzentration der K3 Makroelemente (also auf 1 Liter 75 mg NaH₂PO₄·H₂O, 450 mg CaCl₂·2H₂O, 1250 mg KNO₃, 125 mg NH₄NO₃, 67 mg (NH₄)₂SO₄, 125 mg MgSO₄·7H₂O) und 84 g Mannit, pH 5.6; die Osmolarität ist auf 500 mOsm eingestellt. Die Blätter werden anschliessend in schmale Streifen geschnitten und im gleichen Osmotikum für eine Stunde inkubiert. Die Blattstreifen werden dann abgetropft und in 10 ml/Schale Verdauungslösung gelegt. Die Verdauungslösung enthält 0.4 % Macerozym R10 (Serva) und 0.6 % Cellulysin (Calbiochem) in K3M (das 75.6 g Mannit enthält, damit die Osmolarität mit den Enzymen wieder 500 mOsm beträgt). Die Petrischalen werden mit Parafilm verschlossen und über Nacht ohne Schütteln bei 26°C im Dunkeln inkubiert. Zur schnelleren Verdauung können die Blattstreifen auch mit der doppelten Enzymkonzentration vakuuminfiltriert werden und dann mit langsamem Schütteln (30-40 Upm) für 2-3 Stunden inkubiert werden.

Die Protoplasten werden durch einen 100 µm Filter filtriert, der dann mit einem halben Volumen 0.6M Saccharose gespült wird. Die Suspension wird in 15 ml Zentrifugenröhrchen überführt, mit 2 ml K3M überschichtet und 10 Minuten bei 1000 g zentrifugiert. Die Protoplasten werden von der Interphase abgesaugt, mit K3M verdünnt und 10 Minuten bei 700 g abzentrifugiert. Anschliessend werden die abzentrifugierten Protoplasten in K3M-Medium resuspendiert.

### Beispiel 21.2: Vakuolenisolation (adaptiert nach Boudet und Alibert, 1987):

Die Protoplasten werden in einem K3M-Medium (pH 6.5) enthaltend 20 % Ficoll resuspendiert. 2.5 ml davon werden in Zentrifugenröhrchen überführt und dann mit folgenden Lösungen suksessive überschichtet: 2 ml 15 % Ficoll, 7 mg DEAE-Dextran (pH 6.5); 2 ml 10 % Ficoll, 3 mg Dextransulfat (pH 8.0); 2 ml 6 % Ficoll, 3 mg Dextransulfat (pH 8.0); und ca. 4 ml 0 % Ficoll (pH 8.0) (randvoll). Diese Röhrchen werden in einem Ausschwingrotor (SW 41.14, Kontron) zuerst 15 Minuten bei 3500 Upm, dann 105 Minuten bei 40000 Upm zentrifugiert. Die Vakuolen werden von der 0-6 % Ficoll-Interphase abgesaugt.

Marker werden nach Boller und Kende (1979) gemessen. Zur Messung der Hexosephosphat Isomerase muss der Dextransulfat zuerst mit DEAE-Dextran ausgefällt werden, bevor die Messung möglich ist. Eine Proteinbestimmung nach Bradford ist nich möglich, da beide Polybasen diese Bestimmung stören. Die Resultate in Tabelle 5 wurden auf den Vakuolenmarkerenzym α-Mannosidase als 100 % normalisiert.
Der Vergleich der spezifischen Chitinaseaktivitäten in Tabelle 4 bestätigt die Resultate aus Tabelle 2:
Bei den mit Plasmid pSCM13 bzw. pSCU11 transformierten Pflanzen M13.4 und U11.4.2 findet man eine Sekretion der Chitinasen, während bei den mit Plasmid pSCH12 und pSCU13 transformierten Pflanzen M10.4 und U13.15 eine intrazelluläre Lokalisation eindeutig nachweisbar ist. Ein Westernblot bestätigt, dass die endogenen *N.sylvestris-*Chitinase intrazellulär vorliegt und bei den Protoplasten M13.4 für den grösseren Teil der residualen Aktivität verantwortlich ist.

Aus den Pflanzen M10.7.4 und U13.15 konnten Protoplasten isoliert werden. Die Messung verschiedener Marker (Tabelle 5) bestätigt die vakuoläre Lokalisation der Chitinasen, die eine C-terminale Sequenz tragen. Auch hier bestätigt ein Westernblot diese Lokalisation. Die endogene Chitinase ist ebenfalls vakuolär.

Von allen Serien konnten Samen einer oder mehrerer selbstbefruchteter Pflanzen gewonnen werden. Es konnte dabei nachgewiesen werden, dass die Chitinaseüberproduktion und die jeweilige Lokalisation an die Folgegenerationen weitervererbt wird.

### HINTERLEGUNG

Die folgenden Stämme sind bei der 'American Type Culture Collection' in Rockville, Maryland, USA gemäss den Bestimmungen des Budapester Vertrages hinterlegt:

| **Plasmid** | **Hinterlegungsdatum** | **Hinterlegungsnummer [ATCC]** |
|---|---|---|
| **pBscucchi/chitinase (Abk.: pBSCucCht5)** | **29.12.1988** | **40528** |
| **pBSGluc39.1** | **29.12.1988** | **40526** |
| **pCIB1005B** | **13.03.1990** | **40770** |

### MEDIEN UND PUFFERLÖSUNGEN

### Medium A

| | | |
|---|---|---|
| NH₄NO₃ | 1650 | mg/l |
| KNO₃ | 1900 | mg/l |
| CaCl₂x 2H₂O | 440 | mg/l |
| MgSO₄x 7H₂O | 370 | mg/l |
| KH₂PO₄ | 170 | mg/l |
| Na₂EDTA | 37.3 | mg/l |
| FeSO₄x 7H₂O | 27.8 | mg/l |
| H₃BO₃ | 6.2 | mg/l |
| MnSO₄x 4H₂O | 22.3 | mg/l |
| ZnSO₄x 7H₂O | 8.6 | mg/l |
| KI | 0.83 | mg/l |
| Na₂MoO₄x 2H₂O | 0.25 | mg/l |
| CuSO₄x 5H₂O | 0.025 | mg/l |
| CoCl₂x 6H₂O | 0.025 | mg/l |
| Saccharose | 30.0 | g/l |
| Thiamin.HCl | 0.400 | mg/l |
| myo-Inosit | 100.0 | mg/l |
| Kinetin | 0.3 | mg/l |
| a-Naphthylessigsäure 2.0 | | mg/l |
| Chlorphenolrot | 5.0 | mg/l |
| Agar | 10.0 | g/l |

### Medium B

### gleiche Zusammensetzung wie Medium A, jedoch ohne a-Naphthylessigsäure sowie folgenden Zusätzen:

| | | |
|---|---|---|
| Cefotaxim | 500 | mg/l |
| Kanamycin | 75 | mg/l |

### Medium C

### gleiche Zusammensetzung wie Medium B, jedoch ohne Kinetin

### MG/L Medium für Agrobacterium

| | |
|---|---|
| L-Broth | 50% |
| Mannit-Glutamat Medium (Holsters *et al* 1978) | 50% |

### K3M Osmotikum [500 mOsm; pH 5.6]

| | | |
|---|---|---|
| NaH₂PO₄·H₂O | 75 | mg/l |
| CaCl₂·2H₂O | 450 | mg/l |
| KNO₃ | 1250 | mg/l |
| NH₄NO₃ | 125 | mg/l |
| (NH₄)₂SO₄ | 67 | mg/l |
| MgSO₄·7H₂O) | 125 | mg/l |
| Mannit | 84 | g/l |

### W5-Salzlösung

| | |
|---|---|
| NaCl | 154 mM |
| CaCl₂ x 2H₂O | 125 mM |
| KCl | 5 mM |
| Glucose | 5 mM |
| pH 5.6-6.0 | |

### Rinse I-Lösung

| | | |
|---|---|---|
| Saccharose | 154.0 | g/l |
| MES | 0.59 | g/l |
| KNO₃ | 250.0 | mg/l |
| NH₄NO₃ | 25.0 | mg/l |
| NaH₂PO₄.H₂O | 15.0 | mg/l |
| CaCl₂.2H₂O | 90.0 | mg/l |
| MgSO₄.7H₂O | 25.0 | mg/l |
| (NH₄)₂SO₄ | 13.4 | mg/l |

### TENP Puffer

| | |
|---|---|
| Tris-HCl (pH 8.0) | 100 mM |
| EDTA | 10 mM |
| NP-40 (Sigma Chem.) | 1% (v/v) |

### TBE-Puffer

| | |
|---|---|
| Tris-Borat | 89 mM |
| Borsäure | 89 mM |
| EDTA | 2 mM |

### TE-Puffer

| | |
|---|---|
| Tris-HCl (pH 8.0) | 10 mM |
| EDTA | 1 mM |

### SSC

| | |
|---|---|
| NaCl | 1.54 mM |
| Natriumcitrat | 0.154 mM |
| (pH 7.0) | |

### TABELLEN

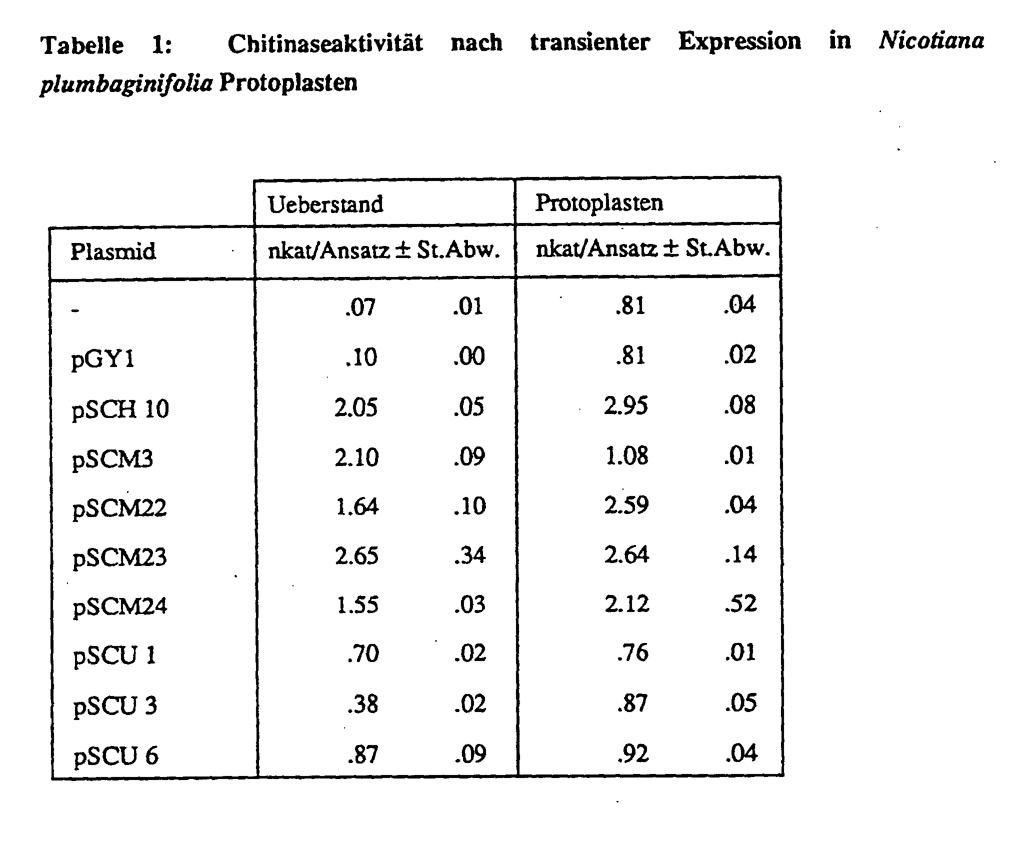

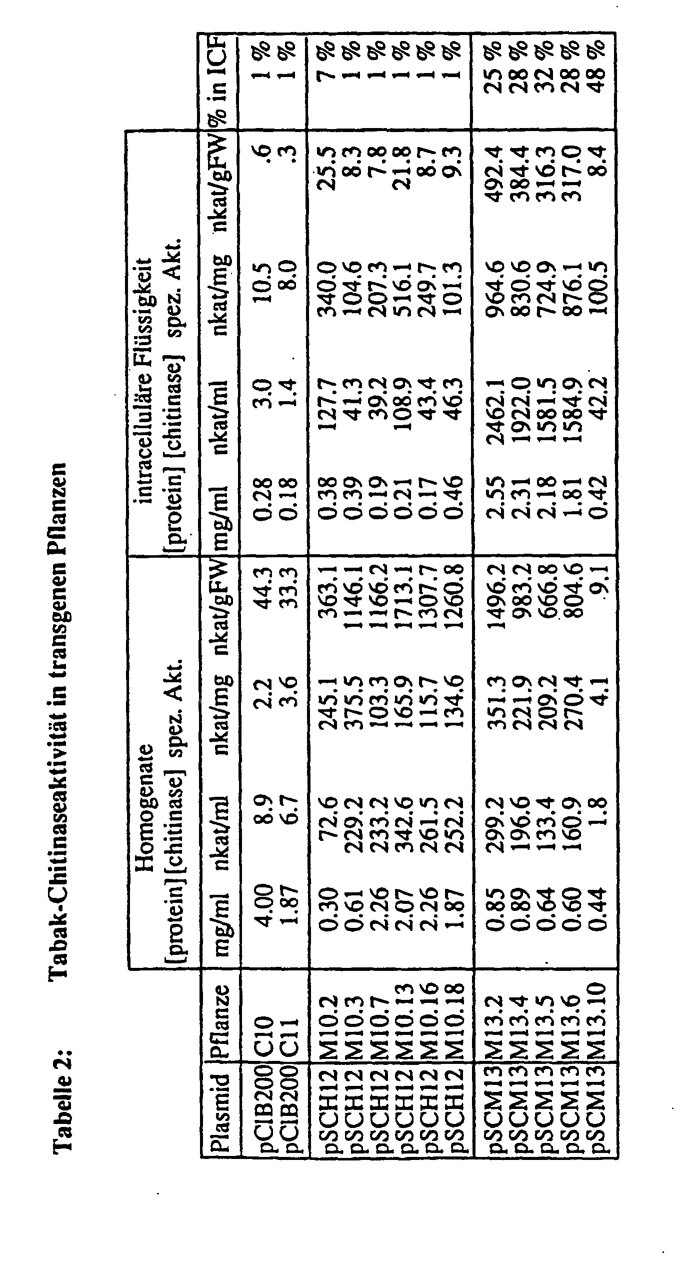

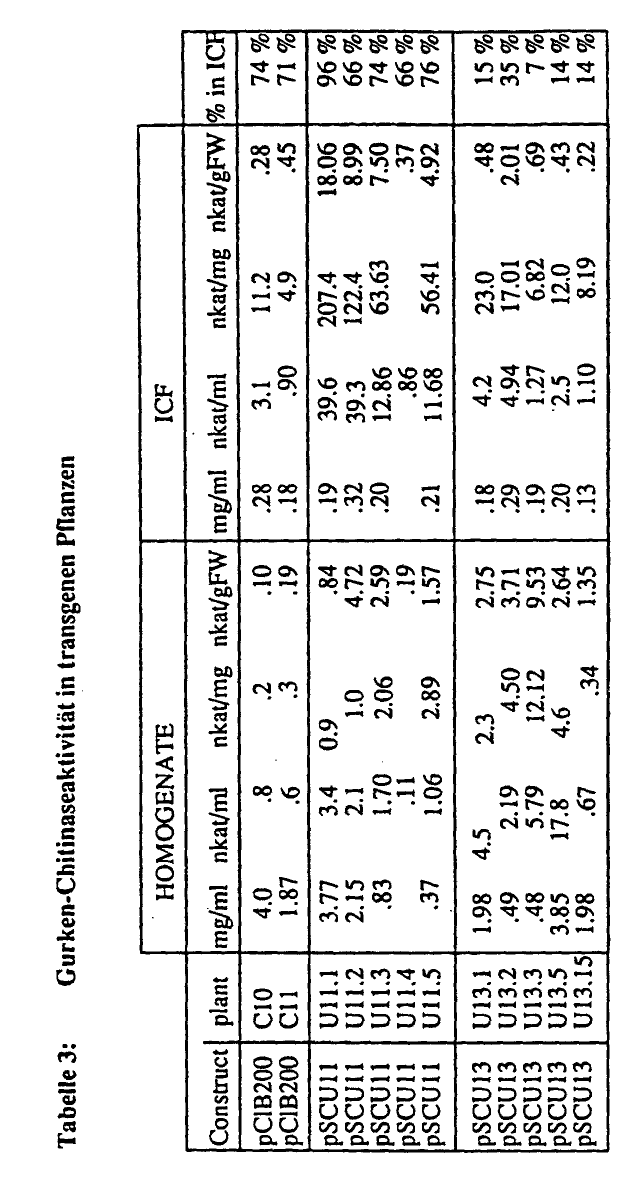

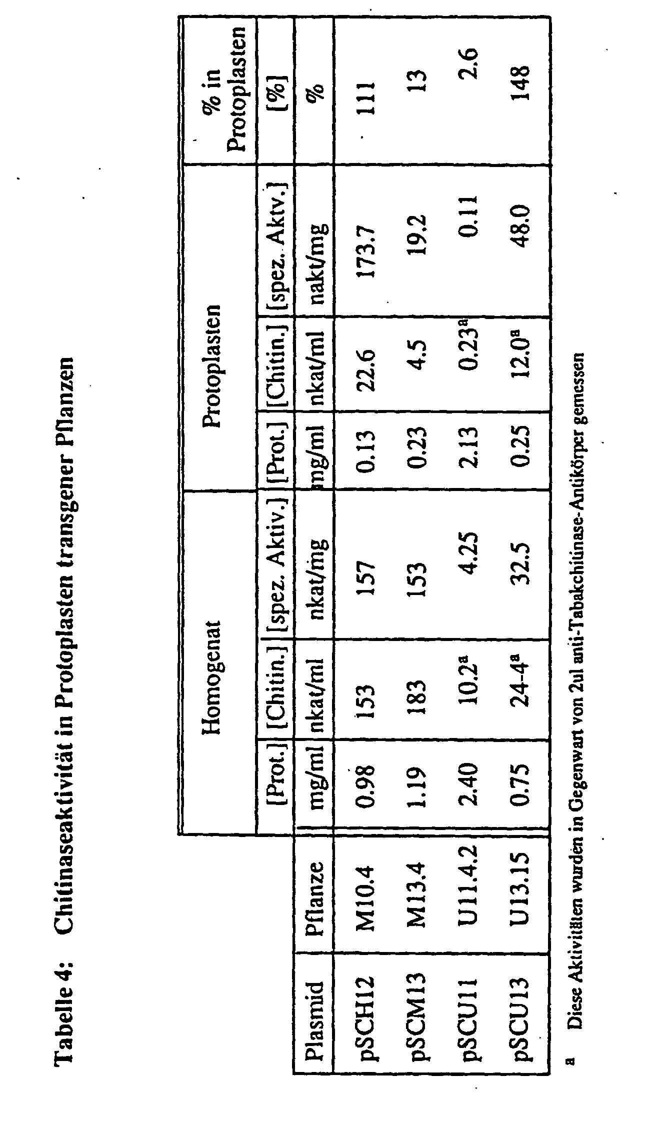

**Tabelle 5:**

| **Lokalisation intrazellulärer Marker in Vakuolenpräparationen aus Chitinase-überproduzierenden Pflanzen** | | | |
|---|---|---|---|
| Experiment 1. Lokalisation der Tabakchitinase mit ihrer C-terminalen Sequenz (Pflanze M10.7.4.) | | | |

| Marker | Units^{a}/10⁶ Protoplasten | Units/10⁶ Vakuolen^{b} | % total in Vakuolen |
|---|---|---|---|
| α-Mannosidase | 89 | 89 | 100 % |
| Tabakchitinase | 66300 | 75100 | 113 % |
| Hexose-6-Phosphate Isomerase | 2640 | 170 | 6 % |
| Chlorophyll | 27 | <4.5 | <17 % |
| Experiment 2. Lokalisation der Gurkenchitinase mit der C-terminalen Sequenz der Tabakchitinase (Pflanze U13.15) | | | |

| Marker | Units^{a}/10⁶ Protoplasten | Units/10⁶ Vakuolen^{b} | % total in Vakuolen |
|---|---|---|---|
| α-Mannosidase | 59 | 59 | 100 % |
| Gurkenchitinase | 12000 | 12200 | 102 % |
| Hexose-6-Phosphate Isomerase | 490 | 90 | 18 % |
| Chlorophyll | 62 | <2.2 | <4 % |

| | | | |
|---|---|---|---|
| ^{a} pkat für Enzyme, µg für Chlorophyll | | | |
| ^{b} Zahlen normalisiert für α-Mannosidase 100 % | | | |

**Tabelle 6:**

| **SEGREGATION DER KANAMYCINRESISTENZ IN DER ERSTEN NACHKOMMENGENERATION GESELBSTETER TRANSGENER PFLANZEN (erwartet wird 3 KanR: 1 KanS)** | | | |
|---|---|---|---|
| Plasmid | Elternpflanzen | KanR | KanS |
| pCIB200 | C2 | - | - |
| | C4 | 39 | 11 |
| pSCH12 | M10.3 | 27 | 12 |
| | M10.7 | 36 | 14 |
| | M10.13 | 39 | 9 |
| pSCM13 | M13.2 | 37 | 11 |
| | M13.4 | 39 | 14 |
| | M13.7 | - | - |
| | M13.10 | 24 | 12 |
| pSCU11 | U11.3 | 33 | 13 |
| | U11.4 | 15 | 39 |
| | U11.5 | 36 | 11 |

**Tabelle 7:**

| **ANALYSE DER NACHKOMMEN TRANSGENER PFLANZEN (nur KanR Pflanzen wurden getestet)** **Konzentration in homogenisierten Blättern** | | | | | |
|---|---|---|---|---|---|
| Plasmid | Eltempflanze | Pflanze | mg/ml | nkat/ml | nkat/mg |
| pCIB200 | C2 | C2.1 | 2.77 | 27.3 | 9.9 |
| | | C2.2 | 2.23 | 21.7 | 9.8 |
| | | C2.3 | 2.01 | 42.5 | 21.1 |
| | C4 | C4.1 | 2.88 | 8.3 | 2.9 |
| | | C4.2 | 2.82 | 9.6 | 3.4 |
| | | C4.3 | 2.15 | 6.1 | 2.8 |
| | | C4.4 | 2.66 | 7.2 | 2.7 |
| pSCH12 | M10.3 | M10.3.1 | 1.59 | 243.4 | 152.8 |
| | | M10.3.2 | 1.82 | 179.0 | 98.3 |
| | | M10.3.3 | 2.96 | 212.4 | 71.7 |
| | | M10.3.4 | 1.80 | 163.9 | 91.1 |
| | M10.7 | M10.7.1 | 1.92 | 196.5 | 102.1 |
| | | M10.7.3 | 1.72 | 148.2 | 86.3 |
| | | M10.7.4 | 2.23 | 112.5 | 50.5 |
| | M10.13 | M10.13.1 | 2.81 | 283.6 | 100.8 |
| | | M10.13.2 | 2.52 | 217.6 | 86.5 |
| pSCM13 | M13.2 | M13.2.1 | 1.43 | 330.1 | 231.4 |
| | | M13.2.2 | 1.03 | 8.5 | 8.2 |
| | | M13.2.3 | 2.15 | 65.9 | 30.7 |
| | | M13.2.4 | 1.15 | 1.1 | .9 |
| | M13.4 | M13.4.1 | 1.78 | 332.7 | 186.7 |
| | | M13.4.3 | 2.86 | 357.8 | 124.9 |
| | | M13.4.5 | 1.14 | 11.0 | 9.7 |
| | | M13.4.6 | 1.64 | 324.7 | 198.6 |
| | M13.7 | M 13.7.3 | 2.60 | 259.3 | 99.9 |
| | M13.10 | M13.10.1 | 1.83 | 235.1 | 128.6 |
| | | M13.10.2 | 2.89 | 153.1 | 52.9 |
| | | M13.10.3 | 2.53 | 504.9 | 199.3 |

**Tabelle 8:**

| **ANALYSE DER NACHKOMMEN TRANSGENER PFLANZEN (nur KanR Pflanzen wurden getestet)** **Konzentration in homogenisierten Blättern** | | | | | |
|---|---|---|---|---|---|
| Plasmid | Elternpflanze | Pflanze | mg/ml | nkat/ml | nkat/mg |
| pClB200 | C2 | C2.1 | 2.77 | 4.40 | 1.59 |
| | | C2.2 | 2.23 | 4.06 | 1.82 |
| | | C2.3 | 2.01 | 4.95 | 2.46 |
| PSCU11 | U11.3 | U11.3.1 | 2.52 | 7.38 | 2.93 |
| | | U11.3.2 | 2.14 | 7.69 | 3.59 |
| | U11.4 | U11.4.1 | 1.87 | 35.58 | 19.05 |
| | | U11.4.2 | 1.81 | 46.75 | 25.77 |
| | U11.5 | U11.5.1 | 2.30 | 6.95 | 3.02 |
| | | U11.5.2 | 2.79 | 13.88 | 4.98 |
| Die Chitinaseaktivität wurde in Gegenwart von Immunoglobulinen gegen die Tabakchitinase gemessen | | | | | |

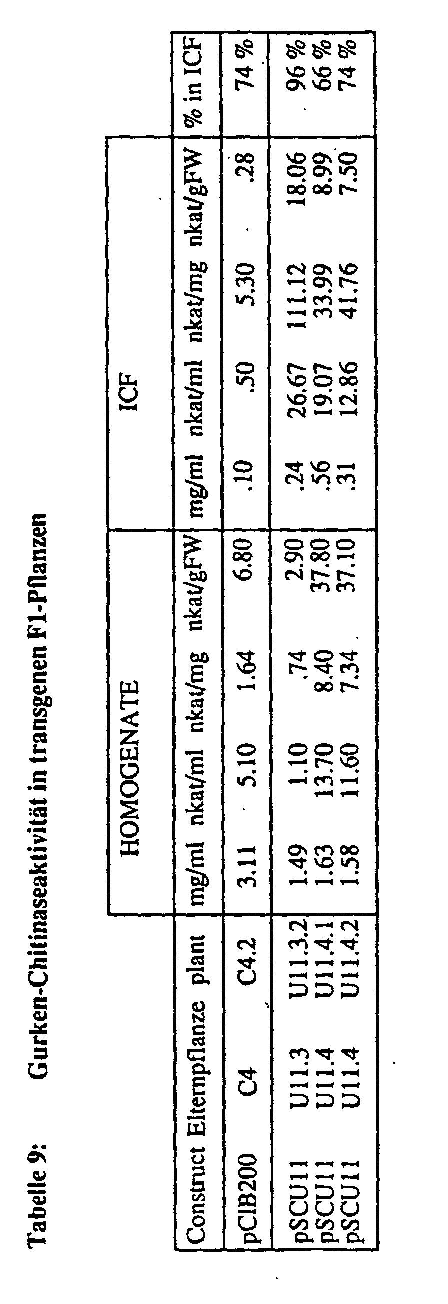

**Tabelle 10:**

| **Aktivität** ^{**35**}**S-markierter, reifer β-1,3-Glucanase in *Nicotiana plumbaginifolia* Protoplasten** | | |
|---|---|---|
| **Plasmid Plasmid** | **Protoplasten- Extrakt** | **Inkubations- Medium** |
| **----** | **mässig** | **Spur** |
| **pCIB1005B** | **stark** | **Spur** |
| **pCIB1005BΔVTP** | **mässig** | **mässig** |

### LITERATURVERZEICHNIS

**Allen G,** "Sequencing of proteins and peptides", in: Laboratory Techniques in Biochemistry and Molecular Biology, Vol 8, eds. TS Work and RH Bordon, Elsevier, North-Holland Biomedical Press, Amsterdam (1981).
**An** G *et al,* EMBO J., 4: 277-284 (1985).
**Birk** Y *et al,* Biochim. Biophys. Acta, 67: 326-328 (1963).
**Bohlmann** *et al,* EMBO J 7: 1559-1565 (1988)
**Boller** *et al,* Planta, 157: 22-31 (1983).
**Boller T und Wiemken A,** Ann Rev Plant Physiol, 37: 137-164 (1986)
**Boller T und Kende H,** Plant. Physiol. 63: 1123-1132 (1979)
**Boudet AM und Alibert G,** Meth. Enzymol. 148: 74-81 (1987)
**Cashmore A,** Genetic Engineering of Plants, an Agricultural Perspective,Plenum, New York 1983, Seite 29-38.
**Devereux** *et al*, Nucl. Acids Res., 12: 387-395 (1984).
**Erlich** *et al*, PCR Technology Principles and Applications for DNA Amplification, Stockton Press, New York (1989)
**Eichholz** R *et al*, Planta, 158: 410-415 (1983).
**Facciotti** und Pilet, Plant Science Letters, 15: 1-7 (1979).
**Felix** G und Meins FJr, Planta, 164: 423-428 (1985).
**Frank** G *et al*, Cell, 21: 285-294 (1980).
**Gardner** RC *et al,* Nucl. Acids Res., 9: 2871-2888 (1981).
**Garfinkel** und Nester, J. Bact., 144: 732-743 (1980).
**Glover** DM, DNA cloning, volume 1: a practical approach; DM Glover ed.,IRL Press, Oxford and Washington DC, p.33 (198)
**Goodall** G et *al,* Methods in Enzymology, 181: 148-161 (1990)
**Grimsley** NH *et al*, Nature, 325: 177-179 (1987).
**Gubler U und Hoffman BJ,** Gene, 25: 263 (1983).
**Haymes** BT *et al,* Nucleic Acid Hybridisation a Practical Approach, IRLPress, Oxford, England (1985).
**Hilder** *et al*, Nature, 330: 160-163 (1987).
**Hoekema** *et al*, Nature, 303: 179-180 (1983).
**Hohn** T *et al*, in: "Molecular Biology of Plant Tumors", Academic Press, New York, pp. 549-560 (1982).
**Horn** *et al,* Plant Cell Reports, 7: 469-472 (1988).
**Horsch** *et al,* Science, 227: 1229 (1985).
**Howard** *et al,* Planta, 170: 535 (1987).
**Innis** *et al,* PCR Protocols: A Guide to Methods and Applications, Academic Press, Inc., New York, 1990
**Lagrimini** LM *et al,* Proc. Natl. Acad. Sci., USA 84: 7542, (1987).
**Laurell** and McKay, Methods Enzymology, 73: 339-361 (1981).
**Lathe** R *et al,* J. Mol. Bio., 183: 1-12 (1985).
**Linsmeier** und Skoog, Physiol. Plant., 18: 101-127 (1965).
**Maniatis** *et al,* Molecular Cloning, A Laboratory Manual, Cold Spring Harbor Laboratory, 1982.
**Matsuoka K** and Nakamura K, Proc. Natl. Acad. Sci. USA, 88: 834-838 (1991)
**Maxam** and Gilbert, 'Sequencing end-labelled DNA with base-specific chemical cleavage', in: Methods in Enzymology 65: 499-560, Academic Press, New York, London, (1980).
**Meins** & Lutz, Differentiation, 15: 1-6 (1979).
**Métraux JP** *et al*, Physiol Mol Plant Pathol, 33: 1-9 (1988).
**Mohnen,** "Regulation of Glucanohydrolases in Nicotiana tabacum on the messanger RNA level", Dissertation University of Illinois at Urbana-Champaign, 1985.
**Mohnen** *et al,* EMBO J., 4: 1631-1635 (1985).
**Morelli** *et al,* Nature, 315: 200 (1985).
**Muller JF** *et al,* Physiol. Plant. 57: 35-41 (1983)
**Murashige** und Skoog, Physiol. Plant., 15: 473-497 (1962).
**Negrutiu** I *et al,* Plant Mol. Biol., 8: 363-373 (1987).
**Neuhaus** *et al,* Theor. Appl. Genet., 74: 30-36 (1987).
**Parent und Asselin,** Can J Bot, Vol 62: 564-569 (1984).
**Paszkowski** J *et al*, EMBO J, 3: 2717 (1984).
**Petit** *et al,* Mol. Gen. Genet., 202: 388 (1986).
**Pietrzak** *et al,* Nucl. Acids Res., 14: 5857-5868 (1986).
**Potrykus** I and Shillito RD, Methods in Enzymology, Vol 118, Plant Molecular Biology, eds.A and H Weissbach, Academic Press, Orlando,1986.
**Rhodes** *et al,* Biotechnology, 6: 56-60 (1988).
**Rogers SG** *et al,* Methods in Enzymology, 118: 630-633 (1986).
**Rothstein** SJ *et al*, Gene, 53: 153-161 (1987).
**Sambrook** *et al,* Molecular Cloning, A Laboratory Manual, Second Edition (1989)
**Sanger** *et al,* Proc. Natl. Acad. Sci., USA 74: 5463-5467 (1977).
**Schmidhauser** und Helinski, J. Bacteriol., 164: 446-455 (1985).
**Schocher** RJ *et al,* Bio/Technology, 4: 1093-1096 (1986).
**Selsted** et al, Infection and Immunity, 55: 2281-2286 (1987)
**Shillito** *et al,* Bio Technology, 3: 1099-1103 (1985).
**Shillito RD und Potrykus I,** In: Methods in Enzymology, eds. Wu R und Grossman L, Academic Press, Orlando, Florida, Vol. 153: 313-306 (1987).
**Shillito** RD *et al,* Biotechnology, 7: 581-587 (1989).
**Shinshi** *et al,* Proc. Natl. Acad. Sci., USA 84: 89-93 (1987).
**Shinshi** *et al,* Planta, 164: 423-428 (1985).
**Shinshi** *et al,* Proc. Natl. Acad. Sci., USA 85: 5541-5545 (1988).
**Simpson RJ und Nice,** Biochem Intl, 8: 787 (1984).
**Southern** EM**,** J. Mol. Biol. 98: 503-517 (1975).
**Spena** *et al,* EMBO J., 4: 2736 (1985).
**Tague BW und Chrispeels MV,** J Cell Biol, 105: 1971-1979 (1987).
**Terry** *et al*, J. Biol. Chem. 263: 5745-5751 (1988)
**Vierra** und Messing, Gene, 19: 259-268 (1982).
**Wang** Y-C *et al,* Plant Mol. Biol., 11: 433-439 (1988).
**Wang** *et al,* Proc. Nattl. Acad. Sci. USA, 86: 9717-9721 (1989)
**Yamada** Y *et al,* Plant Cell Rep, 5:85-88 (1986) .
**Yanisch-Perron** *et al,* Gene, 33: 103-119 (1985).

| | |
|---|---|
| WO 89/1 1291 | EP-A 0,332,104 |
| WO 86/0 4356 | EP-A 0.392,225 |
| WO 88/0 5826 | |
| WO 89/0 4371 | |
| US-P 4,810,777 | |

### SEQUENZPROTOKOLL

**SEQ ID NO: 1**
   **ART DER SEQUENZ:** Nukleotid mit entsprechendem Peptid
      **SEQUENZLÄNGE:** 39 Basenpaare
   **ART DES MOLEKÜLS:** hypothetische DNA-Sequenz erstellt aufgrund der Degeneration des genetischen Kodes.
   **EIGENSCHAFTEN:** kodiert Signalpeptid, das verantwortlich ist für die vakuoläre Lokalisation assoziierter Proteinmoleküle 
CGN/AGR TCN/AGW TTY GGN AAY GGN CTN/TTR TTR/CTN GTN
Arg Ser Phe Gly Asn Gly Leu Leu Val

GAY ACN ATG TAA
Asp Thr Met End
**SEQ ID NO: 2**
   **ART DER SEQUENZ:** Nukleotid mit entsprechendem Peptid
      **SEQUENZLÄNGE:** 39 Basenpaare
   **ART DES MOLEKÜLS:** cDNA von mRNA
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
AGG TCT TTT GGA AAT GGA CTT TTA GTC GAT ACT ATG TAA
Arg Ser Phe Gly Asn Gly Leu Leu Val Asp Thr Met End
**SEQ ID NO: 3**
   **ART DER SEQUENZ:** Nukleotid mit entsprechendem Peptid
      **SEQUENZLÄNGE:** 39 Basenpaare
   **ART DES MOLEKÜLS:** cDNA von mRNA (mutiert *via Oligonukleotid vermittelter Mutagenese)*
   **URSPRÜNGLICHE HERKUNFT**
      **ORGANISMUS:** *Nicotiana tabacum*
   **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
AGG TCT TTT GGA AAA GAT CTT TTA GTC GAT ACT ATG TAA
Arg Ser Phe Gly Lys Asp Leu Leu Val Asp Thr Met End
**SEQ ID NO: 4**
   **ART DER SEQUENZ:** Nukleotid mit entsprechendem Peptid
      **SEQUENZLÄNGE:** 39 Basenpaare
   **ART DES MOLEKÜLS:** cDNA von mRNA (mutiert via *Oligonukleotid* *vermittelter Mutagenese)*
   **URSPRÜNGLICHE HERKUNFT**
      **ORGANISMUS: *Nicotiana tabacum***
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
AGG TCT TTT GGA AAT GGA CTT TTA GTC AAT ACT ATG TAA
Arg Ser Phe Gly Asn Gly Leu Leu Val Asn Thr Met End
**SEQ ID NO: 5**
   **ART DER SEQUENZ:** Nukleotid mit entsprechendem Peptid
      **SEQUENZLÄNGE:** 39 Basenpaare
   **ART DES MOLEKÜLS:** cDNA von mRNA (mutiert *via* Oligonukleotid-vermittelter Mutagenese)
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
AGG TCT TTT GGA AAT GGA CTT TTA GTC CGT ACT ATG TAA
Arg Ser Phe Gly Asn Gly Leu Leu Val Arg Thr Met End
**SEQ ID NO: 6**
   **ART DER SEQUENZ:** Nukleotid mit entsprechendem Peptid
      **SEQUENZLÄNGE:** 40 Basenpaare
   **ART DES MOLEKÜLS:** cDNA von mRNA (mutiert *via* Oligonukleotid-vermittelter Mutagenese)
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
A/T GAT CTT TTG GGA AAT GGA CTT TTA GTC GAT ACT ATG TAA
Asp Leu Leu Gly Asn Gly Leu Leu Val Asp Thr Met End
**SEQ ID NO: 7**
   **ART DER SEQUENZ:** Nukleotid mit entsprechendem Peptid
      **SEQUENZLÄNGE:** 30 Basenpaare
   **ART DES MOLEKÜLS:** cDNA von RNA (mutiert *via* Oligonukleotid-vermittelter Mutagenese)
   **URSPRÜNGLICHE HERKUNFT**
      **ORGANISMUS:** *Nicotiana tabacum*/Gurke
      **UNMITTELBARE EXPERIMENTELLE**
      **HERKUNFT**
      **NAME DER ZELLKLONE:** pCHN48/pBSCucCht5
   **EIGENSCHAFTEN:** kodiert Signalpeptid, das verantwortlich ist für die vakuoläre Lokalisation assoziierter Proteinmoleküle 
ATC GGT GAT CTT TTA GTC GAT ACT ATG TAA
Ile Gly Asp Leu Leu Val Asp Thr Met End
**SEQ ID NO: 8**
   **ART DER SEQUENZ:** Nukleotid mit entsprechendem Peptid
      **SEQUENZLÄNGE:** 21 Basenpaare
   **ART DES MOLEKÜLS:** cDNA von mRNA [Fragment der in SEQ ID 2 wiedergegebenen Sequenz]
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
CTT TTA GTC GAT ACT ATG TAA
Leu Leu Val Asp Thr Met End
**SEQ ID NO: 9**
   **ART DER SEQUENZ:** Nukleotid mit entsprechendem Peptid
      **SEQUENZLÄNGE:** 24 Basenpaare
   **ART DES MOLEKÜLS:** cDNA von mRNA [Fragment der in SEQ ID 2 wiedergegebenen Sequenz]
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pCHN48
   **EIGENSCHAFTEN:** kodiert Signalpeptid, das verantwortlich ist für die vakuoläre Lokalisation assoziierter Proteinmoleküle 
GGA CTT TTA GTC GAT ACT ATG TAA
Gly Leu Leu Val Asp Thr Met End
**SEQ ID NO: 10**
   **ART DER SEQUENZ:** Nukleotid mit entsprechendem Protein
      **SEQUENZLÄNGE:** 3850 Basenpaare
   **STRANGFORM:** Doppelstrang
      **TOPOLOGIE:** linear
      **ART DES MOLEKÜLS:** Genom-DNA
   **URSPRÜNGLICHE HERKUNFT**
      **ORGANISMUS:** *Nicotiana tabacum* L. cv. Havana 425
      **UNMITTELBARE EXPERIMENTELLE HERKUNFT**
      **NAME DES ZELLKLONS:** λCHN17 [λ Phage]
   **EIGENSCHAFTEN:** basiches Chitinasegen GAATTCAATC AAAATGTGTT TTGTATATAG GGTGTCAACT ACTAATATAT 50 
TGTTATTTTC TAAAGACATA CATGTATACA TGTAAAATTT ACCGAACTTT 100
ACGGATGTCG ATAACCCCTC TCGATATAGC ATAGGTCCGC CTCTGATTTA 150
CGAAGGGACA CGAGGAAATT CCTCTATGTA ATTAGTTTTA GCAGTTACAC 200
GTTAAAGTAT AAATACATAT TACTTTACCA TAGTTAAGAC CAAACATGTG 250
TATGATTGAC ATACATCTTG CATTCATTAA TTAATTTGAT TTGATGCGAT 300
TAAATTTTTT AAGGATAGAG TTTTTAGTCC AAGTTGAGCT AGTGTAACTC 350
TTATAGTCAA TTGGACTCTC TATTACTAGA TACTATATCA GTTCAAAAGA 400
CACCAATATT GTATTTTAAC AGAAGGAGGC AAATAAGAAA TTGCAAATTC 450
TCAATTCTTT TTAATTATAT CTAATGAACC AAAAGGAGGA AGAGGAGCTA 500
CATATTGGAT TTATAAATAT AAAGCTAGCT GAGGCTCAAA TAATTGTGGA 550
TGCAATACAA GCAATTTACT TAAAACACGA AACAGAAGAG GATTTCGGTC 600
AAATATCGAC ACCTAAGTTT TGAAATACAT TACTGAACAA ATTATGAGAT 650
CATAGACTAG TAATTTAGGA TATTATTCTG TGATTGACTT GATTTTGCAC 700
ATGAAGAAAC GTGAACGGCT TTCTTTTTAG GGCTGCCGTA GAATTGATCA 750
AAACATATCT CAACATATTA AAATAGGGTC TCAACTAAAC CGGATTCATG 800
CGGAATGAGA CCCATTTAAA AGGAGCAGTG GTTCCTATTC AAAGAATTAG 850
ATACATTTCT ACATATTTTT AATTATGAAA ATTACTCCTA TACTAATTTG 900
TGTGGTTTTA ATCGAATATG TAAATTTTAT TTGAAAATAA AATAAAAAAT 950
CACAGTCCAA CTTTAATCAT AACACTCAAA TTAAATTCAG CTATCTTTCT 1000
AGGACATAGG AAACATTATC AGTGGAAATA TTATATTATA TCCATAAGAC 1050
TTTAGCAAAT CCTATAAGAA GTCTAAACAT GTAATTGACT ACTTTTAGAA 1100
GACGCACTTA TCTAACCCAA GAAACACCTG GCGTAACTCG AATTTGCTTT 1150
TGCCAAAACC AAAAGTCTAG GAATTAAGCT CCAAATTAAA GACATAGATT 1200
TTGGCTTACT TTTTTTCAAA AAAAAAATAA AATTAAAAAT TAAAATATTT 1250
TTTGTTCATG TAATTTAATC AGTTTTTGGG TGAAATTTTT TCTTCCACAC 1300
ACAAGATTTT AACTTTTTTC CAAATAAAAT ACACGTCGAA ACATAAATTC 1350
AAATTTCAGA ACTATTTTTC AACGTAATTT TAAAATTTTT ATTTTCTAGT 1400
TTTAACTAAA TCTATGTCCT GATTAAGTCT CCAGTCTTAA CTCTTAAAGT 1450
ATTGAAAATA CATGTTCGAG AATTGTCTGG GATGAAGCTA AGAGCCGCCA 1500
CTAAGAAAAA AAATCTAAAA ATATATAAAA AGGTAAGAGC CGCCACATAA 1550
TATATGTAAC CTGTCGGCGT AATCTACTGA ATTAATTTTC TGGATAAGAA 1600
AGATATGACT GAGCTCCGGT TTGCTCATAG ATTTTGACTT TACTTTTTTA 1650
ATTTCTTTTT GAAAATATTG TTTGTTTAAT AAAATATGAT CATGTTTTAG 1700
AAAAACAAAT TTCAAAAAAC TTCAAGTTCC CAAAAGTTGT ATGTCCAAAC 1750
ACAACTTTCA AAAATTATTT TTTAAAACAA CTTTAAAAAC TTTTTTTTTA 1800
AATTTTAATT AAATCTATGT CCAAACTAGC CGAAATTCGA GCCTTGGTTA 1850
 
TTCAACCAAT TGATTTGGTC AGAAAGTCAG TCCTCTCAAC AACTAAAATA 1900
GACATTAATT AAGCCATGTC TCCAGCATCT TCCTTAGCAA TAAATACCTT 1950
GCATTTCACC AGTTTACTAC TACATTAAA ATG AGG CTT TGT AAA TTC 1997
Met Arg Leu Cys Lys Phe

ACA GCT CTC TCT TCT CTA CTA TTT TCT CTC CTA CTG CTT TCT 2039
Thr Ala Leu Ser Ser Leu Leu Phe Ser Leu Leu Leu Leu Ser

GCC TCG GCA GAA CAA TGT GGT TCC CAG GCC GGA GGT GCG CGT 2081
Ala Ser Ala Glu Gln Cys Gly Ser Gln Ala Gly Gly Ala Arg

TGT CCC TCG GGT CTC TGC TGC AGC AAA TTT GGT TGG TGT GGT 2123
Cys Pro Ser Gly Leu Cys Cys Ser Lys Phe Gly Trp Cys Gly

AAC ACC AAT GAC TAC TGT GGC CCT GGC AAT TGC CAG AGC CAG 2165
Asn Thr Asn Asp Tyr Cys Gly Pro Gly Asn Cys Gln Ser Gln

TGC CCT GGT GGT CCC ACA CCT ACA CCC CCC ACC CCA CCC GGT 2207
Cys Pro Gly Gly Pro Thr Pro Thr Pro Pro Thr Pro Pro Gly

GGT GGG GAC CTC GGC AGT ATC ATC TCA AGT TCC ATG TTT GAT 2249
Gly Gly Asp Leu Gly Ser Ile Ile Ser Ser Ser Met Phe Asp

CAG ATG CTT AAG CAT CGC AAC GAT AAT GCA TGC CAA GGA AAG 2291
Gln Met Leu Lys His Arg Asn Asp Asn Ala Cys Gln Gly Lys

GGA TTC TAC AGT TAC AAT GCC TTT ATC AAT GCT GCT CGG TCT 2333
Gly Phe Tyr Ser Tyr Asn Ala Phe Ile Asn Ala Ala Arg Ser

TTT CCT GGC TTT GGT ACC AGT GGC GAT ACC ACT GCC CGT AAA 2375
Phe Pro Gly Phe Gly Thr Ser Gly Asp Thr Thr Ala Arg Lys

AGA GAA ATC GCG GCT TTC TTT GCT CAA ACC TCC CAT GAA ACT 2417
Arg Glu Ile Ala Ala Phe Phe Ala Gln Thr Ser His Glu Thr

ACT GGT AAGTCTAGTT ACGTTGAACT ATATGATCGT CTTATTCAAA 2463
 
Thr Gly

AGTTTAATCA ATTAGAGAGA TCATACTTTT ATTTAATCAT ACTGGTCTAT 2513
TCTGATTTCA TGAGACAAAC ACATAGAACT TCCTTTTAAA ATGATTGCGC 2563
TGAGACTTGA ATTCAGGACC TCTATCTGCT CATCACTGGA GTATCCAATT 2613
TTGAGATATC ACAATGCTTC TTAAATTTCG AAGTTTTTTA TAAGCTGACG 2663
CGTTCAATAA TTGACCATGT AACCGTTGAC AGGA GGA TGG GCA ACA 2709
Gly Trp Ala Thr

GCA CCA GAT GGT CCA TAT GCA TGG GGT TAT TGC TGG CTT AGA 2751
Ala Pro Asp Gly Pro Tyr Ala Trp Gly Tyr Cys Trp Leu Arg

GAA CAA GGT AGC CCC GGC GAC TAC TGT ACC CCA AGT GGT CAG 2793
Glu Gln Gly Ser Pro Gly Asp Tyr Cys Thr Pro Ser Gly Gln

TGG CCT TGT GCT CCT GGT CGA AAA TAT TTC GGA CGA GGC CCC 2835
Trp Pro Cys Ala Pro Gly Arg Lys Tyr Phe Gly Arg Gly Pro

ATC CAA ATT TCA CAG TAAGTTCCTT CTTACCCACA CGGAGTGTTT 2880
Ile Gln Ile Ser His

ACACCAAAGT CGTGGGACGG AATGCTTACT ACCTACTATA TATTTCATTG 2930
TGAGAGTAGG TACACAATAT CATGATATTT CTATGATTAT AAGAGTATGT 2980
GATTAATTTC TATGAGAAGT GTAAAGTTAA ATAGTTTCCA CAACACAAAA 3030
AAAATGTCAT TTTTTTAACA GATTAAAAAA GAAAAAGTAT ATGATGAACT 3080
TGTAGGATCT AATTAAGTGT ATTTTGACAT AAATACAGC AAC TAT AAC 3128
Asn Tyr Asn

TAC GGG CCT TGT GGA AGA GCC ATA GGA GTG GAC CTG CTA AAC 3170
Tyr Gly Pro Cys Gly Arg Ala Ile Gly Val Asp Leu Leu Asn

AAT CCT GAT TTA GTG GCC ACA GAT CCA GTC ATC TCA TTT AAG 3212
Asn Pro Asp Leu Val Ala Thr Asp Pro Val Ile Ser Phe Lys

TCA GCT CTC TGG TTC TGG ATG ACT CCT CAA TCA CCA AAA CCT 3254
Ser Ala Leu Trp Phe Trp Met Thr Pro Gln Ser Pro Lys Pro
 
TCT TGC CAC GAT GTC ATC ATC GGA AGA TGG CAG CCA TCA GCT 3296
Ser Cys His Asp Val Ile Ile Gly Arg Trp Gln Pro Ser Ala

GGT GAT CGC GCA GCC AAT CGC CTC CCT GGA TTT GGC GTC ATC 3338
Gly Asp Arg Ala Ala Asn Arg Leu Pro Gly Phe Gly Val Ile

ACA AAC ATC ATC AAT GGT GGC TTG GAA TGT GGT CGT GGC ACT 3380
Thr Asn Ile Ile Asn Gly Gly Leu Glu Cys Gly Arg Gly Thr

GAC TCA AGG GTC CAG GAT CGC ATT GGG TTT TAC AGG AGG TAT 3422
Asp Ser Arg Val Gln Asp Arg Ile Gly Phe Tyr Arg Arg Tyr

TGC AGT ATT CTT GGA GTT AGT CCT GGT GAC AAT CTG GAT TGC 3464
Cys Ser Ile Leu Gly Val Ser Pro Gly Asp Asn Leu Asp Cys

GGC AAC CAG AGG TCT TTT GGA AAT GGA CTT TTA GTC GAT ACT 3506
Gly Asn Gln Arg Ser Phe Gly Asn Gly Leu Leu Val Asp Thr

ATG TAATTTCATG ATCTGTTTTG TTGTATTCCC TTGCAATGCA 3549
Met

GGGCCTAGGG CTATGAATAA AGTTAATGTG TGAATGTGAA TGTGTGATTG 3599
TGACCTGAAG GGATCACGAC TATAATCGTT TATAATAAAC AAAGACTTTG 3649
TCCCAATATA TGTGTTAATG AGCATTACTG TAGTTGGTTT AATTCGGCAC 3699
CAGATAAATA GATAACCACC CGCACTATTA TATTTCATTA TTTAGAAAAC 3749
CGAGATCTTT ATTTGAGTGA ATGAAAATCT TCCTAACCAG ATAGTCATAC 3799
TAATCAGTCA AAAAAAAATC TAACCTCAAA ATTTAAGCAT CCGAGCTGCAG 3850
**SEQ ID NO: 11**
   **ART DER SEQUENZ:** Nukleotid
      **SEQUENZLÄNGE:** 1103 Basenpaare
   **STRANGFORM:** Doppelstrang
      **TOPOLOGIE:** linear
      **ART DES MOLEKÜLS:** cDNA
   **URSPRÜNGLICHE HERKUNFT**
      **ORGANISMUS:** TNV infizierte Gurkenblätter
      **UNMITTELBARE EXPERIMENTELLE HERKUNFT**
      **NAMEDESZELLKLONS:** pBSCucCht5 [ATCC 40528]
   **EIGENSCHAFTEN:** saures Chitinasegen 
AAAGAAAGCT CTTTAAGCAA TGGCTGCCCA CAAAATAACT ACAACCCTTT 50
CCATCTTCTT CCTCCTTTCC TCTATTTTCC GCTCTTCCGA CGCGGCTGGA 100
ATCGCCATCT ATTGGGGTCA AAACGGCAAC GAGGGCTCTC TTGCATCCAC 150
CTGCGCAACT GGAAACTACG AGTTCGTCAA CATAGCATTT CTCTCATCCT 200
TTGGCAGCGG TCAAGCTCCA GTTCTCAACC TTGCTGGTCA CTGCAACCCT 250
GACAACAACG GTTGCGCTTT TTTGAGCGAC GAAATAAACT CTTGCAAAAG 300
TCAAAATGTC AAGGTCCTCC TCTCTATCGG TGGTGGCGCG GGGAGTTATT 350
CACTCTCCTC CGCCGACGAT GCGAAACAAG TCGCAAACTT CATTTGGAAC 400
AGCTACCTTG GCGGGCAGTC GGATTCCAGG CCACTTGGCG CTGCGGTTTT 450
GGATGGCGTT GATTTCGATA TCGAGTCTGG CTCGGGCCAG TTCTGGGACG 500
TACTAGCTCA GGAGCTAAAG AATTTTGGAC AAGTCATTTT ATCTGCCGCG 550
CCGCAGTGTC CAATACCAGA CGCTCACCTA GACGCCGCGA TCAAAACTGG 600
ACTGTTCGAT TCCGTTTGGG TTCAATTCTA CAACAACCCG CCATGCATGT 650
TTGCAGATAA CGCGGACAAT CTCCTGAGTT CATGGAATCA GTGGACGGCG 700
TTTCCGACAT CGAAGCTTTA CATGGGATTG CCAGCGGCAC GGGAGGCAGC 750
GCCGAGCGGG GGATTTATTC CGGCGGATGT GCTTATTTCT CAAGTTCTTC 800
CAACCATTAA AGCTTCTTCC AACTATGGAG GAGTGATGTT ATGGAGTAAG 850
 
GCGTTTGACA ATGGCTACAG CGATTCCATT AAAGGCAGCA TCGGCTGAAG 900
GAAGCTCCTA AGTTTAATTT TAATTAAAGC TATGAATAAA CTCCAAAGTA 950
TTATAATAAT TAAAAAGTGA GACTTCATCT TCTCCATTTA GTCTCATATT 1000
AAATTAGTGT GATGCAATAA TTAATATCCT TTTTTTCATT ACTATACTAC 1050
CAATGTTTTA GAATTGAAAA GTTGATGTCA ATAAAAACAT TCCAAGTTTA 1100
TTT 1103
**SEQ ID NO: 12**
   **ART DER SEQUENZ:** Nukleotid mit entsprechendem Peptid
      **SEQUENZLÄNGE:** 69 Basenpaare
   **ART DES MOLEKÜLS:** cDNA von mRNA
   **URSPRÜNGLICHE HERKUNFT**
      **ORGANISMUS:** *Nicotiana tabacum*
      **UNMITTELBARE EXPERIMENTELLE**
      **HERKUNFT**
      **NAME DES KLONS:** pBS-Gluc39.1 [ATCC 40526; beschrieben in EP-A 0,332,104]
   **EIGENSCHAFTEN:** kodiert Signalpeptid, das verantwortlich ist für die vakuoläre Lokalisation assoziierter Proteinmoleküle

GTC TCT GGT GGA GTT TGG GAC AGT TCA GTT GAA ACT AAT GCT ACT
Val Ser Gly Gly Val Trp Asp Ser Ser Val Glu Thr Asn Ala Thr

GCT TCT CTC GTA AGT GAG ATG TGA
Ala Ser Leu Val Ser Glu Met End

## Patentansprüche

1. DNA-Sequenz, die ein Peptid kodiert, **dadurch gekennzeichnet, dass** besagte Sequenz aus der 3'-terminalen Region eines Gens stammt, das für ein natürlicherweise vakuolär vorliegendes Proteinmolekül kodiert und dass besagte DNA Sequenz ein C-terminales Peptid [C-terminale Extension] kodiert, das für die gezielte Einschleusung ['Targeting'] eines assoziierten Genproduktes heterologen Ursprungs in die pflanzliche Vakuole verantwortlich ist, wobei besagte DNA-Sequenz aus der 3'-terminalen Region eines pflanzlichen Chitinasegens stammt.

2. DNA-Sequenz gemäss Anspruch 1, **dadurch gekennzeichnet, dass** besagte Sequenz folgende, in SEQ ID NO: 1 wiedergegebene, allgemeine Nukleotid-Sequenz aufweist: 
CGN/AGR TCN/AGW TTY GGN AAY GGN CTN/TTR TTR/CTN GTN GAY ACN ATG
TAA
 worin
N A oder G oder C oder T/U;
R G oder A;
W A oder T/U; und
Y T/U oder C
bedeuten.

3. DNA-Sequenz gemäss Anspruch 1, **dadurch gekennzeichnet, dass** besagte Sequenz aus der 3'-terminalen Region eines basischen Chitinasegens von *Nicotiana tabacum L.* c. v. Havana 425 Pflanzen stammt und folgende, in SEQ ID NO: 2 wiedergegebene Nukleotid-Sequenz aufweist:

3'- AGG TCT TTT GGA AAT GGA CTT TTA GTC GAT ACT ATG TAA - 5'

4. DNA-Sequenz, die den in einem der Ansprüche 1 bis 3 wiedergegebenen DNA Sequenzen im wesentlichen homolog ist und noch die erfindungswesentlichen Eigenschaften dieser Sequenzen aufweist, d.h. ein C-terminales Peptid kodiert, das als 'Targeting'-Signal für die pflanzliche Vakuole fungiert, **dadurch gekennzeichnet, dass** besagte Sequenz eine der in SEQ ID NO: 3 bis 7 wiedergegebenen Nukleotidsequenzen aufweist:
(a) AGG TCT TTT GGA AAA GAT CTT TTA GTC GAT ACT ATG TAA
(b) AGG TCT TTT GGA AAT GGA CTT TTA GTC AAT ACT ATG TAA
(c) AGG TCT TTT GGA AAT GGA CTT TTA GTC CGT ACT ATG TAA
(d) A/T GAT CTT TTG GGA AAT GGA CTT TTA GTC GAT ACT ATG TAA
(e)

ATC GGT GAT CTT TTA GTC GAT ACT ATG TAA

5. DNA-Sequenz gemäss Anspruch 4, **dadurch gekennzeichnet, dass** es sich um eine natürlicherweise vorkommende DNA-Sequenz handelt.

6. DNA-Sequenz gemäss Anspruch 4, **dadurch gekennzeichnet, dass** es sich um eine mit Hilfe bekannter Mutationsverfahren hergestellte Sequenz handelt.

7. DNA-Sequenz gemässe Anspruch 6, **dadurch gekennzeichnet, dass** es sich bei besagtem Mutationsverfahren um eine Oligonukleotid-vermittelte Mutagenese handelt.

8. DNA-Sequenz gemäss Anspruch 1, wobei die DNA-Sequenz eine Teilsequenz der in SEQ ID NO:2 wiedergegebenen DNA-Sequenz darstellt und ein Peptid kodiert, das für die gezielte Einschleusung (Targeting) eines assoziierten Genprodukts heterologen Ursprungs in die pflanzliche Vakuole verantwortlich ist.

9. DNA-Sequenz gemäss Anspruch 8, die eine der in SEQ ID NO: 8 und 9 wiedergegebenen Nukleotid-Sequenzen aufweist:

CTT TTA GTC GAT ACT ATG TAA

GGA CTT TTA GTC GAT ACT ATG TAA

10. Peptid, **dadurch gekennzeichnet dass** es aus der C-terminalen Region eines Gens stammt, das für ein natürlicherweise vakuolär vorliegendes Proteinmolekül kodiert und das für die gezielte Einschleusung ['Targeting'] des assoziierten Proteinmoleküls in die pflanzliche Vakuole verantwortlich ist, wobei das besagte Peptid aus der C-terminalen Extension einer pflanzlichen Chitinase stammt und von einer DNA-Sequenz gemäss einem der Ansprüche 1 bis 9 kodiert wird.

11. Peptid gemäss Anspruch 10, das als 'Targeting'-Signal für die pflanzliche Vakuole fungiert und das die folgende, in SEQ ID NO: 1 und 2 wiedergegebene Aminosäuresequenz aufweist:
Arg Ser Phe Gly Asn Gly Leu Leu Val Asp Thr Met

12. Peptid gemäss Anspruch 10, das in Assoziation mit einem Proteinmolekül als 'Targeting'-Signal für die pflanzliche Vakuole fungiert und das eine der in SEQ ID NO: 3 bis 7 wiedergegeben Aminosäuresequenzen aufweist:
(a) Arg Ser Phe Gly Lys Asp Leu Leu Val Asp Thr Met End
(b) Arg Ser Phe Gly Asn Gly Leu Leu Val Asn Thr Met End
(c) Arg Ser Phe Gly Asn Gly Leu Leu Val Arg Thr Met End
(d) Asp Leu Leu Gly Asn Gly Leu Leu Val Asp Thr Met End
(e)

Ile Gly Asp Leu Leu Val Asp Thr Met End

13. Peptid gemäss Anspruch 10, das durch eine der DNA-Sequenzen gemäss Anspruch 9 kodiert wird und eine der in SEQ ID NO: 8 und 9 wiedergegebenen Aminosäuresequenzen aufweist:

Leu Leu Val Asp Thr Met End

Gly Leu Leu Val Asp Thr Met End

14. Rekombinantes DNA Molekül, **dadurch gekennzeichnet, dass** es eine chimäre genetische Konstruktion enthält, worin eine DNA-Sequenz, die aus der 3'-terminalen Region eines Gens erhältlich ist, das für ein natürlicherweise vakuolär vorliegendes Proteinmolekül kodiert und worin besagte DNA Sequenz ein C-terminales Peptid [C-terminale Extension] kodiert, das für die gezielte Einschleusung ['Targeting'] eines assoziierten Genproduktes heterologen Ursprungs in die pflanzliche Vakuole verantwortlich ist, wobei besagte DNA-Sequenz nicht die ca. 20 C-terminalen Aminosäuren des primären Translationsprodukts von Wild-Typ Osmotin kodiert, mit einem heterologen Gen operabel verknüpft ist.

15. Rekombinantes DNA Molekül gemäss Anspruch 14, **dadurch gekennzeichnet, dass** es eine chimäre genetische Konstruktion enthält, worin eine DNA-Sequenz gemäss einem der Ansprüche 1 bis 9 mit einem heterotogen Gen operabel verknüpft ist.

16. Rekombinantes DNA Molekül gemäss Anspruch 14 oder 15, **dadurch gekennzeichnet, dass** die chimäre genetische Konstruktion mit in pflanzlichen Zellen aktiven Expressionssignalen und gegebenenfalls weiteren kodierenden und/oder nicht-kodierenden Sequenzen der 3'- und/oder 5'-Region operabel verknüpft ist, sodass bei Transformation in einen pflanzlichen Wirt eine gezielte Einschleusung des Expressionsproduktes in die pflanzliche Vakuole erfolgt.

17. Rekombinantes DNA Molekül gemäss Anspruch 16, **dadurch gekennzeichnet, dass** es sich um Expressionssignale handelt, die aus Genen von Pflanzen oder Pflanzenviren stammen oder aber um bakterielle Expressionssignale handelt.

18. Rekombinantes DNA Molekül gemäss Anspruch 17, **dadurch gekennzeichnet, dass** es sich um Promotor- und/oder Terminationssignale von Cauliflower Mosaik Virus Genen (CaMV) handelt.

19. Rekombinantes DNA Molekül gemäss Anspruch 17, **dadurch gekennzeichnet, dass** es sich um die Expressionssignale der Nopalin-Synthase-Gene (nos) und/oder der Octopin-Synthase-Gene (ocs) aus den Ti-Plasmiden von *Agrobacterium tumefaciens* handelt.

20. Rekombinantes DNA Molekül gemäss Anspruch 16, **dadurch gekennzeichnet, dass** besagtes, rekombinantes DNA-Molekül zusätzliche, nicht-kodierende, regulatorische DNA-Sequenzen der 3'- und/oder 5'-Region enthält, die in der Lage sind die Transkription einer assoziierten DNA-Sequenz in pflanzlichen Geweben im Sinne einer Induktion oder Repression zu regulieren.

21. Rekombinantes DNA Molekül gemäss Anspruch 16, **dadurch gekennzeichnet, dass** besagtes heterologes Gen in seiner 5'-terminalen Region eine Sequenz enthält oder aber mit einer solchen Sequenz operabel verknüpft ist, die für ein N-terminales Signalpeptid kodiert.

22. Rekombinantes DNA Molekül gemäss einem der Ansprüche 14 bis 16, **dadurch gekennzeichnet, dass** es sich bei besagtem heterologen Gen um ein Strukturgen handelt, das in der Lage ist, den transformierten pflanzlichen Zellen sowie den sich daraus entwickelnden Geweben und insbesondere aber den Pflanzen selbst einen Schutzeffekt gegenüber Pathogenen, Chemikalien sowie nachteiligen Umwelteinflüssen zu verleihen.

23. Rekombinantes DNA Molekül gemäss Anspruch 22, **dadurch gekennzeichnet, dass** es sich bei besagtem heterologen Gen um ein Gen handelt, das Chitinase in pflanzlichen Zellen exprimiert.

24. Rekombinantes DNA Molekül gemäss Anspruch 22, **dadurch gekennzeichnet, dass** es sich bei besagtem heterologen Gen um ein Gen handelt, das Glucanase in pflanzlichen Zellen exprimiert.

25. Rekombinantes DNA Molekül gemäss einem der Ansprüche 14 bis 16, **dadurch gekennzeichnet, dass** besagte chimäre genetische Konstruktion bei Expression in der transformierten pflanzlichen Zelle als solcher oder als Bestandteil einer höher organisierten Einheit ausgewählt aus der Gruppe bestehend aus einem Gewebe, Organ, Kallus, Embryo oder einer ganzen Pflanze, zu einem Expressionsprodukt führt das in die pflanzliche Vakuole eingeschleust wird.

26. Rekombinantes DNA Molekül gemäss einem der Ansprüche 14 bis 16, **dadurch gekennzeichnet, dass** es zusätzlich eine DNA Sequenz enthält, die für einen selektierbaren phaenotypischen Marker kodiert.

27. Rekombinantes DNA Molekül gemäss einem der Ansprüche 14 bis 16, **dadurch gekennzeichnet, dass** es zusätzlich einen Replikationsursprung enthält, der eine Replikation in einem oder mehreren Mikroorganismen zulässt.

28. Klonierungsvektor enthaltend ein rekombinantes DNA Molekül gemäss einem der Ansprüche 14 bis 27.

29. Transformationsvoktor und/oder Expressionsvektor enthaltend ein rekombinantes DNA Molekül gemäss einem der Ansprüche 14 bis 27.

30. Shuttle Vektor enthaltend ein rekombinantes DNA Molekül gemäss Anspruch 27, der in der Lage ist sowohl in *E. coli* als auch in A. *tumefaciens* stabil zu replizieren.

31. Nicht-menschlicher Wirtsorganismus enthaltend ein rekombinantes DNA Molekül gemäss einem der Ansprüche 14 bis 27 oder einen Vektor gemäss einem der Ansprüche 28 bis 30.

32. Wirtsorganismus gemäss Anspruch 31, **dadurch gekennzeichnet, dass** es sich bei besagtem Wirtsorganismus um ein Bakterium oder aber um eine Pflanze handelt.

33. Pflanzliches Material, ausgewählt aus der Gruppe bestehend aus Protoplasten, Zellen, Kalli, Gewebe, Organe, Samen, Embryonen, Pollen, Eizellen, Zygoten, das ein rekombinantes DNA Molekül gemäss einem der Ansprüche 14 bis 27 oder einen Vektor gemäss einem der Ansprüche 28 bis 30 enthält.

34. Transgene Pflanze einschliesslich ihrer sexuellen und asexuellen Nachkommenschaft enthaltend ein rekombinantes DNA Molekül gemäss einem der Ansprüche 14 bis 27 und/oder einen Vektor gemäss einem der Ansprüche 28 bis 30.

35. Transgene Pflanze regeneriert aus pflanzlichem Material gemäss Anspruch 33, das ein rekombinantes DNA Molekül gemäss einem der Ansprüche 14 bis 27 oder einen Vektor gemäss einem der Ansprüche 28 bis 30 enthält.

36. Transgene Pflanze gemäss einem der Ansprüche 34 bis 35, **dadurch gekennzeichnet, dass** es sich um eine fertile Pflanze handelt.

37. Vermehrungsgut einer transgenen Pflanze gemäss einem der Ansprüche 34 bis 36 **dadurch gekennzeichnet, dass** das Vermehrungsgut ein rekombinantes DNA Molekül gemäss einem der Ansprüche 14 bis 27 und/oder einen Vektor gemäss einem der Ansprüche 28 bis 30 enthält.

38. Teile einer transgenen Pflanze gemäss einem der Ansprüche 34 bis 36 **dadurch gekennzeichnet, dass** diese Teile Blüten, Stengel, Früchte, Blätter oder Wurzeln sind.

39. Verfahren zur Herstellung eines rekombinanten DNA Moleküls zur zielgerichteten Einschleusung von Expressionsprodukten in die pflanzliche Vakuole, das im wesentlichen **dadurch gekennzeichnet ist, dass** man
(a) zunächst die 3' Region eines Gens, das für ein natürlicherweise vakuolär vorliegendes Proteinmolekül kodiert, isoliert oder diese mit Hilfe bekannter Verfahren synthetisiert;
(b) besagte DNA-Sequenz in das 3'-terminale Ende eines beliebigen Strukturgens, das heterolog ist im Bezug auf die in (a) definierte assoziierte 'Targeting'-Sequenz, operabel inseriert.

40. Verfahren gemäss Anspruch 39, **dadurch gekennzeichnet, dass** besagtes Strukturgen in seiner 5'-terminalen Region eine Sequenz enthält oder aber mit einer solchen Sequenz operabel verknüpft wird, die für ein N-terminales Signalpeptid kodiert.

## Claims

1. A DNA sequence that codes for a peptide, wherein the said sequence originates from the 3'-terminal region of a gene coding for a protein molecule present naturally in the vacuole, and the said DNA sequence codes for a C-terminal peptide [C-terminal extension] that is responsible for directing [targeting] an associated gene product of heterologous origin specifically into the plant vacuole, the said DNA sequence originating from the 3'-terminal region of a plant chitinase gene.

2. A DNA sequence according to claim 1 that has the following general nucleotide sequence, which is shown in SEQ ID NO: 1: 
CGN/AGR TCN/AGW TTY GGN AAY GGN CTN/TTR TTR/CTN GTN GAY
ACN ATG TAA
 wherein
N is A or G or C or T/U;
R is G or A;
W is A or T/U; and
Y is T/U or C.

3. A DNA sequence according to claim 1 that originates from the 3'-terminal region of a basic chitinase gene of *Nicotiana tabacum* L. c.v. Havana 425 plants and has the following nucleotide sequence, which is shown in SEQ ID NO: 2:

3' - AGG TCT TTT GGA AAT GGA CTT TTA GTC GAT ACT ATG TAA - 5'

4. A DNA sequence that is substantially homologous to the DNA sequences shown in any one of claims 1 to 3 and that still has the properties of those sequences that are essential to the invention, that is to say that codes for a C-terminal peptide that acts as the targeting signal for the plant vacuole, wherein the said sequence has one of the nucleotide sequences shown in SEQ ID NO: 3 to 7:
(a) AGG TCT TTT GGA AAA GAT CTT TTA GTC GAT ACT ATG TAA
(b) AGG TCT TTT GGA AAT GGA CTT TTA GTC AAT ACT ATG TAA
(c) AGG TCT TTT GGA AAT GGA CTT TTA GTC CGT ACT ATG TAA
(d) A/T GAT CTT TTG GGA AAT GGA CTT TTA GTC GAT ACT ATG TAA
(e)

ATC GGT GAT CTT TTA GTC GAT ACT ATG TAA

5. A DNA sequence according to claim 4 that is a naturally occurring DNA sequence.

6. A DNA sequence according to claim 4 produced by means of known mutation methods.

7. A DNA sequence according to claim 6, wherein the said mutation method is oligonucleotide-mediated mutagenesis.

8. A DNA sequence according to claim 1 that is a partial sequence of the DNA sequence shown in SEQ ID NO: 2 and that codes for a peptide that is responsible for directing [targeting] an associated gene product of heterologous origin specifically into the plant vacuole.

9. A DNA sequence according to claim 8 that has one of the nucleotide sequences shown in SEO ID NO: 8 and 9:

CTT TTA GTC GAT ACT ATG TAA

GGA CTT TTA GTC GAT ACT ATG TAA

10. A peptide originating from the C-terminal region of a gene that codes for a protein molecule present naturally in the vacuole and that is responsible for directing [targeting] the associated protein molecule specifically into the plant vacuole, the said peptide originating from the C-terminal extension of a plant chitinase and being coded for by a DNA sequence according to any one of claims I to 9.

11. A peptide according to claim 10 that acts as the targeting signal for the plant vacuole and that has the following amino acid sequence, which is shown in SEQ ID NO: 1 and 2:

Arg Ser Phe Gly Asn Gly Leu Leu Val Asp Thr Met

12. A peptide according to claim 10 that, in association with a protein molecule, acts as the targeting signal for the plant vacuole and that has one of the amino acid sequences shown in SEQ ID NO: 3 to 7:
(a) Arg Ser Phe Gly Lys Asp Leu Leu Val Asp Thr Met End
(b) Arg Ser Phe Gly Asn Gly Leu Leu Val Asn Thr Met End
(c) Arg Ser Phe Gly Asn Gly Leu Leu Val Arg Thr Met End
(d) Asp Leu Leu Gly Asn Gly Leu Leu Val Asp Thr Met End
(e)

Ile Gly Asp Leu Leu Val Asp Thr Met End

13. A peptide according to claim 10 that is coded for by one of the DNA sequences according to claim 9 and that has one of the amino acid sequences shown in SEQ ID NO: 8 and 9:

Leu Leu Val Asp Thr Met End

Gly Leu Leu Val Asp Thr Met End

14. A recombinant DNA molecule that comprises a chimaeric genetic construction in which a DNA sequence obtainable from the 3'-terminal region of a gene that codes for a protein molecule present naturally in the vacuole, which DNA sequence codes for a C-terminal peptide [C-terminal extension] that is responsible for directing [targeting] an associated gene product of heterologous origin specifically into the plant vacuole, is linked in operable manner to a heterologous gene, the said DNA sequence not coding for the about 20 C-terminal amino acids of the primary translation product of wild-type osmotin.

15. A recombinant DNA molecule according to claim 14 that comprises a chimaeric genetic construction in which a DNA sequence according to any one of claims 1 to 9 is linked in operable manner to a heterologous gene.

16. A recombinant DNA molecule according to claim 14 or 15, wherein the chimaeric genetic construction is operably linked to expression signals active in plant cells and, optionally, to further coding and/or non-coding sequences of the 3' and/or 5' region, so that, on transformation into a plant host, the expression product is directed specifically into the plant vacuole.

17. A recombinant DNA molecule according to claim 16, wherein the expression signals originate from genes of plants or plant viruses or are bacterial expression signals.

18. A recombinant DNA molecule according to claim 17, wherein the expression signals are promoter and/or termination signals of Cauliflower Mosaic Virus genes (CaMV).

19. A recombinant DNA molecule according to claim 17, wherein the expression signals are the expression signals of the nopaline synthase genes (nos) and/or of the octopine synthase genes (ocs) from the Ti-plasmids of *Agrobacterium tumefaciens*.

20. A recombinant DNA molecule according to claim 16 that comprises additional non-coding regulatory DNA sequences of the 3' and/or 5' region that are capable of regulating the transcription of an associated DNA sequence in plant tissues in the sense of induction or repression.

21. A recombinant DNA molecule according to claim 16, wherein the said heterologous gene comprises in its 5'-terminal region a sequence that codes for an N-terminal signal peptide, or is linked in operable manner to such a sequence.

22. A recombinant DNA molecule according to any one of claims 14 to 16, wherein the said heterologous gene is a structural gene that is capable of conferring on the transformed plant cells and also on the tissues developing therefrom, and especially on the plants themselves, a protective effect against pathogens, chemicals and adverse environmental factors.

23. A recombinant DNA molecule according to claim 22, wherein the said heterologous gene is a gene that expresses chitinase in plant cells.

24. A recombinant DNA molecule according to claim 22, wherein the said heterologous gene is a gene that expresses glucanase in plant cells.

25. A recombinant DNA molecule according to any one of claims 14 to 16, wherein the said chimaeric genetic construction, on expression in the transformed plant cell as such or as part of a unit of higher organisation selected from the group consisting of a tissue, an organ, a callus, an embryo and a whole plant, results in an expression product that is directed into the plant vacuole.

26. A recombinant DNA molecule according to any one of claims 14 to 16 that comprises in addition a DNA sequence that codes for a selectable phenotypic marker.

27. A recombinant DNA molecule according to any one of claims 14 to 16 that comprises in addition an origin of replication that permits replication in one or more microorganisms.

28. A cloning vector comprising a recombinant DNA molecule according to any one of claims 14 to 27.

29. A transformation vector and/or expression vector comprising a recombinant DNA molecule according to any one of claims 14 to 27.

30. A shuttle vector comprising a recombinant DNA molecule according to claim 27, which vector is capable of stable replication both in *E. coli* and in *A. tumefaciens*.

31. A non-human host organism comprising a recombinant DNA molecule according to any one of claims 14 to 27 or a vector according to any one of claims 28 to 30.

32. A host organism according to claim 31 that is a bacterium or a plant.

33. Plant material selected from the group consisting of protoplasts, cells, calli, tissues, organs, seeds, embryos, pollen, ovules and zygotes, which plant material comprises a recombinant DNA molecule according to any one of claims 14 to 27 or a vector according to any one of claims 28 to 30.

34. A transgenic plant, including its sexual and asexual progeny, comprising a recombinant DNA molecule according to any one of claims 14 to 27 and/or a vector according to any one of claims 28 to 30.

35. A transgenic plant regenerated from plant material according to claim 33 comprising a recombinant DNA molecule according to any one of claims 14 to 27 or a vector according to any one of claims 28 to 30.

36. A transgenic plant according to either claim 34 or claim 35 that is a fertile plant.

37. Propagation material of a transgenic plant according to any one of claims 34 to 36, wherein the propagation material comprises a recombinant DNA molecule according to any one of claims 14 to 27 and/or a vector according to any one of claims 28 to 30.

38. A part of a transgenic plant according to any one of claims 34 to 36 that is blossom, a stem, a fruit, a leaf or a root.

39. A process for the production of a recombinant DNA molecule for directing expression products specifically into the plant vacuole, which process essentially comprises:
(a) first isolating or synthesising by means of known processes the 3' region of a gene that codes for a protein molecule present naturally in the vacuole;
(b) inserting the said DNA sequence in operable manner into the 3'-terminal end of any desired structural gene that is heterologous in respect of the associated targeting sequence defined in (a).

40. A process according to claim 39, wherein the said structural gene comprises in its 5'-terminal region a sequence that codes for an N-terminal signal peptide, or is linked in operable manner to such a sequence.

## Revendications

1. Séquence d'ADN qui code pour un peptide, **caractérisée en ce que** ladite séquence provient de la région 3'-terminale d'un gène qui code pour une molécule de protéine naturellement présente dans la vacuole, et **en ce que** ladite séquence d'ADN code pour un peptide C-terminal [extension C-terminale] qui est responsable de l'insertion ciblée ["ciblage"] d'un produit génique associé d'origine hétérologue dans la vacuole végétale, ladite séquence d'ADN provenant de la région 3'-terminale d'un gène de chitinase végétale.

2. Séquence d'ADN selon la revendication 1, **caractérisée en ce que** ladite séquence présente la séquence nucléotidique générale suivante, représentée en SEQ ID NO: 1 : 
CGN/AGR TCN/AGY TTY GGN AAY GGN CTN/TTR TTR/CTN GTN GAY ACN ATG
TAA
 dans laquelle
N est A ou G ou C ou T/U ;
RestGouA;
W est A ou T/u ; et
Y est T/U ou C.

3. Séquence d'ADN selon la revendication 1, **caractérisée en ce que** ladite séquence provient de la région 3'-terminale d'un gène de chitinase basique de plants de *Nicotiana tabacum* L. c.v. Havana 425, est présente la séquence nucléotidique suivante, présentée en SEQ ID NO: 2 :

3'- AGG TCT TTT GGA AAT GGA CTT TTA GTC GAT ACT ATG TAA - 5'

4. Séquence d'ADN qui pour l'essentiel est homologue des séquences d'ADN présentées dans l'une des revendications 1 à 3, et qui présente encore les propriétés importantes selon l'invention de ces séquences, c'est-à-dire qui code pour un peptide C-terminal, lequel joue un rôle d'un signal de ciblage pour la vacuole végétale, **caractérisée en ce que** ladite séquence présente l'une des séquences nucléotidiques présentées en SEQ ID NO : 3 à 7 :

(a) AGG TCT TTT GGA AAA GAT CTT TTA GTC GAT ACT ATG TAA
(b) AGG TCT TTT GGA AAT GGA CTT TTA GTC AAT ACT ATG TAA
(c) AGG TCT TTT GGA AAT GGA CTT TTA GTC CGT ACT ATG TAA
(d) A/T GAT CTT TTG GGA AAT GGA CTT TTA GTC GAT ACT ATG TAA
(e) ATC GGT GAT CTT TTA GTC GAT ACT ATG TAA

5. Séquence d'ADN selon la revendication 4, **caractérisée en ce qu'**il s'agit d'une séquence d'ADN naturelle.

6. Séquence d'ADN selon la revendication 4, **caractérisée en ce qu'**il s'agit d'une séquence préparée à l'aide de technique connue de mutation.

7. Séquence d'ADN selon la revendication 6, **caractérisée en ce que**, pour ce qui concerne ledit procédé de mutation, il s'agit d'une mutagenèse induite par des oligonucléotides.

8. Séquence d'ADN selon la revendication 1, dans laquelle la séquence d'ADN représente une séquence partielle de la séquence d'ADN présentée en SEQ ID NO: 2 et code pour un peptide qui est responsable de l'insertion ciblée ["ciblage"] d'un produit génétique associé d'origine hétérologue dans la vacuole végétale.

9. Séquence d'ADN selon la revendication 8, qui comprend l'une des séquences nucléotidiques présentées en SEQ ID NO : 8 et 9 :

CTT TTA GTC GAT ACT ATG TAA
GGA CTT TTA GTC GAT ACT ATG TAA

10. Peptide, **caractérisé en ce qu'**il provient de la région C-terminale d'un gène qui code pour une molécule protéique naturellement vacuolaire, et qui est responsable de l'insertion ciblée ["ciblage"] de la molécule protéique associée, dans la vacuole végétale, ledit peptide provenant de l'extension C-terminale d'une chitinase végétale et étant codé par une séquence d'ADN selon l'une des revendications 1 à 9.

11. Peptide selon la revendication 10, qui joue une rôle d'un signal de ciblage pour la vacuole végétale, et qui présente la séquence d'acides aminés suivante, présentée en SEQ ID NO : 1 et 2 :

Arg Ser Phe Gly Asn Gly Leu Leu Val Asp Thr Met

12. Peptide selon la revendication 10, qui en association avec une molécule protéique joue le rôle d'un signal de ciblage pour la vacuole végétale, et qui présente l'une des séquences d'acides aminés présentées en SEQ ID NO 3 à 7 :

(a) Arg Ser Phe Gly Lys Asp Leu Leu Val Asp Thr Met End
(b) Arg Ser Phe Gly Asn Gly Leu Leu Val Asn Thr Met End
(c) Arg Ser Phe Gly Asn Gly Leu Leu Val Arg Thr Met End
(d) Asp Leu Leu Gly Asn Gly Leu Leu Val Asp Thr Met End
(e) Ile Gly Asp Leu Leu Val Asp Thr Met End

13. Peptide selon la revendication 10, qui est codé par l'une des séquences d'ADN selon la revendication 9, et comprend l'une des séquences d'acides aminés présentées en SEQ ID NO : 8 et 9

Leu Leu Val Asp Thr Met End
Gly Leu Leu Val Asp Thr Met End

14. Molécule d'ADN recombinante, **caractérisée en ce qu'**elle contient une construction génétique chimère, dans laquelle une séquence d'ADN, pouvant être obtenue à partir de la région 3'-terminale d'un gène, code pour une molécule protéine naturellement vacuolaire, ladite séquence d'ADN codant pour peptide C-terminale [extension C-terminale] qui est responsable de l'insertion ciblée ["ciblage"] d'un produit génique associé d'origine hétérologue dans la vacuole végétale, ladite séquence d'ADN ne codant pas pour les environ 20 acides aminés C-terminaux du produit de traduction primaire de l'Osmotin de type sauvage, et étant lié d'une manière opérationnelle à un gène hétérologue.

15. Molécule d'ADN recombinante selon la revendication 14, **caractérisée en ce qu'**elle contient une construction génétique chimère, dans laquelle une séquence d'ADN selon l'une des revendications 1 à 9 est liée d'une manière opérationnelle à un gène hétérologue.

16. Molécule d'ADN recombinante selon la revendication 14 ou 15, **caractérisée en ce que** la construction génétique chimère est liée d'une manière opérationnelle à des signaux d'expression actifs dans des cellules végétales et éventuellement à d'autres séquences, codantes et/ou non codantes, de la région 3' et/ou de la région 5', de telle sorte que, lors d'une transformation dans un hôte végétal, il en résulte une insertion ciblée du produit d'expression dans la vacuole végétale.

17. Molécule d'ADN recombinante selon la revendication 16, **caractérisée en ce qu'**il s'agit de signes d'expression qui proviennent de gènes de plantes ou de virus végétaux, ou encore il s'agit de signes d'expression bactériens.

18. Molécule d'ADN recombinante selon la revendication 17, **caractérisée en ce qu'**il s'agit de signaux promoteurs et/ou de terminaison d'un gène du virus de la mosaïque du chou-fleur (CaMV).

19. Molécule d'ADN recombinante selon la revendication 17, **caractérisée en ce qu'**il s'agit des signaux d'expression des gènes de la nopalline-5-synthase (nos) et/ou des gènes de l'octopine-synthase (ordinateurs client 20, 30 et 40) provenant des plasmides Ti d'*Agrobacterium tumefaciens*.

20. Molécule d'ADN recombinante selon la revendication 16, **caractérisée en ce que** ladite molécule d'ADN recombinante contient des séquences d'ADN régulatrices, non codantes, supplémentaires, de la région 3' et/ou de la région 5', qui sont à même de réguler au sens d'une réduction ou d'une répression la transcription d'une séquence d'ADN associée dans des tissus végétaux.

21. Molécule d'ADN recombinante selon la revendication 16, **caractérisée en ce que** ledit gène hétérologue contient dans sa région 5'-terminale une séquence, ou encore est lié d'une manière opérationnelle à une séquence, qui code pour un peptide signal N-terminal.

22. Molécule d'ADN recombinante selon l'une des revendications 14 à 16, **caractérisée en ce que**, pour ce qui concerne ledit gène hétérologue, il s'agit d'un gène de structure qui est à même de conférer aux cellules végétales transformées, ainsi qu'aux tissus qui se développent à partir d'elles, et en particulier aux plantes proprement dites, un effet de protection contre les microorganismes pathogènes, les agents chimiques, et ainsi que les effets gênants de l'environnement.

23. Molécule d'ADN recombinante selon la revendication 22, **caractérisée en ce que**, pour ce qui concerne ledit gène hétérologue, il s'agit d'un gène qui exprime la chitinase dans des cellules végétales.

24. Molécule d'ADN recombinante selon la revendication 22, **caractérisée en ce que**; pour ce qui concerne ledit gène hétérologue, il s'agit d'un gène qui exprime la glucanase dans des cellules végétales.

25. Molécule d'ADN recombinante selon l'une des revendications 14 à 16, **caractérisée en ce que** ladite construction génétique chimère conduit, lors d'une expression dans la cellule végétale transformée en l'état ou sous forme d'un constituant d'une unité organisée supérieure choisie dans l'ensemble consistant à un tissu, un organe, un cals, un embryon ou une plante entière, à un produit d'expression qui est inséré dans la vacuole végétale.

26. Molécule d'ADN recombinante selon l'une des revendications 14 à 16, **caractérisée en ce qu'**elle contient en outre une séquence d'ADN qui code pour un marqueur phénotypique sélectionnable.

27. Molécule d'ADN recombinante selon l'une des revendications 14 à 16, **caractérisée en ce qu'**elle contient en outre une origine de réplication qui autorise une réplication dans un ou plusieurs microorganismes.

28. Vecteur de clonage contenant une molécule d'ADN recombinante selon l'une des revendications 14 à 27.

29. Vecteur de transformation et/ou vecteur d'expression contenant une molécule d'ADN recombinante selon l'une des revendications 14 à 27.

30. Vecteur navette contenant une molécule d'ADN recombinante selon la revendication 27, qui est à même de se répliquer dans des conditions stables tant dans un *E. coli* que dans A. *tumefaciens*.

31. Organisme hôte non humain contenant une molécule d'ADN recombinante selon l'une des revendications 14 à 27 ou un vecteur selon l'une des revendications 28 à 30.

32. Organisme hôte selon la revendication 31, **caractérisé en ce que**, pour ce qui concerne ledit organisme hôte, il s'agit d'une bactérie, ou encore d'une plante.

33. Matériel végétal choisi dans l'ensemble consistant en les protoplastes, les cellules, les cals, les tissus, les organes, les semences, les embryons, le pollen, les ovules, les zygotes, qui contient une molécule d'ADN recombinante selon l'une des revendications 14 à 27 ou un vecteur selon l'une des revendications 28 à 30.

34. Plante transgénique, y compris sa descendance sexuée et asexuée contenant une molécule d'ADN recombinante selon l'une des revendications 14 à 27 et/ou un vecteur selon l'une des revendications 28 à 30.

35. Plante transgénique régénérée à partir d'un matériel végétal selon la revendication 33, qui contient une molécule d'ADN recombinante selon l'une des revendications 14 à 27 ou un vecteur selon l'une des revendications 28 à 30.

36. Plante transgénique selon l'une des revendications 34 à 35, **caractérisée en ce qu'**il s'agit d'une plante fertile.

37. Produit de multiplication d'une plante transgénique selon l'une des revendications 34 à 36, **caractérisée en ce que** le produit de multiplication contient une molécule d'ADN recombinante selon l'une des revendications 14 à 27 et/ou un vecteur selon l'une des revendications 28 à 30.

38. Parties d'une plante transgénique selon l'une des revendications 34 à 36, **caractérisées en ce que** ses parties sont des fleurs, des tiges, des fruits, des feuilles ou des racines.

39. Procédé de préparation d'une molécule d'ADN recombinante pour insertion ciblée de produits d'expression dans la vacuole végétale, caractérisé pour l'essentiel en ce que
(a) on isole d'abord la région 3' d'un gène qui code pour une molécule protéique naturellement vacuolaire, ou encore on l'a synthétise par des procédés connus ;
(b) on insert d'une manière opérationnelle ladite séquence d'ADN dans l'extrémité 3'-terminale d'un gène de structure quelconque, qui est hétérologue par rapport à la séquence de ciblage associée définie en (a).

40. Procédé selon la revendication 39, **caractérisé en ce que** ledit gène de structure, dans sa région 5'-terminale, contient une séquence, ou est lié d'une manière opérationnelle à une séquence, qui code pour un peptide de signal N-terminal.
